# EUROPEAN PATENT APPLICATION

(11) **EP 0 674 003 A2**
(43) Date of publication of application: **27.09.1995**
(21) Application number: 95301997.3
(22) Date of filing: 24.03.1995
(51) Int. Cl.: C12N 15/12, C07K 14/705, C12N 5/10, G01N 33/68, C07K 16/28

(54) **Modulatory proteins of human CNS receptors**

(30) Priority: 25.03.1994 US 217704; 23.06.1994 US 264578
(71) Applicant: Allelix Biopharmaceuticals Inc., Mississauga Ontario, L4V 1P1 (CA)
(72) Inventor: Foldes, Robert, Willowdale, Ontario M2R 2W6 (CA); Fantaske, Robert, Toronto, Ontario M4J 3X3 (CA); Adams, Sally-Lin, Toronto, M6P 2E9 (CA); Kamboj, Rajender, Mississauga, Ontario, L5N 6N9 (CA)
(74) Representative: Mercer, Christopher Paul

(57) **Abstract**

Neurotransmission by excitatory amino acids (EAAs) such as glutamate is mediated via membrane-bound surface receptors. This neurotransmission has been found to be modulatcd by certain modulatory proteins. DNA coding for a family of such modulatory proteins has now been isolated and the modulatory proteins have been characterized. Herein described are recombinant cell lines which produce these modulatory proteins as heterologous membrane-bound products. Also described are related aspects of the invention, which are of commercial significance, including the use of cell lines which express the modulatory proteins either homomerically, or heteromerically in a complex with an NMDA receptor, as a tool for discovery of compounds which affect the function of the modulatory proteins.

## Description

### Field of the Invention

This invention relates to the application of recombinant DNA technology in the field of neurobiology. More particularly, the invention relates to the cloning and expression of DNA coding for proteins which modulate the function of glutamate receptors.

### Background to the Invention

In the mammalian central nervous system (CNS), the transmission of nerve impulses is controlled by the interaction between a neurotransmitter substance released by the "sending" neuron which then binds to a surface receptor on the "receiving" neuron, to cause excitation thereof. L-glutamate is the most abundant neurotransmitter in the CNS, and mediates the major excitatory pathway in vertebrates. Glutamate is therefore referred to as an excitatory amino acid (EAA) and the receptors which respond to it are variously referred to as glutamate receptors, or more commonly as EAA receptors.

Members of the EAA receptor family can be grouped into three main types based on differential binding to certain glutamate analogs. One type of EAA receptor, which in addition to glutamate also binds the compound NMDA (N-methyl-D-aspartate), is referred to as the NMDA type of EAA receptor. Two other glutamate-binding types of EAA receptor, which do not bind NMDA, are named according to their preference for binding with two other EAA receptor agonists, namely AMPA (alpha-amino-3-hydroxy-5-methyl-isoxazole-4-propionate), and kainate (2-carboxy-4-(1-methylethenyl)-3-pyrrolidineacetate). Accordingly, receptors which bind glutamate but not NMDA, and which bind with greater affinity to kainate than to AMPA, are referred to as kainate-type EAA receptors. Similarly, those EAA receptors which bind glutamate but not NMDA, and which bind AMPA with greater affinity than kainate are referred to as AMPA-type EAA receptors.

The glutamate-binding EAA receptor family is of great physiological and medical importance. Glutamate is involved in many aspects of long-term potentiation (learning and memory), in the development of synaptic plasticity, in epileptic seizures, in neuronal damage caused by ischemia following stroke or other hypoxic events, as well as in other forms of neurodegenerative processes. The development of therapeutics which modulate these processes is being slowed by the lack of any homogeneous source of receptor material with which to discover selectively binding drug molecules, which interact specifically at the interface of an appropriate EAA receptor. The brain derived tissues currently used to screen candidate drugs are heterogeneous receptor sources, possessing on their surface many receptor types which interfere with studies of the EAA receptor/ligand interface of interest. The search for human therapeutics is further complicated by the limited availability of brain tissue of human origin. It would therefore be desirable to obtain cells that are genetically engineered to produce only the receptor of interest. With cell lines expressing cloned receptor cDNA, a substrate which is homogeneous for the desired receptor is provided, for drug screening programs.

Non-human cDNAs which appear to encode the NMDA-type of EAA receptor have recently been identified and isolated. A cDNA encoding a subunit polypeptide of an NMDA receptor in rat, designated NR1, has been isolated as described by Moriyoshi et al. in Nature 354: 31, 1991. An extension of this work has revealed seven isoforms of NR1, presumably generated by combinations of alternative RNA splicing in the amino- and carboxy-terminal regions of NR1 (Anantharam et al. FEBS Lett. 305:27, 1992; Durand et al. Proc. Natl. Acad. Sci. USA 89: 9359, 1992; Nakanishi et al. Proc. Natl. Acad. Sci. USA 89: 8552, 1992; Sugihara et al. Biochem. Biophys. Res. Commun. 185: 826, 1992; Hollmann et al. Neuron 10: 943, 1993; Kusiak and Norton. Mol. Brain. Res. 20: 64, 1993). DNA encoding NR1 and one of its isoforms have also been cloned from mouse brain by Yamazaki et al. as described in FEBS Lett. 300: 39, 1992. Other rat NMDA receptor subunits, designated NR2A, NR2B, NR2C and NR2D, have also been identified (Monyer et al. Science 256: 1217, 1992; Ishii et al. J. Biol. Chem. 268: 2836, 1993), as well as mouse NMDA receptor subunits which have been designated ε1, ε2, ε3 and ε4 (Meguro et al. Nature 357: 70, 1992; Kutsuwada et al. Nature 358: 36, 1992; Ikeda et al. FEBS Lett. 313: 34, 1992).

There has emerged from these molecular cloning advances, a better understanding of the structural features of NMDA receptors and their subunits, as they exist in the non-human brain. According to the current model, each NMDA receptor is heteromeric, consisting of individual membrane-anchored subunits, each comprising transmembrane regions and extracellular domains that dictate ligand-binding properties and contribute to the ion-gating function served by the receptor complex.

In the search for therapeutics useful to treat CNS disorders in humans, it is highly desirable to obtain knowledge of human EAA receptors, and proteins which modulate the activity of these receptors. Such an understanding would provide a means to screen for compounds that selectively interact with this activity, i.e. to stimulate or inhibit receptor activity, thereby providing a means to identify compounds having potential therapeutic utility in humans. Non-human mammalian models are not suitable for this purpose despite significant protein homology due to the fact that minute sequence discrepancies have been found to cause dramatic pharmacological and functional variation between species homologues of the same protein (Oksenberg et al., Nature, 360:161, 1992; Hall et al. Trends Pharmacol. Sci. 14: 376, 1993). It is therefore particularly desirable to provide cloned cDNA encoding human EAA receptor proteins or modulatory proteins thereof, and cell lines expressing these proteins, in order to generate a screening method for compounds therapeutically useful in humans. These, accordingly, are objects of the present invention.

### Summary of the Invention

Human cDNAs encoding a NMDA receptor modulatory proteins have been identified and characterized. These modulatory proteins belong to three families designated herein the NR2A, NR3 and NR4 families, each of which comprise a parent protein (NR2A-1, NR3-1 and NR4-1) as well as functional sequence-related variants and functional fragments of each parent protein.

In one of its aspects, thus, the present invention provides an isolated polynucleotide, consisting either of DNA or of RNA, which codes for a protein from one of the modulatory protein families, or for functional fragments thereof.

In another aspect of the present invention, there is provided a cell that has been genetically engineered to produce a human EAA receptor modulatory protein belonging to the herein-defined protein family. In related aspects of the present invention, there are provided recombinant DNA constructs and methods useful to obtain substantially homogeneous sources of the human modulatory protein, comprising the steps of culturing the genetically engineered cells, and then recovering the cultured cells.

In another aspect of the present invention, there is provided a method for evaluating interaction between a candidate ligand and a human EAA receptor modulatory protein, which comprises the steps of incubating the candidate ligand with a genetically engineered cell as described above, or with a membrane preparation derived therefrom, and then assessing said interaction by determining the extent of protein/ligand binding, or by determining the ligand-induced electrical current across said cell.

In yet another aspect of the present invention, a cell that has been engineered genetically to produce a human heteromeric receptor complex comprising a modulatory protein and an NMDA receptor is provided.

In a further aspect of the present invention, there is provided a method for evaluating interaction between a candidate ligand and a human heteromeric receptor complex comprising a modulatory protein and an NMDA receptor, said method comprising the steps of incubating the candidate ligand with a cell line engineered to produce said receptor complex, or with a membrane preparation derived therefrom, and then assessing the interaction therebetween by determining the extent of protein/ligand binding, or by determining the ligand-induced electrical current across said cell.

Other aspects of the present invention include a human modulatory protein selected from the NR2A, NR3 and NR4 families, in a form essentially free from other proteins of human origin, functional and immunogenic fragments of such proteins, antibodies which bind to said proteins, and oligonucleotides which hybridize to nucleic acids encoding these proteins.

Other aspects of the present invention, which encompass various applications of the discoveries herein described, will become apparent from the following detailed description, and from the accompanying drawings in which:

### Brief Reference to the Drawings

Figure 1 provides the nucleotide sequence (SEQ ID NO: 1) of DNA encoding an EAA receptor modulatory protein (NR2A-1) according to the present invention, and the deduced amino acid sequence (SEQ ID NO: 2) thereof;
Figures 2A and 2B illustrate, with plasmid maps, the strategy used to construct expression vectors harbouring the DNA sequence illustrated in Figure 1;
Figure 3 provides, with reference to Figure 1, the partial DNA and amino acid sequences (SEQ ID NOs: 5 & 6) of a naturally occurring variant of the modulatory protein illustrated in Fig. 1;
Figure 4 provides the nucleotide sequence (SEQ ID NO: 7) of DNA encoding an NR3 modulatory protein, and the deduced amino acid sequence (SEQ ID NO: 8) thereof;
Figures 5A and 5B illustrate, with plasmid maps, the strategy used to construct expression vectors harbouring the DNA sequence illustrated in Figure 4;
Figure 6 provides, with reference to Figure 4, the partial DNA and amino acid sequences (SEQ ID NOs: 11 & 12) of a naturally occurring variant of the modulatory protein illustrated in Fig. 4;
Figure 7 provides the partial nucleotide sequence (SEQ ID NO: 13) of DNA encoding an NR4 modulatory protein, and the deduced amino acid sequence (SEQ ID NO: 14) thereof;
Figure 8 provides the nucleotide sequence (SEQ ID NO: 15) of DNA encoding the NMDAR1-1 receptor, and deduced amino acid sequence (SEQ ID NO: 16) thereof;
Figure 9 provides a comparison of partial nucleotide sequences of NMDAR1-1 (SEQ ID NO: 17) with its variants, NMDAR1-2, NMDAR1-3A and NMDAR1-3C (SEQ ID NOs: 18, 19 & 20, respectively);
Figure 10 provides a comparison of the amino acid sequences of NMDAR1-1 (SEQ ID NO: 21) and NMDAR1-4 (SEQ ID NO: 22);
Figure 11 provides a comparison of the amino acid sequences of NMDAR1-1/2/3/4 (SEQ ID NO: 23) and NMDAR1-5/6/7/8 (SEQ ID NO: 24);
Figures 12A-12D graphically illustrate electrophysiological properties of a heteromeric complex comprising NR2A-1 and NMDAR1-3C; and
Figures 13A-13D graphically illustrate electrophysiological properties of a heteromeric complex comprising NR3-1 and NMDAR1-3C.

### Detailed Description of the Invention and its Preferred Embodiments

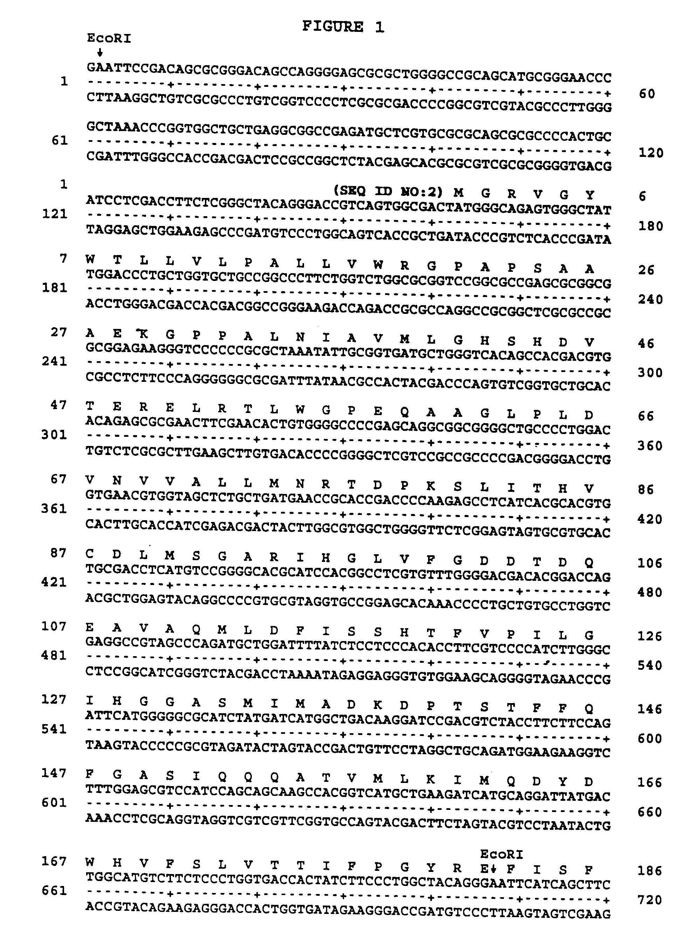

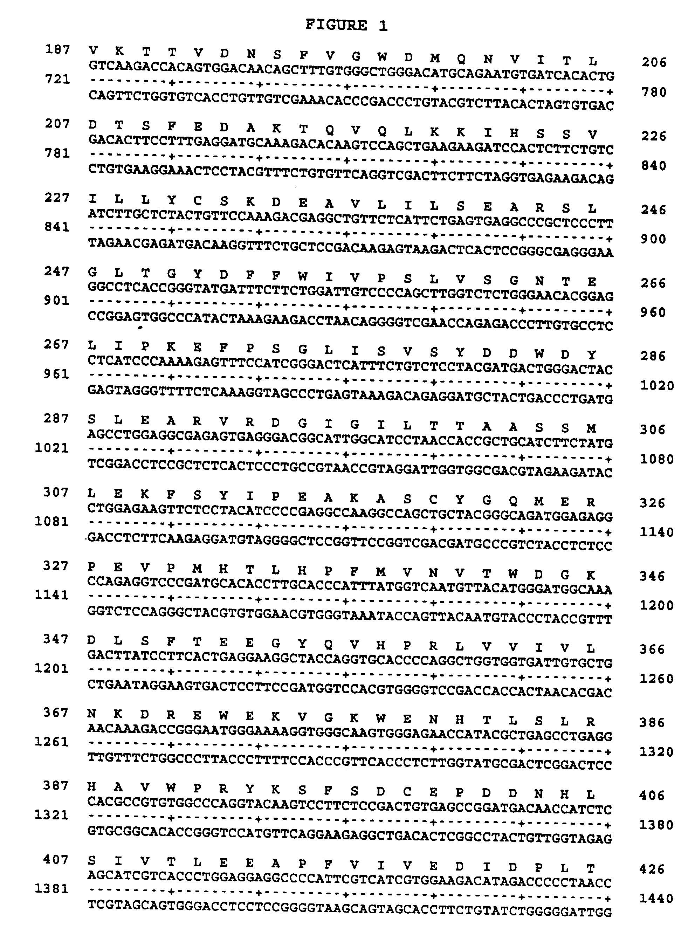

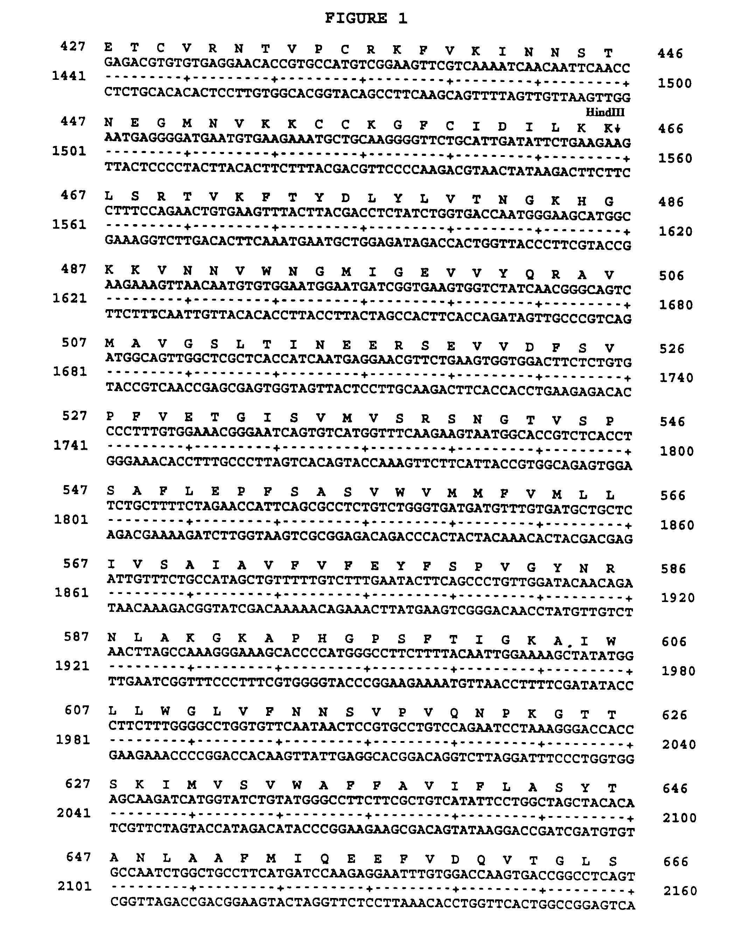

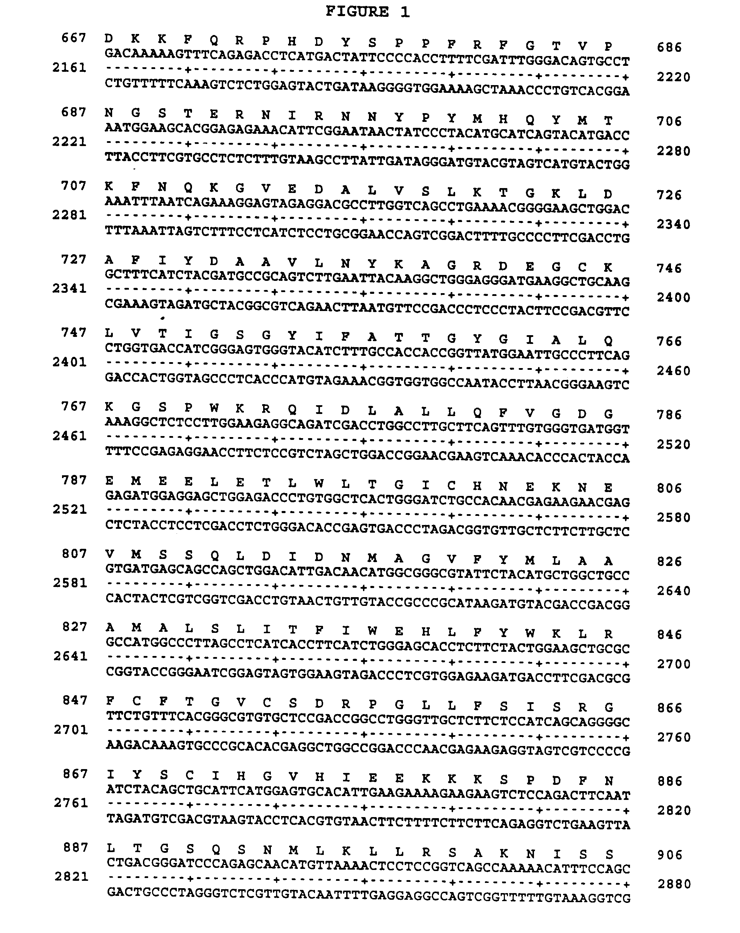

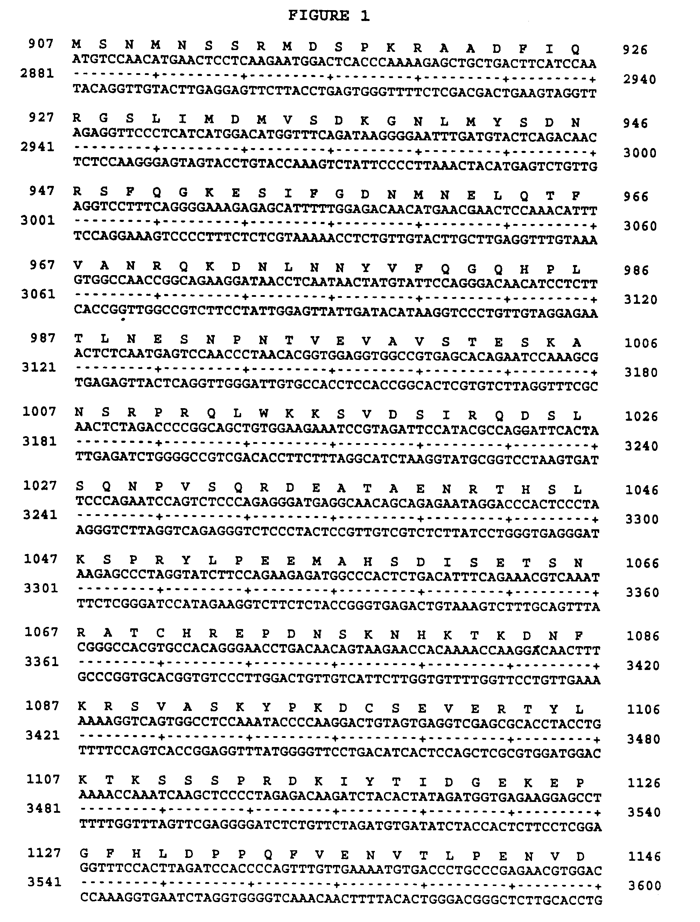

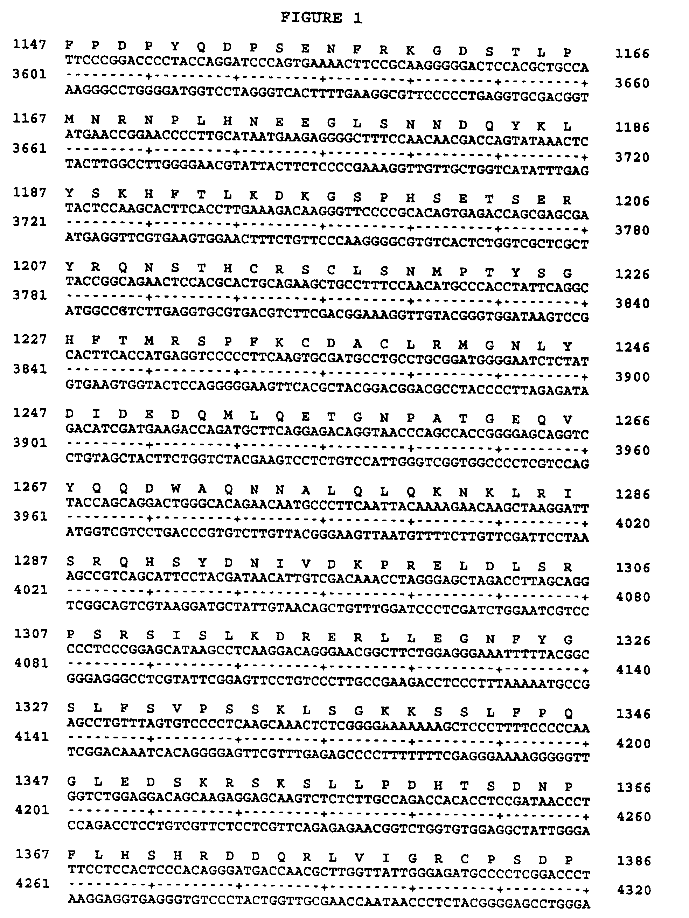

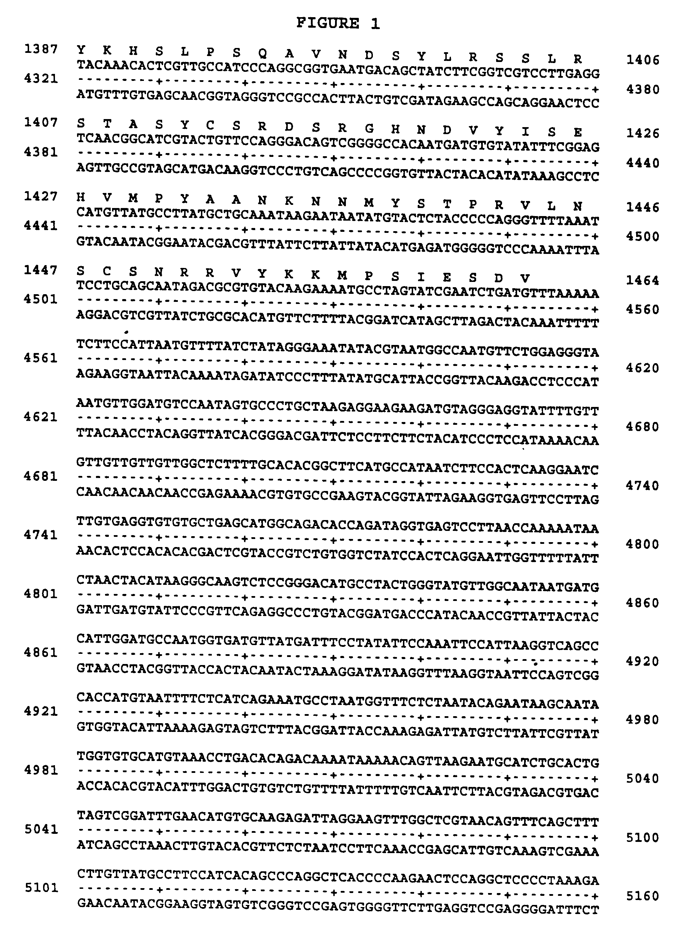

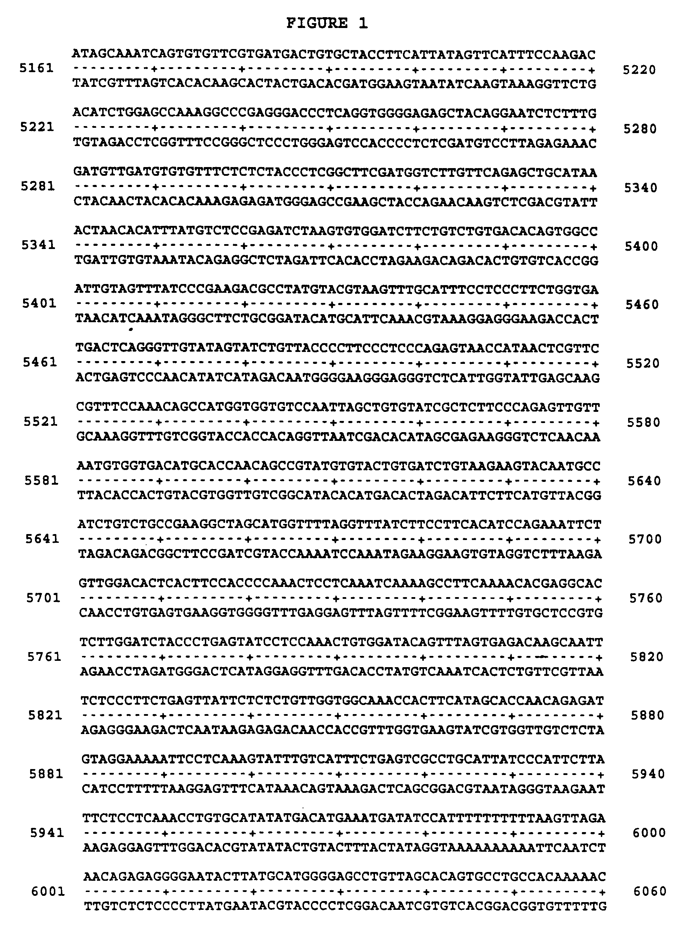

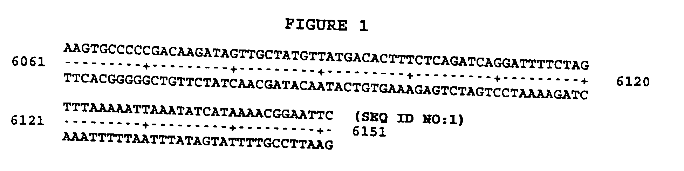

The present invention relates to modulatory proteins of excitatory amino acid (EAA) receptors of human origin, and to isolated polynucleotides encoding them. More particularly, the present invention is directed to novel human modulatory proteins belonging to protein families herein designated the NR2, NR3 and NR4 protein families. These proteins modulate the activity of human EAA receptors of the NMDA-type. The NR2 family of modulatory proteins comprises the human NR2A-1 protein, the amino acid sequence of which is identified in Fig. 1 (SEQ ID NO: 2), as well as functional sequence-related variants of the human NR2A-1 protein and functional fragments of the NR2A-1 protein. The NR3 and NR4 modulatory protein families comprise the NR3-1 (SEQ ID NO: 8) and NR4-1 parent proteins, as well as functional sequence-related variants of the human NR3-1 protein and functional fragments of the NR3-1 protein.

As used herein, the term "modulatory protein" refers to a protein that, when combined with a human EAA receptor, and in particular with a human NMDA receptor, forms a heteromeric receptor complex having electrophysiological properties which are distinct from the electrophysiological properties of a homomeric receptor complex formed from the selected NMDA receptor alone. Thus, the NR2A, NR3 and NR4 proteins of the present invention have been found to modulate the ion channel activity of NMDA-type receptors, i.e. receptors having a ligand binding profile comprising specific binding affinity for glutamate, NMDA and MK-801 [(+)-5-methyl-10,11-dihydro-5H-dibenzo[a,d]cyclohepten-5,10-imine maleate]. The electrophysiological properties, or ion channel activity, of EAA receptors is typically determined using established electrophysiological assays appropriate for detecting conductance across a cell membrane such as the assay described by Hollmann et al. in Nature 342: 643.

The term "isolated" as it is used herein with respect to modulatory protein encoding polynucleotides refers to polynucleotides which are free from human DNA which encodes, or partially encodes, CNS proteins other than modulatory proteins and NMDA receptor proteins.

The term "heteromeric receptor complex" is used to refer to a receptor complex comprising a modulatory protein, in accordance with the present invention, and an NMDA receptor.

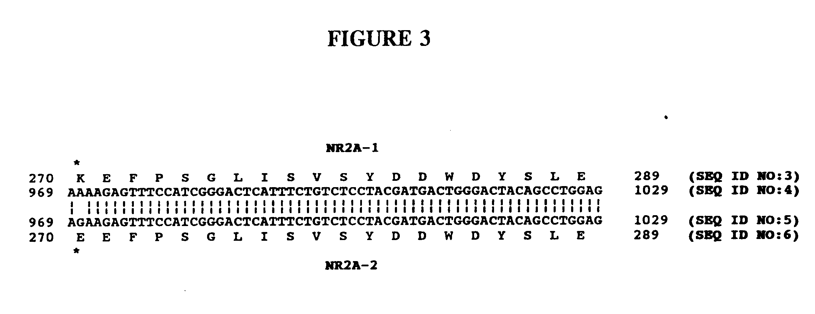

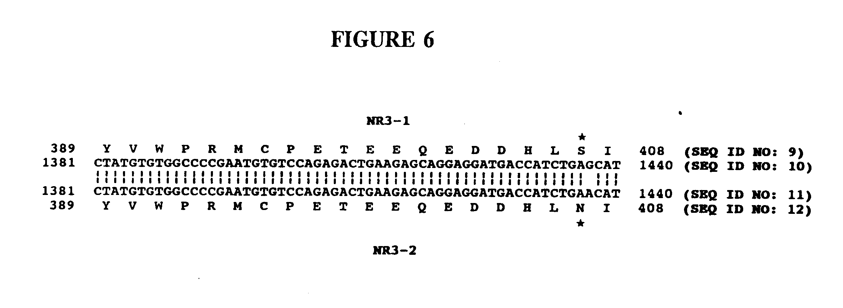

Variants of the parent modulatory proteins also form members of the modulatory protein family as defined above and include functional variants of the human NR2A-1, NR3-1 or NR4-1 proteins which exhibit a modulatory activity similar to that of the parent protein, and which demonstrate substantial sequence homology to the parent protein, i.e. share greater than 96% amino acid identity with the NR2A-1 protein, or share greater than 98.5% amino acid identity with the NR3-1 protein. Variants of the NR2A-1 protein include both naturally occurring variants, an example of which is the NR2A-2 protein, illustrated in part in Fig. 3 by nucleic acid and amino acid sequence (SEQ ID NOs: 5 & 6), as well as synthetically derived variants of the human NR2A-1 protein. Variants of the NR3-1 protein include both naturally occurring variants, an example of which is the NR3-2 protein, illustrated in part in Fig. 6 by nucleic acid and amino acid sequence (SEQ ID NOs: 11 & 12), as well as synthetically derived variants thereof.

The term "fragment" is used herein to denote functional segments of a modulatory protein selected from the NR2A, NR3 and NR4 families.

Variants and fragments of the present proteins are said to be "functional" if, on coexpression with an NMDA receptor in a heteromeric receptor complex as defined above, the complex, when assayed electrophysiologically, exhibits ligand-induced ion channel activity having measurable current, i.e. current which is greater than the current in the absence of the ligand or greater than the "baseline" current, and the channel activity possess properties which are characteristic of an NMDA ion channel, for example the channel activity is blocked by Mg⁺⁺ ions and by MK-801.

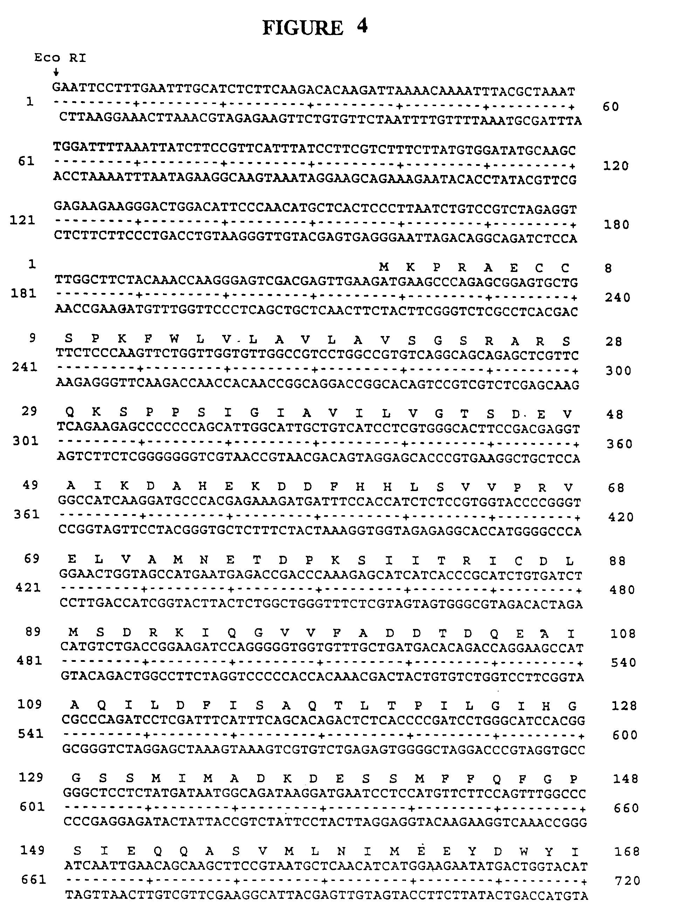

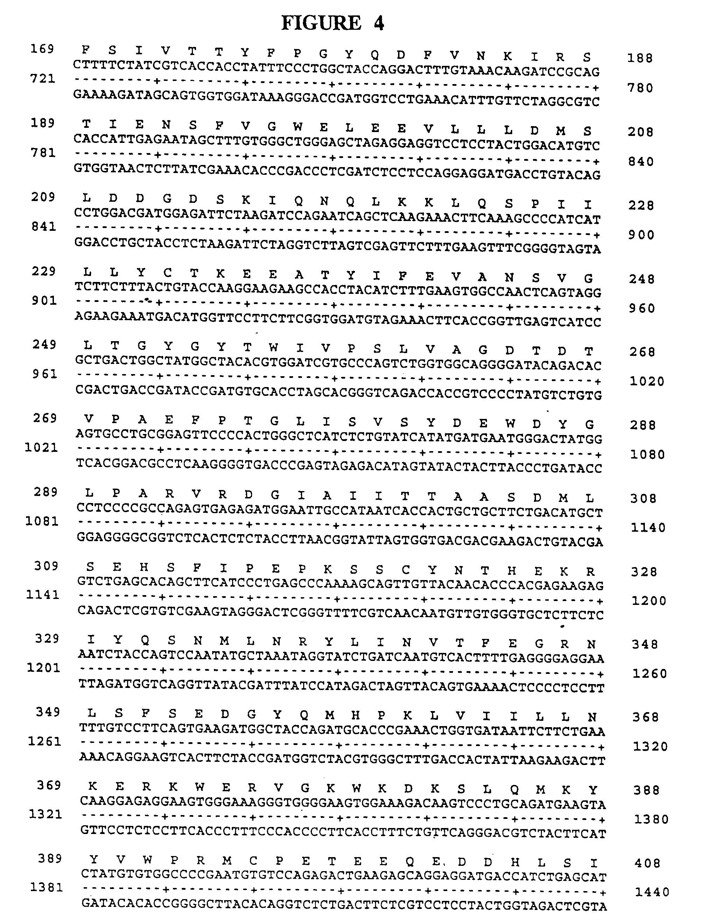

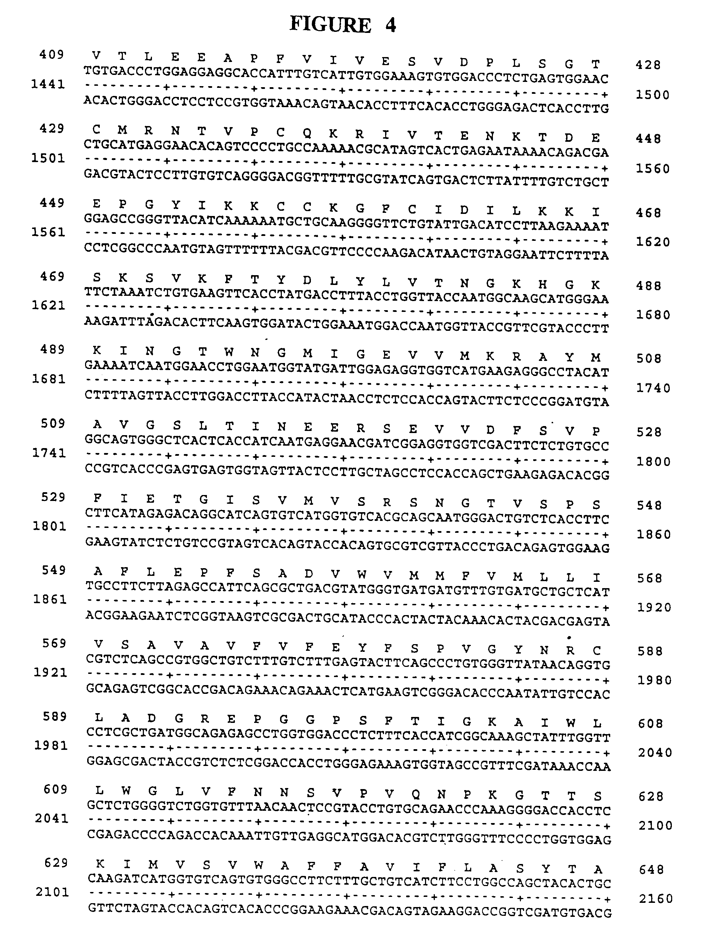

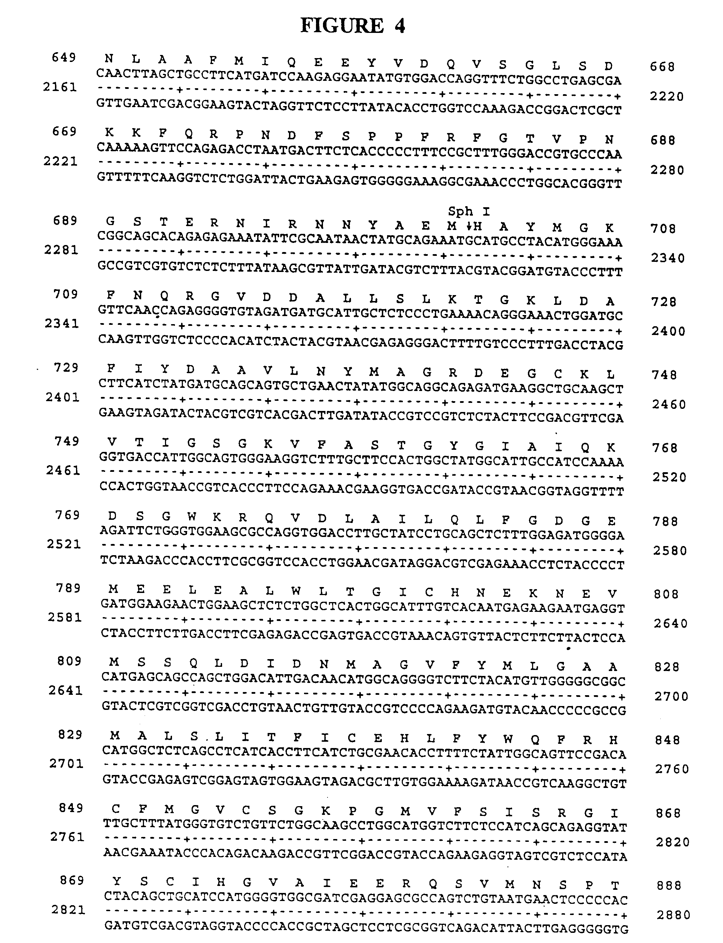

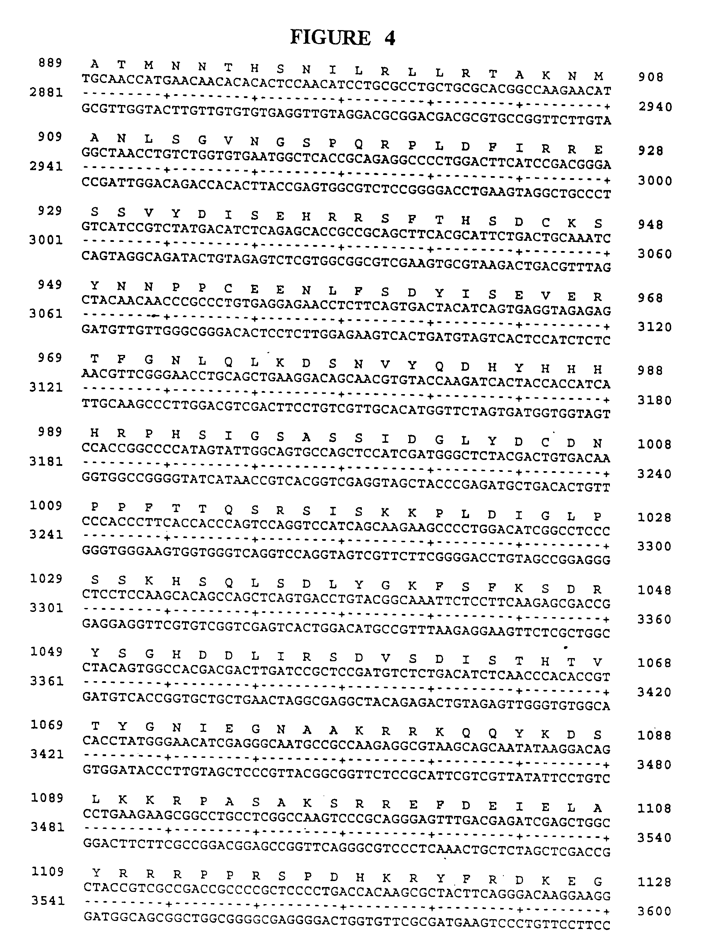

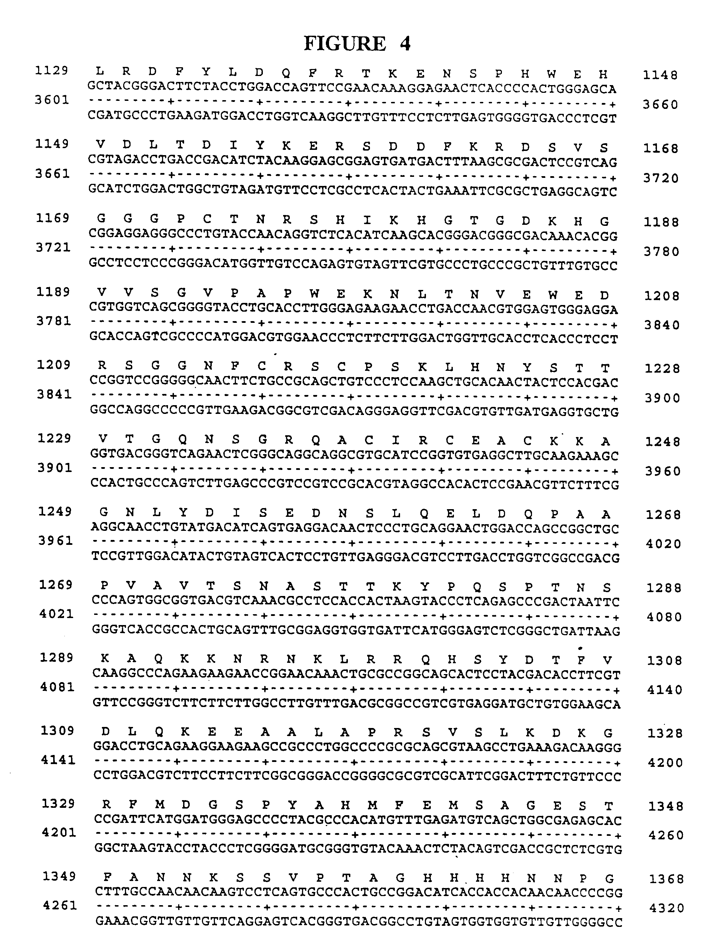

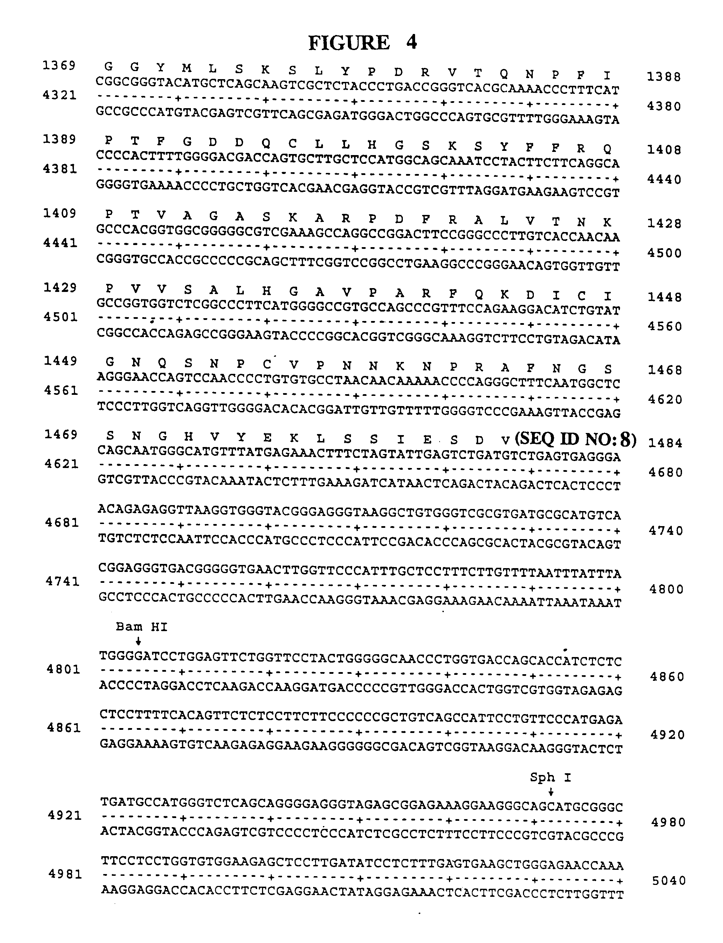

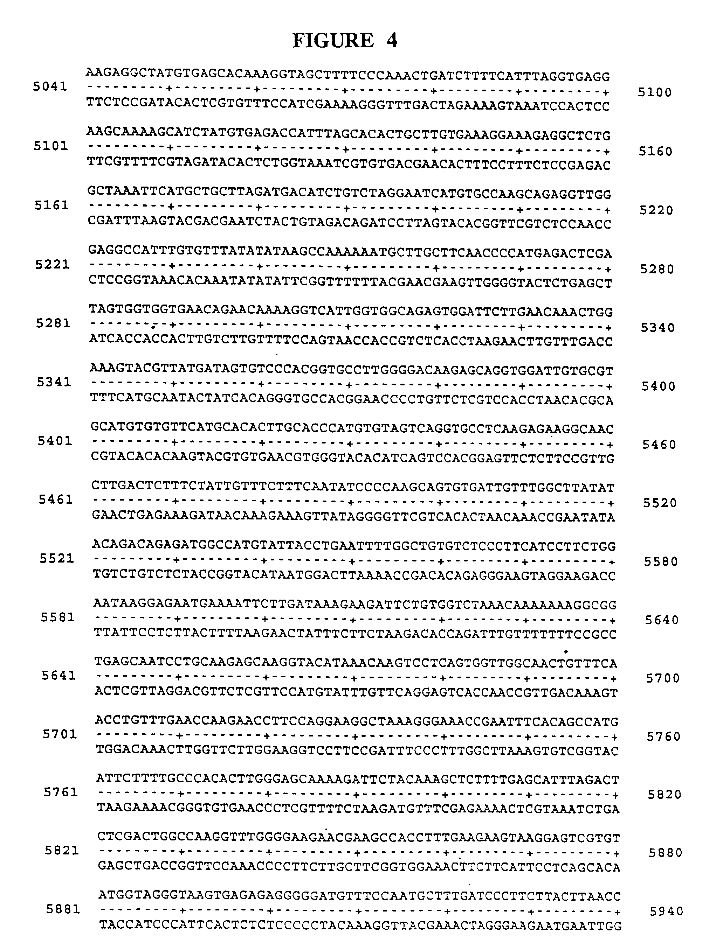

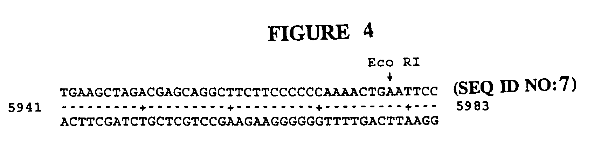

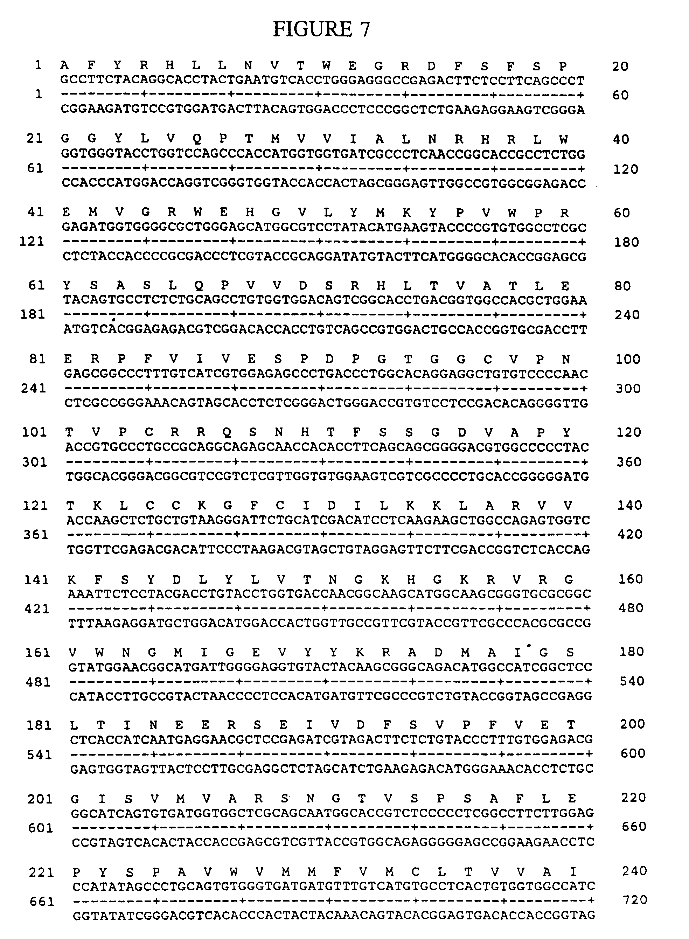

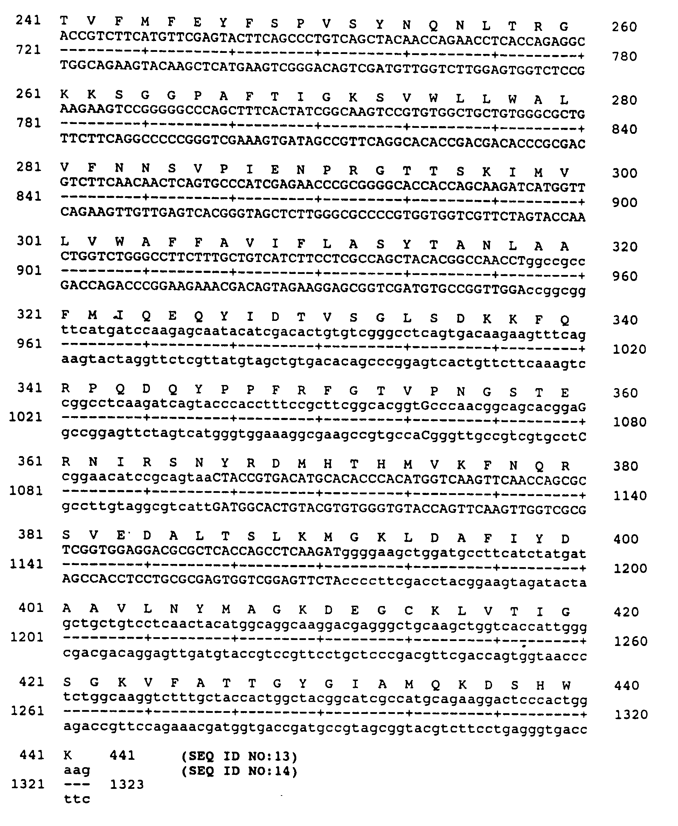

Each of the naturally occurring members of the human modulatory protein families defined herein possess structural features similar to those of EAA receptors, including an extracellular amino-terminal (N-terminal) region, as well as internal hydrophobic domains which serve to anchor the protein within the cell surface membrane. The particular human EAA receptor modulatory proteins designated NR2A-1, NR3-1 and NR4-1 are proteins characterized structurally as single polypeptide chains that are produced initially in precursor form bearing an N-terminal signal peptide, which are transported to the cell surface in mature form, lacking the signal peptide. The NR2A-1 protein, including its signal peptide, consists of 1,464 amino acids arranged in the sequence illustrated, by single letter code, in Figure 1 (SEQ ID NO: 2). The NR3-1 protein, including its signal peptide, consists of 1,484 amino acids arranged in the sequence illustrated, by single letter code, in Figure 4 (SEQ ID NO: 8). The particular human EAA receptor modulatory protein designated NR4-1 is a protein encoded by the partial nucleotide sequence illustrated in Figure 7 (SEQ ID NO: 13).

A naturally occurring structurally-related variant of the NR2A-1 protein has also been identified and is designated herein, the NR2A-2 modulatory protein. This variant protein differs from its NR2A-1 parent by a single amino acid as illustrated in Fig. 3. Specifically, the lysine residue at position 270 in NR2A-1 is a glutamic acid residue in the NR2A-2 variant. This change is reflected as a single nucleotide difference between the nucleic encoding the two proteins, namely a codon change from "AAA" in NR2A-1 to "GAA" in NR2A-2.
A naturally occurring structurally-related variant of the NR3-1 protein has also been identified and is designated herein, the NR3-2 modulatory protein. This variant protein differs from its NR3-1 parent by a single amino acid as illustrated in Fig. 6. Specifically, the serine residue at position 407 in NR3-1 is an asparagine residue in the NR3-2 variant. This change is reflected as a single nucleotide difference between the nucleic acid encoding the two proteins, namely a codon change from "AGC" in NR3-1 to "AAC" in NR3-2.

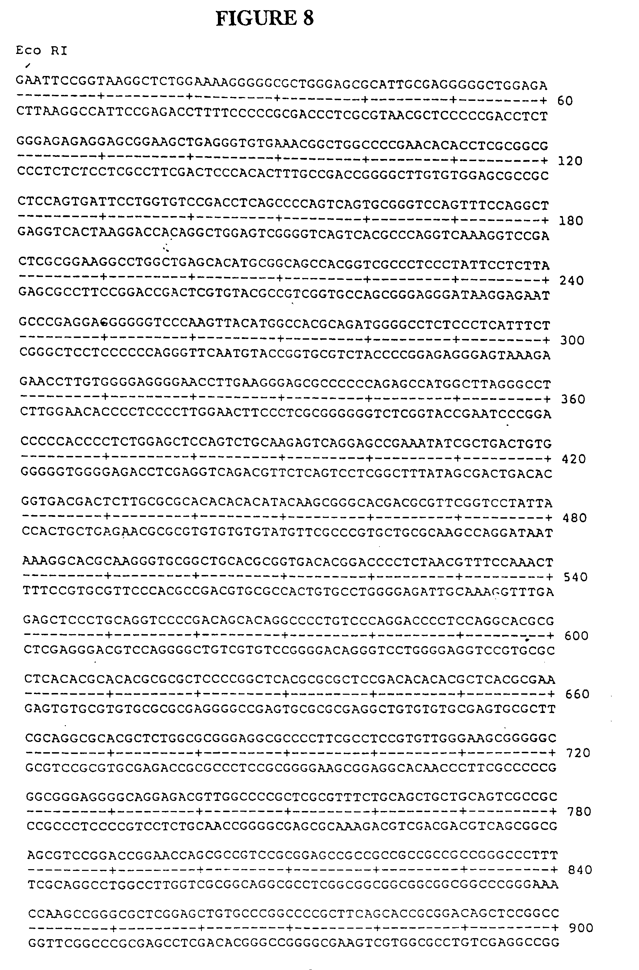

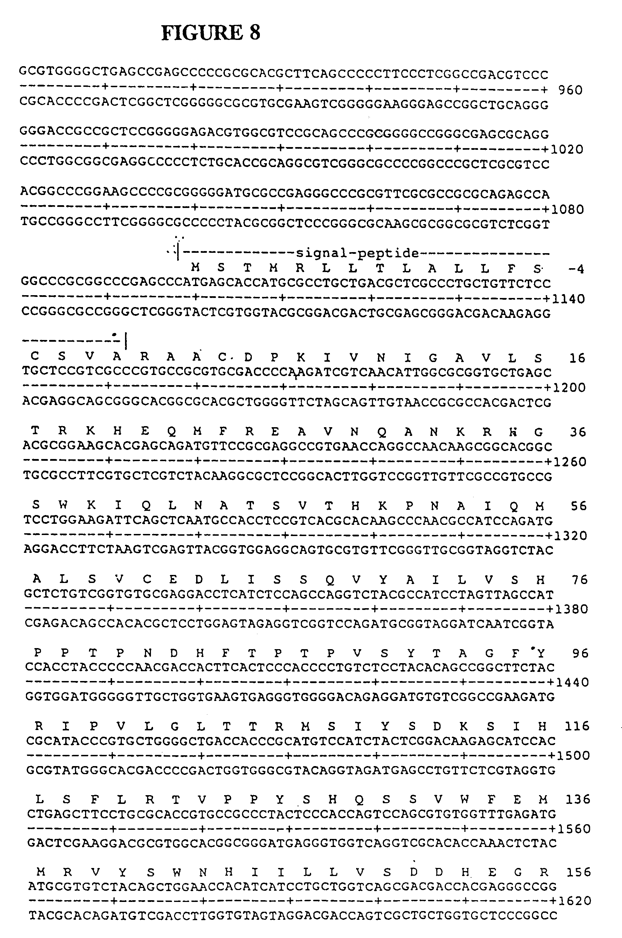

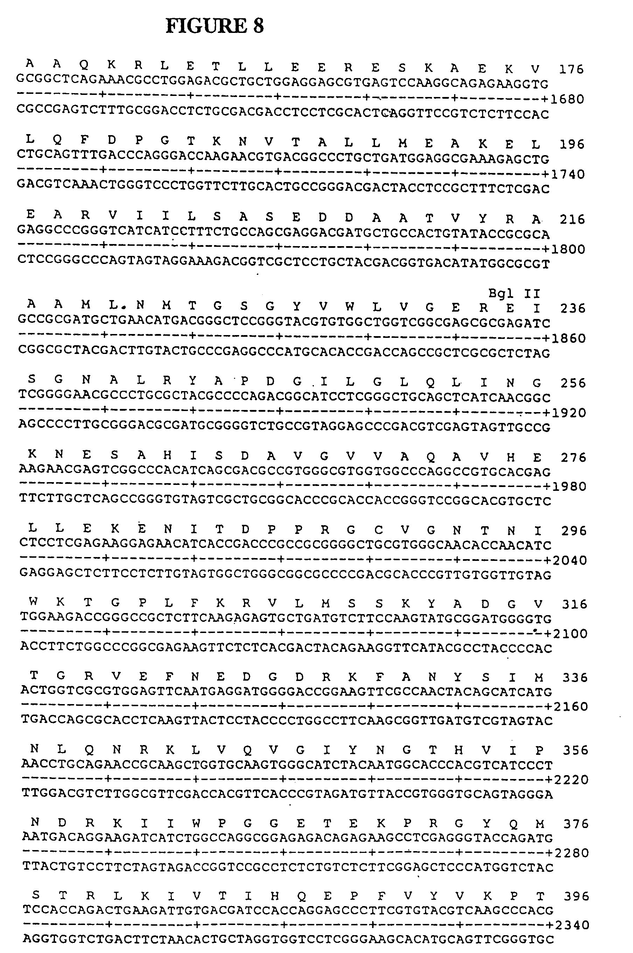

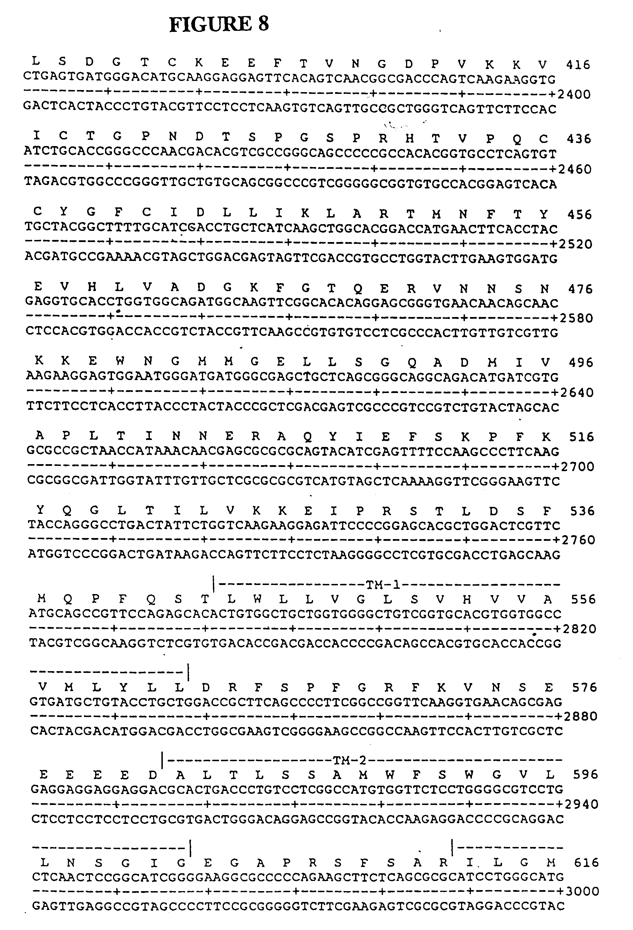

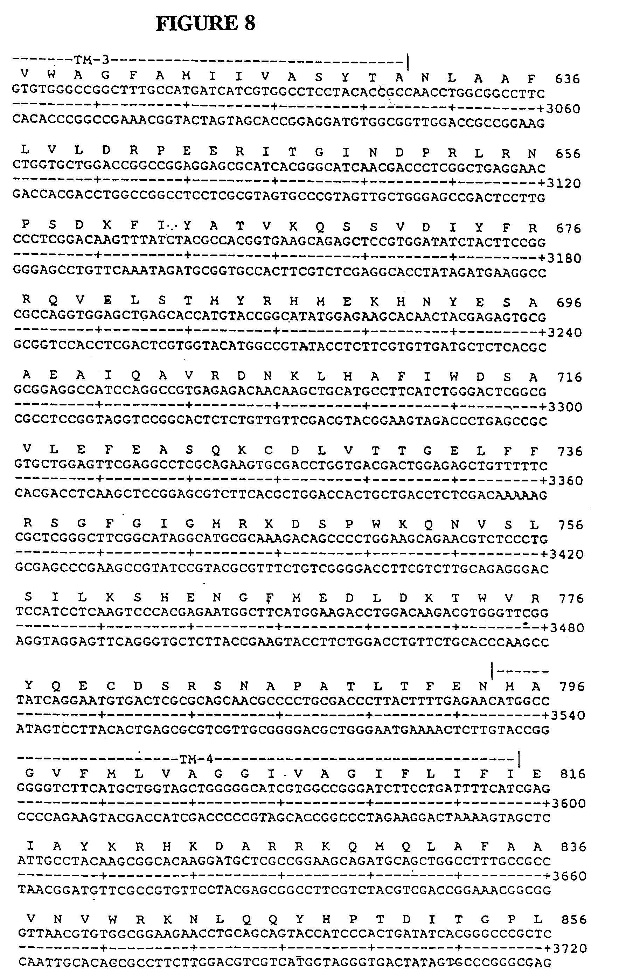

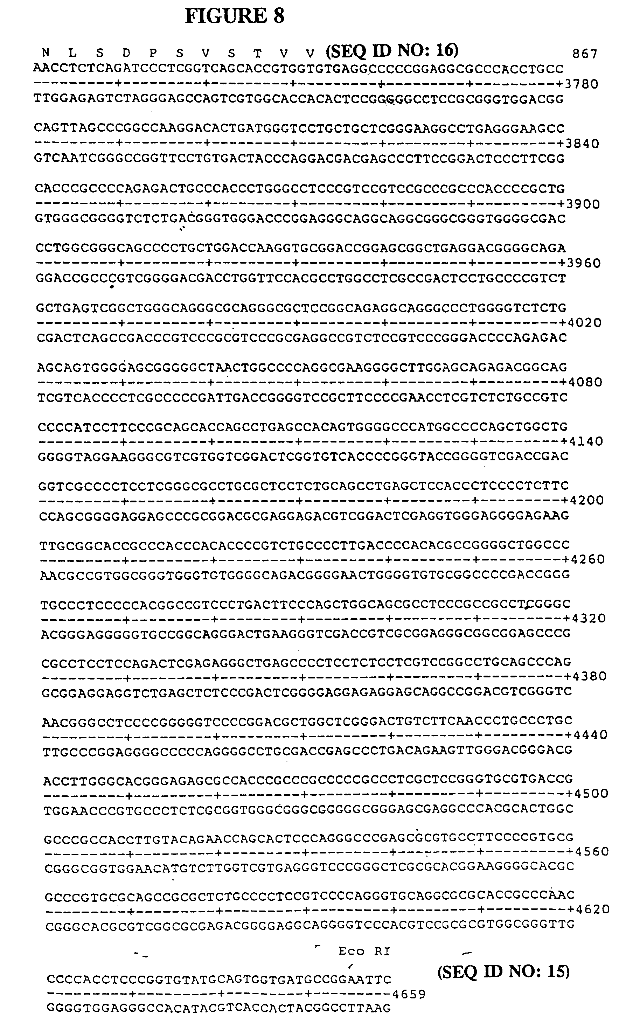

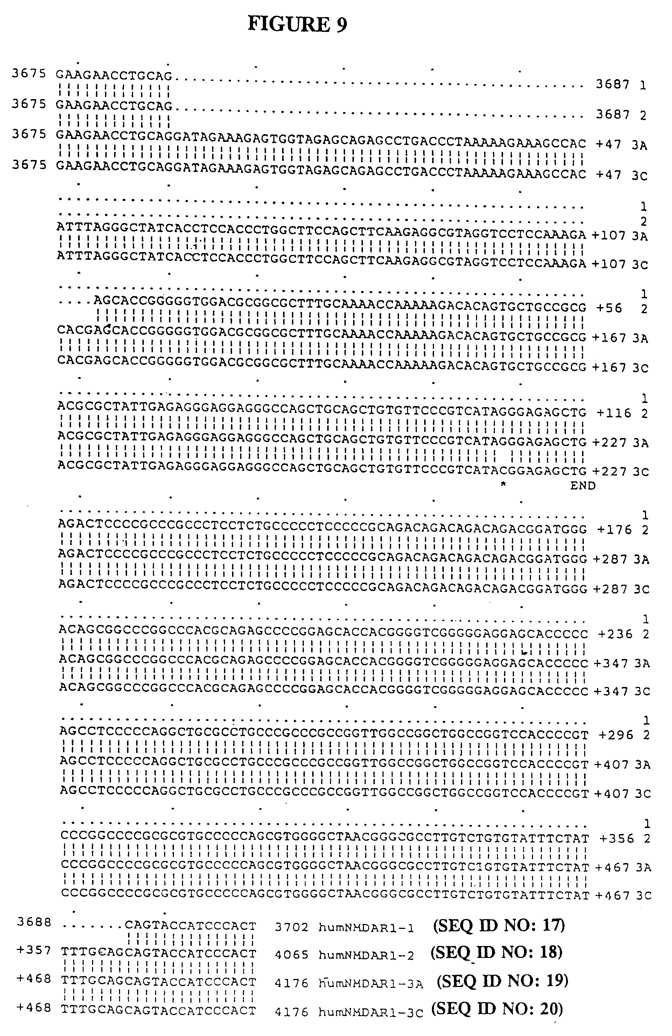

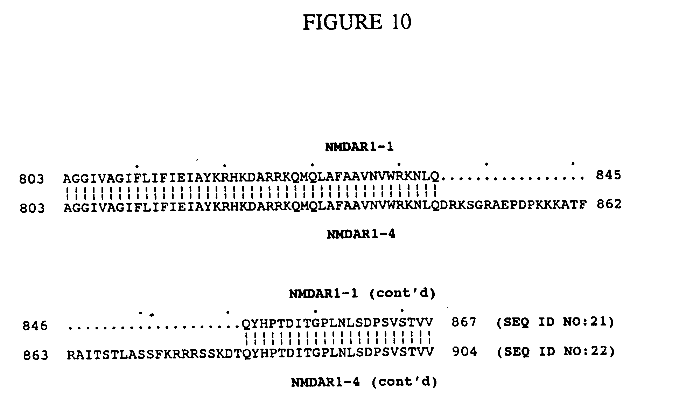

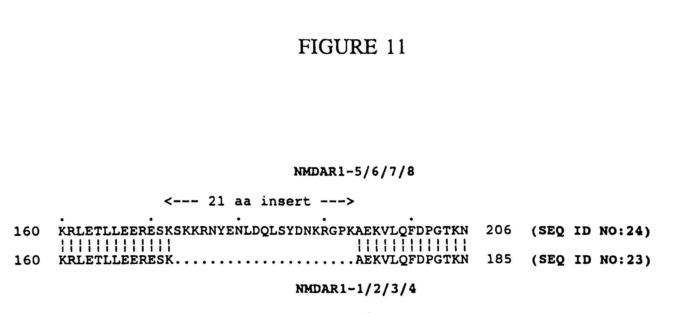

The human modulatory proteins of the present invention are characterized by modulatory activity particularly with respect to human NMDA-type receptors, and more particularly with respect to NMDA receptors of the NMDAR1 family, which are described in detail in co-pending U.S. patent appln. serial no. 07/987,953, the content of which is incorporated herein by reference. The NMDAR1 family of EAA receptors comprises the NMDAR1-1 receptor, the nucleic acid sequence of which is illustrated in Figure 8 (SEQ ID NO: 15), and variants of the NMDAR1-1 receptor which retain an NMDA-type ligand binding profile and which are structurally related to NMDAR1-1, i.e; share at least 99.6% amino acid identity with the 1-845 amino acid region of the NMDAR1-1 receptor, and preferably share 100% amino acid identity in this region. There are both naturally occurring and synthetically derived variants of the human NMDAR1-1 receptor. Naturally occurring variants include, but are not restricted to, receptor variants designated human NMDAR1-2, NMDAR1-3A and NMDAR1-3C, the partial nucleotide sequences of which are illustrated in Figure 9 (SEQ ID NOs: 18, 19 & 20, respectively) and compared to the nucleotide sequence of NMDAR1-1 (SEQ ID NO: 17). Another variant, designated NMDAR1-3B, differs in amino acid sequence from the NMDAR1-1 and NMDAR1-3C receptors by a single amino acid at position 470. This amino acid is lysine in NMDAR1-3B and is glutamic acid in NMDAR1-1 and NMDAR1-3C. This change results from a single base pair change in the codon at position 2560 of NMDAR1-1 and NMDAR1-3C from GAG to AAG in the 3B variant. An NMDAR1-4 variant differs from the NMDAR1-1 receptor by a peptide insert between amino acids 845 and 846 of NMDAR1-1 as illustrated in Fig. 10.
Further variants include NMDAR1-5, NMDAR1-6, NMDAR1-7 and NMDAR1-8, which correspond respectively to the NMDAR1-1, NMDAR1-2, NMDAR1-3 and NMDAR1-4 receptors additionally including a 21 amino acid insert as illustrated in Fig. 11.

One of skill in the art will appreciate that variants of any one of the NMDAR1-1 to NMDAR1-8 receptors which include minor variations from the amino acid sequences thereof, e.g. 1 to 6 amino acid substitutions, deletions or additions, and resulting in receptors retaining the ligand binding profile characteristic of NMDA-type receptors, are also encompassed within the NMDAR1 family of receptors.

Accordingly, the modulatory proteins of the present invention are useful in a heteromeric structure to screen for candidate compounds having the ability to alter the activity of the heteromeric receptor complex. In addition, and despite the understanding that the modulatory family of proteins require a heteromeric structure to function in a modulatory sense, cells producing a modulatory protein homomerically, independent of association with an NMDA receptor, can be exploited for the purpose of screening candidate ligands for the ability to interact specifically therewith. Those compounds found to interact with a modulatory protein represent potential drug compounds which may have agonist or antagonist properties useful in the treatment of neurological disease conditions.

For use in assessing interaction between a modulatory protein, either in homomeric or heteromeric form, and a candidate compound, it is desirable to construct by application of genetic engineering techniques a cell that produces a human modulatory protein in functional form as a heterologous product. The construction of such cell lines is achieved by introducing into a selected host cell a recombinant DNA construct in which DNA coding for a secretable form of the human protein, i.e. a form bearing either its native signal peptide or a functional, heterologous equivalent thereof, is associated with expression controlling elements that are functional in the selected host to drive expression of the modulatory protein-encoding DNA, and thus elaborate the desired modulatory protein. Such cells are herein characterized as having the protein-encoding DNA incorporated "expressibly" therein. The protein-encoding DNA is referred to as "heterologous" with respect to the particular cellular host if such DNA is not naturally found in the particular host.

It is most desirable to use a mammalian cell host to produce the present modulatory proteins due to their human origin; however, other suitably engineered eukaryotic and prokaryotic hosts may also be employed to produce NR2A proteins. Accordingly, bacterial hosts such as E. coli and B. subtilis, fungal hosts such as Aspergillus and yeast and insect cell hosts such as Spodoptera frugiperda, are examples of non-mammalian hosts that may also be used to produce modulatory proteins of the present invention.

The particular cell type selected to serve as host for production of the human modulatory proteins can be any of several cell types currently available in the art. Preferably, where the modulatory protein will be expressed in heteromeric form, i.e. in conjunction with an NMDA receptor, the cell type selected will not be one which in its natural state elaborates a surface receptor that has ion channel activity or that elaborates a protein that is capable of modulating receptor activity, so as to confuse the assay results sought from the engineered cell line. Generally, such problems are avoided by selecting as host a non-neuronal cell type. However, neuronal cells may nevertheless serve as expression hosts, provided that any "background" activity is accounted for in the assay results.

According to one embodiment of the present invention, the cell line selected to serve as host for modulatory protein production is a mammalian cell. Several types of such cell lines are currently available for genetic engineering work, and these include the chinese hamster ovary (CHO) cells for example of K1 lineage (ATCC CCL 61) including the Pro5 variant (ATCC CRL 1281); fibroblast-like cells derived from SV40-transformed African Green monkey kidney of the CV-1 lineage (ATCC CCL 70), of the COS-1 lineage (ATCC CRL 1650) and of the COS-7 lineage (ATCC CRL 1651); murine L-cells, murine 3T3 cells (ATCC CRL 1658), murine C127 cells, human embryonic kidney cells of the 293 lineage (ATCC CRL 1573), human carcinoma cells including those of the HeLa lineage (ATCC CCL 2), and neuroblastoma cells of the lines IMR-32 (ATCC CCL 127), SK-N-MC (ATCC HTB 10) and SK-N-SH (ATCC HTB 11).

A variety of gene expression systems have been adapted for use with these hosts and are now commercially available. Any one of these systems can be exploited to drive expression of modulatory protein-encoding DNA. These systems, available typically in the form of plasmidic vectors, incorporate expression cassettes, the functional components of which include DNA constituting host-recognizable expression controlling sequences which enable expression of the receptor-encoding DNA when linked 5' thereof. The systems further incorporate DNA sequences which terminate expression when linked 3' of the protein-encoding region. Thus, for expression in a selected mammalian cell host, there is generated a recombinant DNA expression construct in which DNA encoding a modulatory protein is linked with expression controlling DNA sequences recognized by the host, including a region 5' of the DNA to drive expression, and a 3' region to terminate expression. The plasmidic vector harbouring the expression construct typically incorporates such other functional components as an origin of replication, usually virally-derived, to permit replication of the plasmid in the expression host, including bacterial hosts such as E. coli. To provide a marker enabling selection of stably transfected recombinant cells, the vector will also incorporate a gene conferring some survival advantage on the transfectants, such as a gene coding for neomycin resistance in which case the transfectants are plated in medium with neomycin.

Included among the various recombinant DNA expression systems that can be used to achieve mammalian cell expression of the modulatory protein-encoding DNA are those that exploit promoters of viruses that infect mammalian cells, such as the promoter from the cytomegalovirus (CMV), the Rous sarcoma virus (RSV), simian virus (SV40), murine mammary tumor virus (MMTV) and others. Also useful to drive expression are promoters such as the long terminal repeat (LTR) of retroviruses, insect cell promoters such as those regulated by temperature, and isolated from Drosophila, as well as mammalian gene promoters such as steroid-inducible promoters and those regulated by heavy metals i.e. the metalothionein gene promoter. In order to achieve expression in bacterial hosts, such as E. coli, expression systems that exploit the expression controlling regions of various E. coli and viral genes can be used to drive NR2A production including the lac gene, the trp gene, and regions of the lambda genome (P_{L} and P_{R}). Expression in yeast can be achieved using the expression-controlling regions of genes such as alcohol dehydrogenase and melibiase, and in Aspergillus, the expression-controlling regions of genes such as alcohol dehydrogenase and glucoamylase may be used. The expression controlling-regions of baculovirus may be used in the case of insect host cells.

For incorporation into the recombinant DNA expression vector, DNA coding for the desired protein, e.g. an NR2A, NR3 or NR4 protein or a variant of one of these proteins, can be obtained by applying selected techniques of gene isolation or gene synthesis. As described in more detail in the examples herein, the present modulatory proteins, and naturally occurring variants thereof, are encoded within the human genome, expressed in human brain tissue, and can therefore be obtained by careful application of conventional gene isolation and cloning techniques. This typically will entail extraction of total messenger RNA from a fresh source of human brain tissue, such as cerebellum, hippocampus or fetal brain tissue, followed by conversion of messenger RNA to cDNA and formation of a library in, for example, a bacterial plasmid, or more typically a bacteriophage. Bacteriophage harbouring fragments of the human DNA are typically grown by plating on a lawn of susceptible E. coli bacteria, such that individual phage plaques or colonies can be isolated. The DNA carried by the phage colony is then typically immobilized on a nitrocellulose or nylon-based hybridization membrane, and then hybridized, under carefully controlled conditions, to a radioactively (or otherwise) labelled nucleotide probe of appropriate sequence to identify the particular phage colony carrying DNA of interest. Typically, the gene or a portion thereof so identified is subcloned into a plasmidic vector for nucleic acid sequence analysis.

Having herein provided the nucleotide sequence of human modulatory proteins, it will be appreciated that automated techniques of gene synthesis and/or amplification can also be performed to generate DNA coding therefor. Because of the length of the DNA encoding the modulatory proteins, application of automated synthesis may require staged gene construction, in which regions of the gene up to about 300 nucleotides in length are synthesized individually and then ligated in correct succession for final assembly. Individually synthesized gene regions can be amplified prior to assembly using polymerase chain reaction (PCR) technology as generally described by Barnett et al. in Nucl. Acids Res. 18:3094, 1990.

The application of automated gene synthesis techniques provides an opportunity to generate sequence variants of naturally occurring members of the modulatory protein gene family. It will be appreciated, due to the degeneracy associated with nucleotide triplet codons, that variant polynucleotides coding for the modulatory proteins herein described can be generated by substituting synonymous codons for those represented in the naturally occurring polynucleotide sequences herein identified, such as those identified in Fig. 1 and Fig. 4. For example, as would be known by one of skill in the art, arginine may be encoded by any one of six codons selected from CGA, CGC, CGG, CGU, AGA and AGG, threonine may be encoded by any one of four codons selected from ACA, ACC, ACG and ACU, while lysine is encoded by two codons, AAA and AAG. In addition, polynucleotides coding for synthetic variants of the modulatory proteins can be generated which, for example, incorporate one or more, e.g. 1-10, single amino acid substitutions, deletions or additions. Since it will for the most part be desirable to retain the modulatory activity of the protein for screening purposes, it is desirable to limit amino acid substitutions to those regions which are less critical for modulatory activity as may be elucidated upon domain mapping of the protein. Such substitutions may include, for example, conservative amino acid substitutions such as isoleucine to leucine, or lysine to arginine.

With appropriate template DNA in hand, the technique of PCR amplification may also be used to directly generate all or part of the final gene. In this case, primers are synthesized which will prime the PCR amplification of the final product, either in one piece, or in several pieces that may be ligated together. This may be via step-wise ligation of blunt-ended, amplified DNA fragments, or preferentially via step-wise ligation of fragments containing naturally occurring restriction endonuclease sites. In this application, it is possible to use either cDNA or genomic DNA as the template for the PCR amplification. In the former case, the cDNA template can be obtained from commercially available or self-constructed cDNA libraries of various human brain tissues, including hippocampus and cerebellum.

Once obtained, the DNA of interest is incorporated for expression into any suitable expression vector using conventional procedures, and host cells are transfected therewith also using conventional procedures which include, for example, DNA-mediated transformation, electroporation, microinjection, or particle gun transformation. Expression vectors may be selected to provide transfected mammalian cell lines that express the DNA of interest either transiently or in a stable manner. For transient expression, host cells are typically transfected with an expression vector harbouring an origin of replication functional in a mammalian cell. For stable expression, such replication origins are unnecessary, but the vectors will typically harbour a gene coding for a product that confers on the transfectants a survival advantage, to enable their selection. Genes coding for such selectable markers include, but are not limited to, the E. coli *gpt* gene which confers resistance to mycophenolic acid, the *neo* gene from transposon Tn5 which confers resistance to the antibiotic G418 and to neomycin, the *dhfr* sequence from murine cells or E. coli which changes the phenotype of DHFR(-) cells into DHFR(+) cells, and the *tk* gene of herpes simplex virus, which makes TK(-) cells phenotypically TK(+) cells. Both transient expression and stable expression can provide transfected cell lines, and membrane preparations derived therefrom, for use in screening assays.

The recombinant techniques described above can be equally applied to EAA receptor production, in particular NMDA receptor production, as set out in the specific examples described herein and using, for example, the DNA sequences provided in Figures 8-11, in the preparation of cells which heteromerically produce an NR2A modulatory protein and an NMDA receptor. In this case, once the appropriate modulatory protein-encoding and NMDA receptor-encoding expression vectors have been prepared, the cells selected for expression are transfected with a mixture of the modulatory protein-encoding and NMDA receptor-encoding expression vectors in the conventional manner.

For use in screening assays, cells transiently expressing the DNA encoding a modulatory protein, and DNA encoding an NMDA receptor, can be stored frozen for later use, but because the rapid rate of plasmid replication will lead ultimately to cell death, usually in a few days, the transfected cells should be used as soon as possible. Such assays may be performed either with intact cells, or with membrane preparations derived from such cells. The membrane preparations typically provide a more convenient substrate for the ligand screening experiments, and are therefore preferred as substrates. To prepare membrane preparations for screening purposes, i.e. ligand binding experiments, frozen intact cells are homogenized while in cold water suspension and a membrane pellet is collected after centrifugation. The pellet is re-suspended and re-centrifuged to remove endogenous ligands that would otherwise compete for binding in the assays. The membranes may then be used as such, or after storage in lyophilized form, in the ligand binding assays. Alternatively, intact, fresh cells harvested about two days after transient transfection or after about the same period following fresh plating of stably transfected cells, can be used for ligand binding assays by the same methods as used for membrane preparations. When cells are used, the cells must be harvested by more gentle centrifugation so as not to damage them, and all washing must be done in a buffered medium, for example in phosphate-buffered saline, to avoid osmotic shock and rupture of the cells.

The binding of a candidate ligand to a selected human modulatory protein of the invention, or a heteromeric receptor complex comprising a modulatory protein and an NMDA receptor, is evaluated typically using a predetermined amount of cell-derived membrane (measured for example by protein determination), generally from about 25 ug to 100 ug. Competitive binding assays will be useful to evaluate the affinity of a candidate ligand for a heteromeric complex relative to glutamate. This competitive binding assay can be performed by incubating a membrane preparation with radiolabelled glutamate, for example [³H]-glutamate, in the presence of unlabelled candidate ligand added at varying concentrations. Following incubation, either displaced or bound radiolabelled glutamate can be recovered and measured to determine the relative binding affinities of the candidate ligand and glutamate for the particular receptor used as substrate. In this way, the affinities of various compounds for the heteromeric complex can be measured. As will be appreciated by one of skill in the art, binding assays such as radioimmunoassays and ELISA can also be used to determine binding affinity of a candidate ligand. Such competitive binding assays cannot be used in the case of a modulatory protein which is expressed homomerically, in a state that does not naturally bind those ligands bound by EAA receptors. Thus, the binding affinity of candidate ligands for a modulatory protein can be determined using a conventional non-competitive type binding assay. Those ligands determined to have an appropriate affinity for the homomeric modulatory protein, i.e. a binding affinity in the micromolar range, and more preferably in the nanomolar range, can then be selected to determine if their binding is specific, and further, if their binding affects the pharmacological and functional characteristics of a heteromeric receptor complex.

The proteins of the present invention are functional in a modulatory context, forming heteromeric receptor complexes, comprising a human modulatory protein and an EAA receptor, which exhibit electrophysiological properties that are distinct from those exhibited by the modulatory protein and NMDA receptor components of the complex. The modulatory proteins are therefore useful, in the established manner, for screening candidate ligands for their ability to modulate the ion channel activity of such heteromeric receptor complexes. The present invention thus further provides, as a ligand screening technique, a method of detecting interaction between a candidate ligand and a human heteromeric receptor complex, comprising the steps of incubating the candidate ligand with a cell that produces a human heteromeric receptor complex, or with a membrane preparation derived therefrom, and then measuring the ligand-induced electrical current across said cell or membrane.

As an alternative to using cells that express a modulatory protein, either homomerically or as a heteromeric receptor complex, ligand characterization may also be performed using cells (for example Xenopus oocytes), that yield functional membrane-bound protein following introduction of messenger RNA coding for the modulatory protein, in the case of homomeric expression, or coding for a heteromeric receptor complex, in the case of heteromeric expression. Thus, DNA encoding a modulatory protein is typically subcloned into a plasmidic vector such that the introduced DNA may be easily transcribed into RNA via an adjacent RNA transcription promoter supplied by the plasmidic vector, for example the T3 or T7 bacteriophage promoters. RNA is then transcribed from the inserted gene in vitro, and isolated and purified therefrom for injection into Xenopus oocytes. In the case of a heteromeric receptor complex, the RNA of the NMDA receptor forming the complex is prepared in the same manner for injection into Xenopus oocytes simultaneously with the RNA encoding the modulatory protein. Following the injection of nanoliter volumes of an RNA solution, the oocytes are left to incubate for up to several days, and are then tested for the ability to respond to a particular ligand molecule supplied in a bathing solution. In the heteromeric case, due to the fact that an active membrane channel is formed through which ions may selectively pass, the response of a particular ligand molecule in the bathing solution may typically be measured as an electrical current utilizing microelectrodes inserted into the cell or placed on either side of a cell-derived membrane preparation using the "patch-clamp" technique.

In addition to using the modulatory protein-encoding DNA to construct cell lines useful for ligand screening, expression of the DNA can, according to another aspect of the invention, be performed to produce fragments of the protein in soluble form, for structure investigation, to raise antibodies and for other experimental uses. It is therefore desirable in the first instance to facilitate the characterization of particular regions of the modulatory protein in quantity and in isolated form, i.e. free from the remainder of the modulatory protein. One region of particular interest with regard to the modulatory function of the modulatory proteins is the extracellular N-terminal region. To prepare a fragment of the N-terminal region, full-length DNA encoding the modulatory protein may be modified by site-directed mutagenesis, to introduce a translational stop codon into the extracellular N-terminal region, immediately 5′ of the first transmembrane domain (TM1). Since there will no longer be produced any transmembrane domain(s) to "anchor" the protein into the membrane, expression of the modified cDNA will result in the secretion, in soluble form, of only the extracellular N-terminal domain. Standard ligand-binding assays may then be performed to ascertain the degree of binding of a candidate compound to the extracellular domain so produced. Alternatively, a translational stop codon may be introduced downstream of the first transmembrane domain to yield a fragment which retains the ability to anchor into the cell membrane. In this way, a heteromeric channel comprising an N-terminal fragment of the modulatory protein can be formed and used to determine the extent of modulatory activity possessed by the fragment. It may of course be necessary, using site-directed mutagenesis, to produce different versions of this extracellular region, or indeed any other extracellular region of the modulatory protein, in order to map the modulatory domain with precision.

Alternatively, it may be desirable to produce other regions of the modulatory protein, for example all or part of the carboxy-terminus thereof. In this case, site-directed mutagenesis and/or PCR-based amplification techniques may readily be used to provide a defined fragment of the cDNA encoding the domain of interest. Once obtained, such DNA fragments can be expressed in the usual manner, either homomerically to determine if the fragment has ligand-binding activity, or heteromerically to determine the extent to which the fragment retains modulatory activity. Conventional peptide synthetic techniques may also be used to make the desired C-terminal fragments or other fragments, e.g. a desired N-terminal fragment as noted above.

It will be appreciated that the production of fragments may be accomplished in a variety of host cells. Mammalian cells such as CHO cells may be used for this purpose, the expression typically being driven by an expression promoter capable of high-level expression, for example, the CMV promoter. Alternately, non-mammalian cells, such as insect Sf9 (Spodoptera frugiperda) cells may be used, with the expression typically being driven by expression promoters of the baculovirus, for example the strong, late polyhedrin protein promoter. Filamentous fungal expression systems may also be used to secrete large quantities of selected domains of the modulatory protein. Aspergillus nidulans for example, with the expression being driven by the alcA promoter, would constitute such an acceptable fungal expression system. In addition to such expression hosts, it will be further appreciated that any prokaryotic or other eukaryotic expression system capable of expressing heterologous genes or gene fragments, whether intracellularly or extracellularly, would be similarly acceptable.

For use particularly in detecting the presence and/or location of a modulatory protein, for example in brain tissue, the present invention also provides, in another of its aspects, antibodies to a human modulatory protein. Such antibodies will also have use as diagnostic agents, e.g. to determine if localized amounts or different forms of modulatory protein in selected tissue types are indicative of a disease condition, and as therapeutic agents, by regulating the modulatory activity of a modulatory protein on an NMDA receptor ion channel, to prevent disease conditions associated with overactive NMDA receptor ion channels. Preferably, for use therapeutically, the antibodies employed are monoclonal antibodies.

To raise such antibodies, there may be used as immunogen either the intact, soluble modulatory protein or an immunogenic fragment thereof, produced in a microbial or mammalian cell host as described above or by standard peptide synthesis techniques. Regions of the modulatory proteins particularly suitable for use as immunogenic fragments include those corresponding in sequence to an extracellular region of the receptor, or a portion of the extracellular region, such as peptides consisting of residues 23-556, or fragments thereof.

The raising of antibodies to the desired protein or immunogenic fragment can be achieved, for polyclonal antibody production, using immunization protocols of conventional design, and any of a variety of mammalian hosts, such as sheep, goats and rabbits. For monoclonal antibody production, immunocytes such as splenocytes can be recovered from the immunized animal and fused, using hybridoma technology, to myeloma cells. The fusion cell products, i.e. hybridoma cells, are then screened by culturing in a selection medium, and cells producing the desired antibody are recovered for continuous growth, and antibody recovery. Recovered antibody can then be coupled covalently to a reporter molecule, i.e. a detectable label, such as a radiolabel, enzyme label, luminescent label or the like, optionally using linker technology established for this purpose.

In detectably labelled form, e.g. radiolabelled form, olignucleotides, including both DNA or RNA, coding for the human modulatory protein and selected regions thereof, may also be used, in accordance with another aspect of the present invention, as hybridization probes for example to identify sequence-related genes resident in the human or other mammalian genomes (or cDNA libraries) or to locate DNA in a specimen, such as brain tissue. This can be done using either the intact coding region, or a fragment thereof, having radiolabelled nucleotides, for example, ³²P-labelled nucleotides, incorporated therein. To identify DNA encoding a modulatory protein in a specimen, it is desirable to use either the full length cDNA coding therefor, or a fragment which is unique thereto. With reference to Figures 1 and 4 and the nucleotide numbering appearing thereon, such nucleotide fragments include those comprising at least about 17 nucleic acids which correspond in sequence to an extracellular region of modulatory protein DNA, e.g. the N-terminus thereof. Examples of suitable nucleotide fragments are the regions spanning nucleotides 8-1830 and 2673-6144 of NR2A-1, and the regions spanning nucleotides 8-1888 and 2732-5976 of NR3-1. Such sequences, and the intact gene itself, may also be used of course to clone related human genes, particularly cDNA equivalents thereof, by standard hybridization techniques.

Embodiments of the present invention are described in detail in the following specific Examples which should not be construed as limiting.

### Example 1 - Isolation of DNA coding for human NR2A-1

A human NR2A DNA probe corresponding to a portion of nucleotide sequence of NR2A-1, namely the nucleotide region 1832-2361 as shown in Fig. 1, was generated by PCR-based amplification of recombinant bacteriophage lambda DNA isolated from an Eco RI-based bacteriophage lambda library of human hippocampus cDNA (obtained from Stratagene Cloning Systems, La Jolla, CA). The following degenerate oligonucleotide primers were used in the PCR amplification: 
1) 5′ GGGGTTTAGATCTGGGT-A/C/G/T-ATGATGTT-C/T-GT-A/C/G/T-ATG 3′
(SEQ ID NO: 25); and
 
2) 5′ GGGGTTTAGATCTGC-A/C/G/T-GC-A/G-TC-A/G-TA-A/G/T-AT-A/G-AA-
A/G/C/T-GC 3′ (SEQ ID NO: 26)
The primers were used at a final concentration of 2 pmol/µl each, in a 50 µl reaction volume (10 mM Tris-HCI, pH 9.0; 50 mM KCl; 1.5 mM MgCl₂) containing 100 ng of recombinant human hippocampus cDNA/bacteriophage lambda DNA, 5 units of Thermus aquaticus DNA polymerase (Promega, Madison, WI) and 0.2 mM of each deoxyribonucleotide Thirty-five cycles of amplification proceeded, with denaturation at 95°C for 1 min., annealing at 50°C for 1 min., and primer extension at 72°C for 1 min., followed by a final cycle at 72°C for 5 min. The 554 bp PCR product was purified from an agarose gel and subcloned into the plasmid vector pT7Blue-T (Novagen, Madison, WI) for DNA sequencing.

The 554 bp human NR2A probe was radiolabeled with [α-³²P]dCTP using the Amersham Megaprime DNA labelling system (Arlington Heights, IL) to a specific activity of 1.0 x 10⁹ cpm/µg. The labelled probe was used to screen approximately 1 x 10⁶ plaques of the Eco RI-based human hippocampus cDNA/ bacteriophage lambda Zap II library identified above and approximately 800,000 plaques of an Eco RI-based human fetal brain cDNA/bacteriophage lambda Zap II library (obtained from Stratagene). Nine positive plaques were identified on replica filters under the following hybridization conditions: 6X SSPE, 50% formamide, 0.5% SDS, 100 µg/ml denatured salmon sperm DNA at 42°C with 1.85 x 10⁶ cpm probe per ml hybridization fluid. The filters were washed twice with 2X SSPE, 0.5% SDS at 25°C for 5 min., followed by a 15 min. wash at 42°C. The filters were exposed to X-ray film (Kodak, Rochester, NY) overnight. The plaques were purified and excised as phagemids according to the supplier's specifications, to generate an insert-carrying Bluescript-SK variant of the phagemid vector.

DNA sequence analysis of the largest overlapping clones (isolated as pBS/FB4A and pBS/H36) revealed a putative ATG initiation codon together with about 155 nucleotides of 5' untranslated (UTR) information and 4,392 nucleotides of amino acid coding information. This analysis also revealed a termination codon as well as 1,590 nucleotides of 3' untranslated information. The entire DNA sequence of the NR2A-1 cDNA is provided in Figure 1.

### Example 2 - Isolation of DNA coding for human NR3-1 and NR4-1

To isolate DNA coding for human NR3-1, the NR2A probe identified in Example 1 was generated and used as described in Example 1 above.

A human NR4 DNA probe corresponding to a portion of nucleotide sequence of NR4, namely the nucleotide region 679-1263 as shown in Fig. 7, was also generated as described above.

The labelled probes were used to screen approximately 1 x 10⁶ plaques of the EcoRI-based human hippocampus cDNA/bacteriophage lambda Zap Il library identified above and approximately 800,000 plaques of an Eco RI-based human fetal brain cDNA/bacteriophage lambda Zap II library (obtained from Stratagene). Fifteen positive plaques were identified and DNA sequence analysis of the largest NR3 overlapping clones (isolated as pBS/FB2C and pBS/FB18) revealed a putative ATG initiation codon together with about 210 nucleotides of 5' untranslated (UTR) information and 4,452 nucleotides of amino acid coding information. This analysis also revealed a termination codon as well as 1,307 nucleotides of 3' untranslated information. The entire DNA sequence of the NR3-1 cDNA is provided in Figure 4.

Partial DNA sequence analysis of the largest NR4 overlapping clones (isolated as pBS/H5A and pBS/H34A) indicated 1,785 nucleotides of amino acid coding information. The DNA sequence of the partial NR4-1 cDNA is provided in Figure 7.

### Example 3 - Isolation of DNA coding for the human NMDAR1-1 receptor

A human NMDAR1 probe corresponding to a portion of nucleotide sequence of NMDAR1-1, namely the nucleotide region 2605-3213 as shown in Fig. 8 (SEQ ID NO: 15), was generated by PCR-based amplification of recombinant bacteriophage lambda DNA isolated from an Eco RI-based bacteriophage lambda library of human hippocampus cDNA (obtained from Stratagene Cloning Systems, La Jolla, CA). The following degenerate oligonucleotide primers were used in the PCR amplification: 
1) 5′ GGGGTTTGGATCCAA-A/G-GA-A/G-TGGAA-C/T-GGNATGATG 3′ (SEQ ID
NO: 27); and
2) 5′ GGGGTTTAAGCTT-C/T-TC-G/A-TA-G/A-TT-G/A-TG-C/T-TT-C/T-TCCAT 3′
(SEQ ID NO: 28)
The primers were used at a final concentration of 5 pmol/µl each, in a 50 µl reaction volume (10 mM Tris-HCl, pH 9.0; 50 mM KCI; 1.5 mM MgCl₂) containing 100 ng of recombinant human hippocampus cDNA/bacteriophage lambda DNA, 5 units of Thermus aquaticus DNA polymerase (Promega, Madison, WI.) and 0.2 mM of each deoxyribonucleotide. Thirty-five cycles of amplification proceeded, with denaturation at 94°C for 1 min., annealing at 51°C for 1 min., and primer extension at 72°C for 1 min., followed by a final cycle at 72°C for 5 min. The 674 bp PCR product was purified from an agarose gel and subcloned into the plasmid vector pTZBlue-T (Novagen, Madison, WI) for DNA sequencing.

The 674 bp human NMDAR1 probe was radiolabelled with [α-³²P]dCTP using the Amersham Megaprime DNA labelling system (Arlington Heights, IL) to a specific activity of 1.0-2.4 x 10⁹ cpm/ug. The labelled probe was used to screen approximately 400,000 plaques of an Eco RI-based human hippocampus cDNA/bacteriophage lambda Zap II library. Thirty-five positive plaques were identified on replica filters under the following hybridization conditions: 6X SSC, 50% formamide, 0.5% SDS, 100 ug/ml denatured salmon sperm DNA at 42°C with 1.85 x 10⁶ cpm probe per ml hybridization fluid. The filters were washed with 2X SSC, 0.5% SDS at 25°C for 5 min., followed by 15 min. washes at 37°C and at 42°C. The filters were exposed to X-ray film (Kodak, Rochester, NY.) overnight. Twenty-eight plaques were purified and excised as phagemids according to the supplier's specifications, to generate an insert-carrying Bluescript-SK variant of the phagemid vector.

DNA sequence analysis of the clone NMDAR1-3C revealed 2,814 nucleotides of amino acid coding information (938 amino acids). The entire DNA sequence of the EcoRI-EcoRI NMDAR1-3C cDNA insert is provided herein by reference to the sequence of NMDAR1-1 set out in Figure 8 and by reference to the sequence differences between NMDAR1-1 and NMDAR1-3C outlined in Figure 9. The NMDAR1-3C cDNA was subcloned into the pcDNA1-Amp mammalian expression vector (to form pcDNA1-Amp/hNR1-3C) using standard techniques such as those described in Example 4 for the subcloning of the NR2A clone into the pcDNA1-Amp vector.

It will be appreciated that the protocol described above can be used to isolate any of the NMDAR1 receptors in accordance with the present invention.

### Example 4 - Construction of genetically engineered cells producing a heteromeric complex of human NR2A-1 and NMDAR1-3C

For transient expression in mammalian cells, cDNA coding for human NR2A-1 was incorporated into the mammalian expression vector pcDNA1-Amp (Invitrogen Corporation, San Diego, CA). This is a multifunctional 5 kbp plasmid vector designed for cDNA expression in eukaryotic systems, and cDNA analysis in prokaryotes. Incorporated on the vector are the CMV immediate early gene promoter and enhancer sequences, SV40 transcription termination and RNA processing signals, SV40 and polyoma virus origins of replication, M13 and ColE1 origins, Sp6 and T7 RNA promoters, and a gene conferring ampicillin resistance. A polylinker is located appropriately downstream of the CMV and T7 promoters.

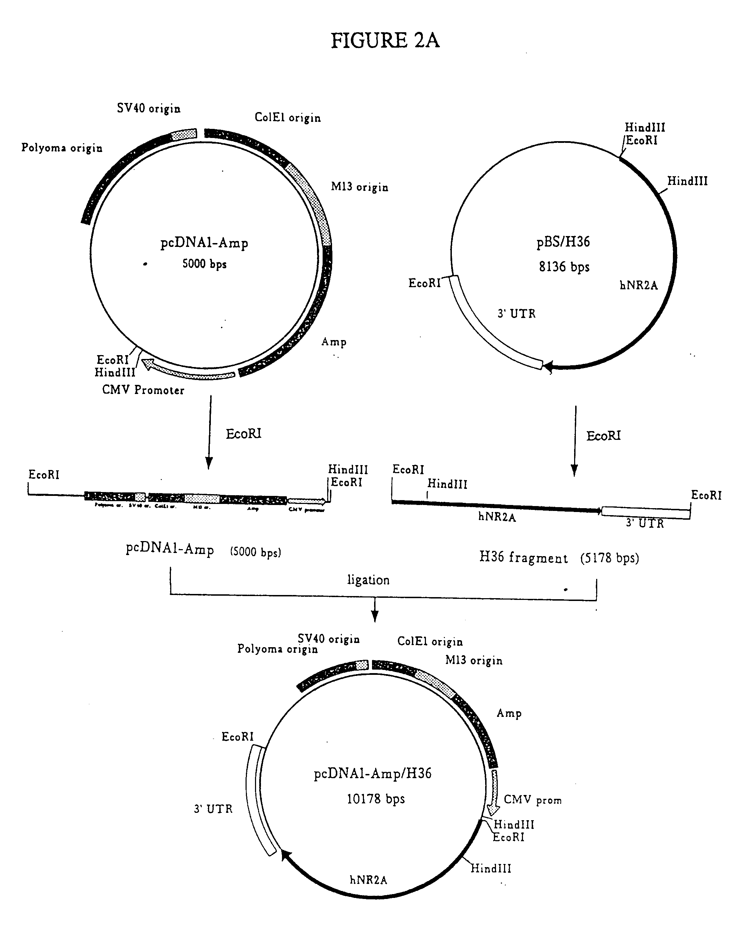

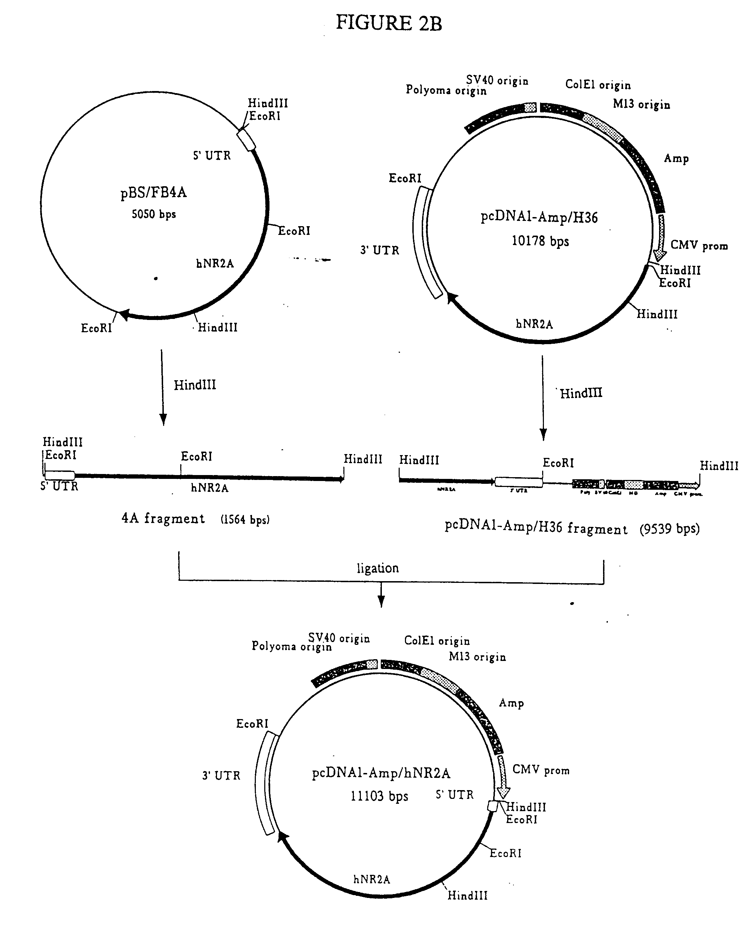

The strategy depicted in Figure 2 was employed to facilitate incorporation of the NR2A-1 cDNA into the expression vector. The H36 cDNA insert was released from pBS/H36 as a 5.2 kbp EcoRI fragment, which was then incorporated at the EcoRI site in the pcDNA1-Amp polylinker. Restriction-endonuclease digestion and DNA sequence analysis across the junctions was performed to confirm proper insert orientation. The FB4A 5' 1.6 kbp Hindlll fragment was released from pBS/FB4A and ligated with the 9.5 kbp HindIII fragment of pcDNA1-Amp/H36. Restriction-endonuclease digestion and DNA sequence analysis across the junctions was performed to confirm proper insert orientation. The resulting plasmid, designated pcDNA1-Amp/hNR2A, was then introduced for transient expression into a selected mammalian cell host, in this case human embryonic kidney cells of the HEK293 lineage (available from the American Type Culture Collection, Rockville, MD; ATCC CRL 1573).

The 11.1 kbp plasmid designated pcDNA1-Amp/hNR2A carrying the NR2A-1 DNA as a 6.1 kbp insert in a 5 kbp pcDNA1-Amp plasmid background, was deposited, under the terms of the Budapest Treaty, with the American Type Culture Collection in Rockville, Maryland, USA on March 16, 1994 and has been assigned accession number ATCC 75708.

For transient expression, HEK293 cells were transfected with approximately 2 µg DNA (as pcDNA1-Amp/hNR2A or pcDNA1-Amp/hNR1-3C) per 10⁵ HEK293 cells, by lipofectin-mediated DNA transfection according to the manufacturer's (Life Technologies Inc., Gaithersburg, MD) specifications. In coexpression experiments, i.e. for heteromeric expression of NR2A-1 and NMDAR1-3C, the HEK293 cells were similarly transfected with 3 µg of a DNA mixture containing pcDNA1-Amp/hNR2A and pcDNA1-Amp/hNR1-3C. Briefly, HEK293 cells were plated at a density of 10⁵ cells/dish and then grown for 24 hours in 10% FBS-supplemented MEM medium (Life Technologies Inc., Gaithersburg, MD). The medium was then removed and cells were washed in OPTI-MEM I medium (Life Technologies Inc;) lacking FBS, prior to transfection. A transfection solution (100 µl) containing 5-7.5 µl of lipofectin and DNA was then applied to the cells. After incubation for 6 hours at 37°C, cells were washed as previously described and then allowed to grow for 36-48 hours in 10% FBS-supplemented MEM medium containing 50 µM DL-AP5 (2-amino-5-phosphonopentanoic acid) prior to electrophysiological recording.

In a like manner, stably transfected cell lines can also be prepared using various cell types as host: HEK293, CHO K1 or CHO Pro5. To construct these cell lines, cDNA coding for NR2A-1 is incorporated into the mammalian expression vector pRc/CMV (Invitrogen Corp., San Diego, CA) which enables stable expression. Insertion of the cDNA places it under the expression control of the CMV promoter and upstream of the polyadenylation site and terminator of the bovine growth hormone gene, and into a vector background comprising the neomycin resistance gene (driven by the SV40 early promoter) as selectable marker. To introduce plasmids constructed as described above, the host cells are first seeded at a density of 5 x 10⁵ cells/dish in 10% FBS-supplemented MEM medium. After growth for 24 hours, fresh medium is added to the plates and three hours later, the cells are transfected using the lipofectin-mediated DNA transfection procedure according to the manufacturers specifications. Cells resistant to neomycin are selected in 10% FBS-supplemented MEM medium containing G418 (1 mg/ml). Individual colonies of G418-resistant cells are isolated about 2-3 weeks later, clonally selected and then propagated for assay purposes.

### Example 5 - Construction of genetically engineered cells producing a heteromeric complex of human NR3-1 and NMDAR1-3C

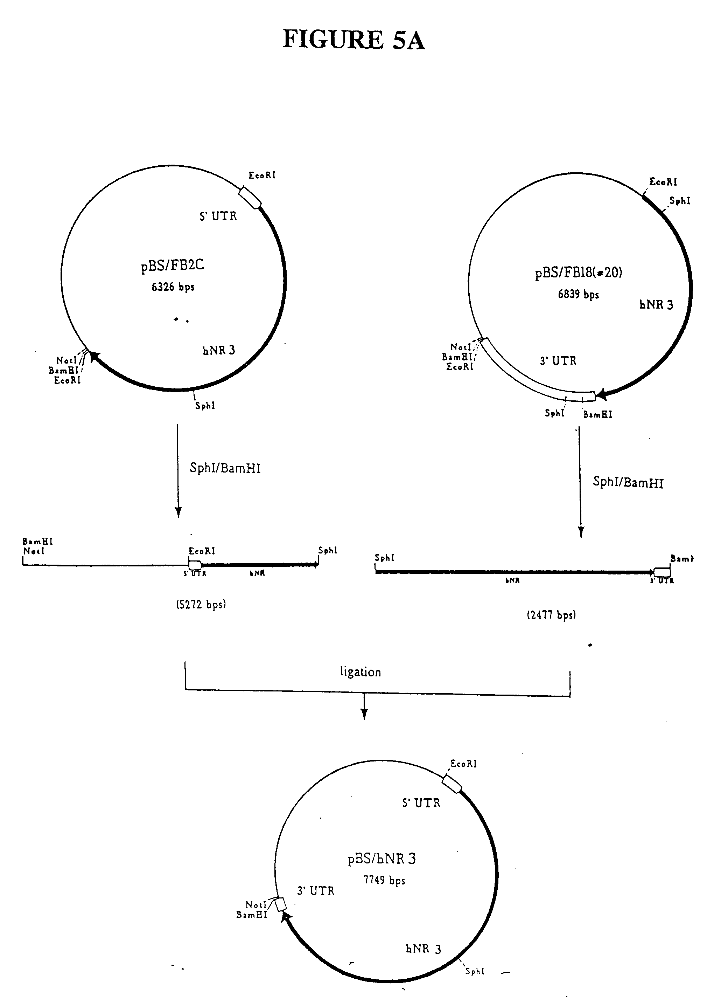

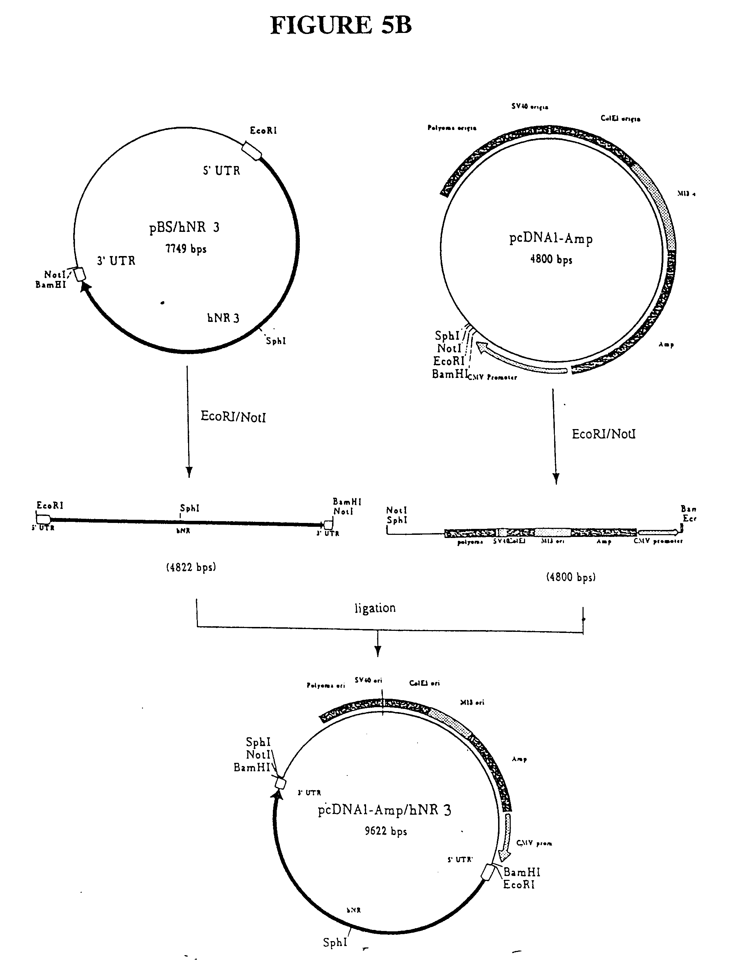

The strategy depicted in Figure 5 was employed to facilitate incorporation of the NR3-1 cDNA into the expression vector. The FB2C 5' 5.3 kbp BamHI/Sphl fragment was released from pBS/FB2C and ligated with the 2.5 kbp BamHI/Sphl fragment of pBS/FB18(#20). Restriction-endonuclease digestion was performed to confirm proper insert orientation. The resulting plasmid was termed pBS/hNR3. The 4.8 kbp EcoRI/Notl fragment of pBS/hNR3 was incorporated at the EcoRI/Notl site in the pcDNA1-Amp polylinker. Restriction-endonuclease digestion and DNA sequence analysis was performed to confirm proper insert orientation. The resulting plasmid, designated pcDNA1-Amp/hNR3, was then introduced for transient expression into a selected mammalian cell host, in this case human embryonic kidney cells of the HEK293 lineage (available from the American Type Culture Collection, Rockville, MD; ATCC CRL 1573).

The 7.8 kbp plasmid designated pBS/hNR3 carrying the NR3-1 DNA as a 4.8 kbp insert in a 3 kbp pBS plasmid background, was deposited, under the terms of the Budapest Treaty, with the American Type Culture Collection in Rockville, Maryland, USA on June 3, 1994 under accession number ATCC 75799.

Transiently and stably transfected cells are prepared as described in Example 4 above.

### Example 6 - Electrophysiological characterization of the NR2A-1/NMDAR1-3C complex

Standard whole-cell voltage-clamp (Axopatch 1B, Axon Instruments, Foster City, CA) techniques were used to record 100 µM NMDA-evoked currents in HEK293 cells transiently transfected as described in Example 4 and expressing hNR2A-1 heteromerically with the NMDAR1-3C receptor. The cells were rinsed prior to recording with a solution of 130 mM NaCI, 5.4 mM KCl, 1.8 mM CaCl₂, 10 µM glycine, 5 mM HEPES, pH 7.2 (300 mOsm.). Single electrode, voltage-clamp recordings were carried out using thin-walled borosilicate glass electrodes (WPI-TW150-F4, WPI Inc., Sarasota, FL) filled with an intracellular solution of 140 mM CsCI, 1 mM MgCl₂, 10 mM EGTA, 10 mM HEPES, pH 7.2 (adjusted with 1 M CsOH). NMDA application using a computer controlled array of perfusion barrels allowed for fast application and continuous perfusion with control or 1 mM Mg²⁺-containing solutions (lag < 50 milliseconds).

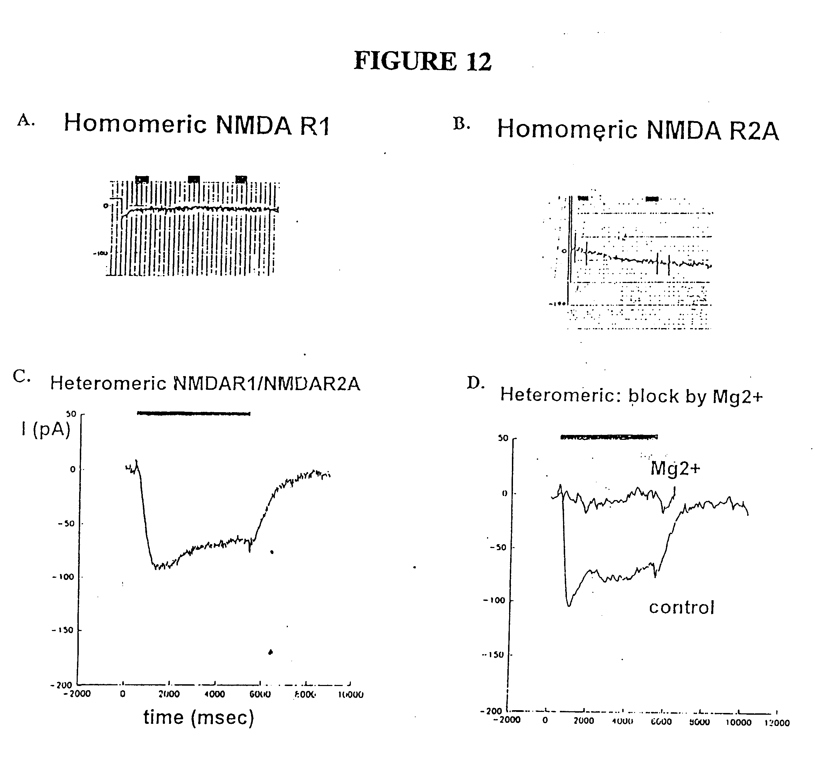

The results of the electrophysiological characterization are depicted in Figures 12A - 12D. Points at which NMDA was applied are indicated with black bars above the recordings. No NMDA-induced depolarizations were observed in HEK293 cells transiently transfected with NMDAR1-3C alone (Figure 12A) or with NR2A alone (Figure 12B). NMDA-induced depolarizations were, however, observed in HEK293 cells transiently transfected with both NR2A and NMDAR1-3C (Figure 12C). These latter currents were blocked by 1 mM MgCl₂, a result which is characteristic of NMDA-gated ion channels, as illustrated in Fig. 12D.

This electrophysiological characterization indicates that the NR2A/NMDA receptor heteromeric complex functions in an authentic manner, and can therefore be used to reliably predict the functional "signature" of its non-recombinant counterpart from intact human brain. These features make the recombinant receptor especially useful for selecting and characterizing ligand compounds which bind to or otherwise modulate the receptor, and/or for selecting and characterizing compounds which may act by displacing other ligands from the receptor. The isolation of the NR2A protein in a pure form, and its expression with an NMDA receptor as a single, homogenous complex, therefore frees the electrophysiological assay from the lack of precision introduced when complex receptor preparations from human and non-human brains are used to attempt such characterizations.

It will be appreciated that the protocol described above can be used to determine the electrophysiological characteristics of other NR2A/NMDA heteromeric receptor complexes, such as for example, the NR2A-1/NMDAR1-1 complex.

### Example 7 - Electrophysiological characterization NR3-1/NMDAR1-3C complex

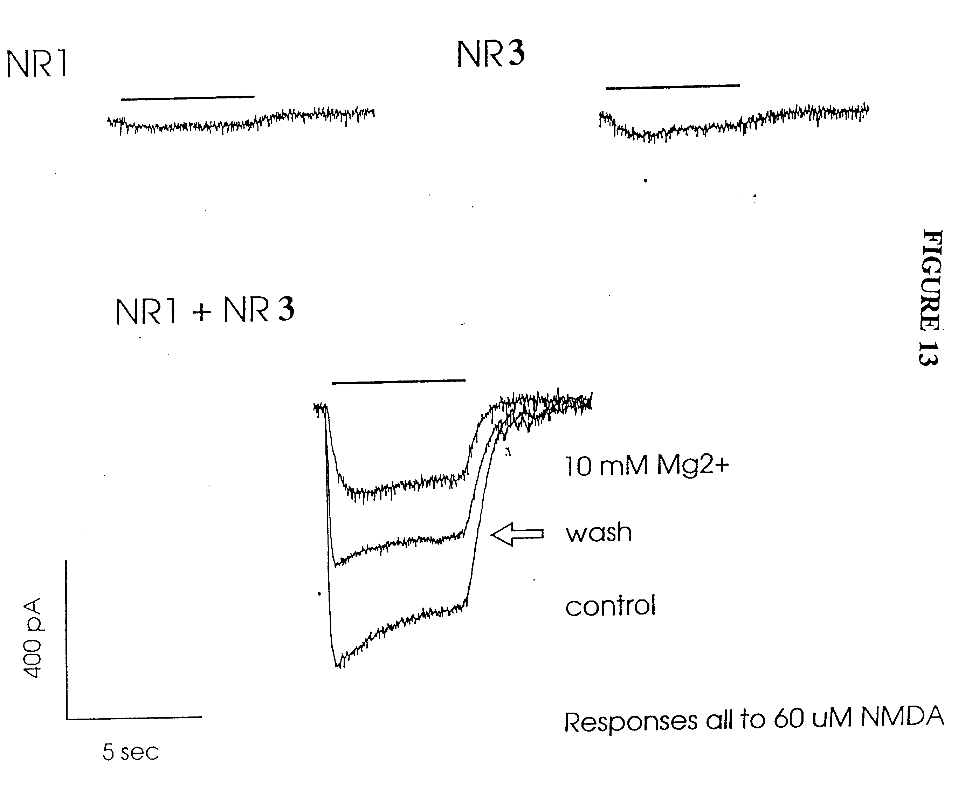

The procedure described above was used to determine the electrophysiological properties of the NR3-1/NMDAR1-3C complex. The results of this determination are illustrated graphically in Fig. 13 and correlate with the results obtained using the NR2A-1/NMDAR1-3C complex.

### Example 8 - Ligand-binding Assays on Heteromeric Complexes

Frozen transfected cells, prepared as described in Example 3 above, are resuspended in ice-cold distilled water, sonicated for 5 seconds, and centrifuged for 10 minutes at 50,000 x g. The supernatant is discarded and the membrane pellet is stored frozen at -70°C.

Cell membrane pellets are resuspended in ice cold 50 mM Tris-HCl, pH 7.55, and centrifuged again at 50,000 x g for 10 minutes in order to remove endogenous glutamate that would otherwise compete for binding. The pellets are resuspended in ice cold 50 mM Tris-HCl, pH 7.55, and used for the binding experiments described below. Protein concentrations are determined using the Pierce reagent with BSA as an internal standard.

Binding assays are performed using a 25-100 µg protein equivalent of the cell membrane preparation, and a selected radiolabeled ligand. In particular, for MK-801-binding assays, incubation mixtures consist of 20 nM (+)-[3-³H]MK-801 (30 Ci/mmole), 20 µM glycine, and 1 mM L-glutamate in cold incubation buffer (50 mM Tris-HCl, pH 7.55) at a final volume of 250 µl. Non-specific binding is determined in the presence of 1 mM (+)MK-801. For glutamate binding assays, incubation mixtures consist of 30 nM [3,4-³H]-L-glutamate (47.3 Ci/mmole) in cold incubation buffer at a final volume of 250 µl. Non-specific binding is determined in the presence of 1 mM L-glutamate and displacement is determined in the presence of 1 mM NMDA, 1 mM kainate, or 1 mM AMPA. The reaction mixtures are incubated on ice for 60 minutes in plastic mini-vials. Bound and free ligand are separated by centrifugation for 30 minutes at 50,000 x g. The pellets are washed three times in 4 ml of the cold incubation buffer, and then 4 ml of Beckman Ready-Protein Plus scintillation cocktail was added for liquid scintillation counting.

It will be appreciated that the protocol described above can be used to determine the pharmacological characteristics of other modulatory/NMDA heteromeric receptor complexes, such as for example, the NR3A-1/NMDAR1-1 complex.

### Example 9 - Ligand-binding Assay for the Homomeric Expression of Modulatory Proteins

Frozen transfected cells, prepared as described in Example 3 above and expressing NR2A-1 in the absence of an NMDA receptor, are resuspended in ice-cold distilled water, sonicated for 5 seconds, and centrifuged for 10 minutes at 50,000 x g. The supernatant is discarded and the membrane pellet is stored frozen at -70°C.

Cell membrane pellets are resuspended in ice cold 50 mM Tris-HCl, pH 7.55, and centrifuged again at 50,000 x g for 10 minutes in order to remove endogenous ligands that might otherwise compete for binding. The pellets are resuspended in ice cold 50 mM Tris-HCl, pH 7.55, and used for the binding experiments described below. Protein concentrations are determined using the Pierce reagent with BSA as an internal standard.

Binding assays are performed using a 25-100 µg protein equivalent of the cell membrane preparation, and a selected radiolabeled ligand in cold incubation buffer (50 mM Tris-HCI, pH 7.55) at a final volume of 250 µl. Non-specific binding is determined in the presence of the unlabeled ligand. The reaction mixtures are incubated on ice for 60 minutes in plastic mini-vials. Bound and free ligand are separated by centrifugation for 30 minutes at 50,000 x g. The pellets are washed three times in 4 ml of the cold incubation buffer, and then 4 ml of Beckman Ready-Protein Plus scintillation cocktail are added for liquid scintillation counting.

Having determined that the selected ligand binds specifically to NR2A-1, i.e. that unlabelled ligand competes for binding with the labelled form of that ligand, and that the binding is saturable, the ligand is then tested for its ability to affect the heteromeric expression of NR2A-1, i.e. when coexpressed with an NMDA receptor as described above. Appropriate experiments for this purpose include the ligand binding experiment described in Example 5, and the electrophysiological characterization described in Example 4.

### Example 10 - Isolation and Cloning of Variant Modulatory Proteins

The procedures described in Examples 1, 2, 4 and 5 for isolating and cloning the NR2A-1 protein are applied equally for the isolation and cloning of NR2A-2, NR3-2 and other naturally occuring variants of the parent modulatory proteins, particularly in view of the high sequence homology seen between the parent modulatory proteins and those variants found to date.

Moreover, the electrophysiological and ligand-binding assays described in Examples 6-9, respectively, are used in the manner described to determine the electrophysiological and ligand binding characteristics of such variants.

## Claims

1. An isolated polynucleotide comprising a region that codes for a human modulatory protein selected from the group consisting of: the NR2A-1 protein; a variant that shares greater than 95% amino acid identity with the NR2A-1 protein; the NR3-1 protein; a variant that shares greater than 98.5% amino acid identity with the NR3-1 protein; the NR4-1 protein; and a functional fragment of one of said proteins which retains the modulatory activity thereof.

2. The isolated polynucleotide of claim 1, which comprises a region that codes for the NR2A-1 protein, the NR3-1 protein or the NR4-1 protein.

3. The isolated polynucleotide of claim 1, which comprises a region that codes for the human NR2A-2 protein or the human NR3-2 protein.

4. A recombinant DNA construct having incorporated therein a polynucleotide as defined in any one of claims 1 to 3.

5. A cell that has been engineered genetically to produce a human modulatory protein or a fragment thereof, said cell having incorporated expressibly therein a heterologous polynucleotide as defined in any one of claims 1 to 3.

6. A membrane preparation derived from a cell as defined in claim 5.

7. A cell that has been engineered genetically to produce a heteromeric human receptor complex comprising a modulatory protein and an NMDA receptor, said cell having incorporated expressibly therein a heterologous polynucleotide as defined in any one of claims 1 to 3 and a heterologous polynucleotide encoding a human NMDA receptor.

8. The cell of claim 7, wherein the human NMDA receptor is the NMDAR1-1, NMDAR1-2, NMDAR1-3, NMDAR1-4, NMDAR1-5, NMDAR1-6, NMDAR1-7 or NMDAR1-8 receptor.

9. A process for obtaining a substantially homogeneous 5 source of a human modulatory protein, which comprises the steps of culturing cells having incorporated expressibly therein a heterologous polynucleotide as defined in any one of claims 1 to 3 and then recovering the cultured cells.

10. A method of assaying a candidate ligand for interaction with a human modulatory protein, which comprises the steps of incubating the candidate ligand under appropriate conditions with a cell as defined in claim 5, or with a membrane preparation as defined in claim 6, and then determining the extent of binding between said protein and the candidate ligand.

11. A method of assaying a candidate ligand for interaction with a human heteromeric receptor comprising a modulatory protein and an NMDA receptor, which comprises the steps of incubating the candidate ligand under appropriate conditions with a cell as defined in claim 7 or claim 8, or with a membrane preparation derived therefrom, and then determining the extent of binding between the complex and the candidate ligand, or determining ligand-induced electrical current across said cell or membrane.

12. A human modulatory protein selected from the group consisting of the NR2A-1, NR2A-2, NR3-1, NR3-2 and NR4-1 proteins, in a form essentially free from other proteins of human origin.

13. A functional fragment of a modulatory protein as defined in claim 12.

14. An antibody which binds a human modulatory protein as defined in claim 12.

15. An oligonucleotide comprising at least about 17 nucleic acids which hybridizes with a polynucleotide as defined in any one of claims 1 to 3.


SEQUENCE LISTING

(1) GENERAL INFORMATION:

(i) APPLICANT:
(A) ADDRESSEE: Allelix Biopharmaceuticals, Inc.
(B) STREET: 6850 Goreway Drive
(C) CITY: Mississauga
(D) STATE: Ontario
(E) COUNTRY: CANADA
(F) ZIP: L4V 1V7

(ii) CORRESPONDENCE ADDRESS:
(A) ADDRESSEE: Carpmaels & Ransford
(B) STREET: 43, Bloomsbury Square
(C) CITY: London
(D) STATE:
(E) COUNTRY: United Kingdom
(F) ZIP: WC1A 2RA

(iii) TITLE OF INVENTION: MODULATORY PROTEINS OF HUMAN CNS
RECEPTORS

(iv) NUMBER OF SEQUENCES: 28

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: WordPerfect 5.1

(vi) CURRENT APPLICATION DATA:
(A) APPLICATION NUMBER: NOT YET ASSIGNED
(B) FILING DATE: CONCURRENTLY HEREWITH

(vii) ATTORNEY INFORMATION:
(A) NAME: Mercer, Christopher Paul
(B) REFERENCE: P13570EP

(iix) PRIOR APPLICATION DATA:
(A) APPLICATION NUMBER: 08/217,704
(B) FILING DATE: 25-MAR-1994

(iix) PRIOR APPLICATION DATA:
(A) APPLICATION NUMBER: 08/264,578
(B) FILING DATE: 23-JUN-1994

(2) INFORMATION FOR SEQ ID NO:1:

(i) SEQUENCE CHARACTERISTICS:
(A) LENGTH: 6151 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: CDS
(B) LOCATION: 163..4554

(xi) SEQUENCE DESCRIPTION: SEQ ID NO:1:

GAATTCCGAC AGCGCGGGAC AGCCAGGGGA GCGCGCTGGG GCCGCAGCAT GCGGGAACCC 60

GCTAAACCCG GTGGCTGCTG AGGCGGCCGA GATGCTCGTG CGCGCAGCGC GCCCCACTGC 120

ATCCTCGACC TTCTCGGGCT ACAGGGACCG TCAGTGGCGA CT ATG GGC AGA GTG 174

1

GGC TAT TGG ACC CTG CTG GTG CTG CCG GCC CTT CTG GTC TGG CGC GGT 222

5                   10                  15                  20
CCG GCG CCG AGC GCG GCG GCG GAG AAG GGT CCC CCC GCG CTA AAT ATT 270

                25                  30                  35

GCG GTG ATG CTG GGT CAC AGC CAC GAC GTG ACA GAG CGC GAA CTT CGA 318

            40                  45                  50

ACA CTG TGG GGC CCC GAG CAG GCG GCG GGG CTG CCC CTG GAC GTG AAC 366

        55                  60                  65

GTG GTA GCT CTG CTG ATG AAC CGC ACC GAC CCC AAG AGC CTC ATC ACG 414

    70                  75                  80

CAC GTG TGC GAC CTC ATG TCC GGG GCA CGC ATC CAC GGC CTC GTG TTT 462

85                  90                  95                  100

GGG GAC GAC ACG GAC CAG GAG GCC GTA GCC CAG ATG CTG GAT TTT ATC 510

                105                 110                 115

TCC TCC CAC ACC TTC GTC CCC ATC TTG GGC ATT CAT GGG GGC GCA TCT 558

            120                 125                 130

ATG ATC ATG GCT GAC AAG GAT CCG ACG TCT ACC TTC TTC CAG TTT GGA 606

        135                 140                 145

GCG TCC ATC CAG CAG CAA GCC ACG GTC ATG CTG AAG ATC ATG CAG GAT 654

    150                 155                 160

TAT GAC TGG CAT GTC TTC TCC CTG GTG ACC ACT ATC TTC CCT GGC TAC 702

165                 170                 175                 180

AGG GAA TTC ATC AGC TTC GTC AAG ACC ACA GTG GAC AAC AGC TTT GTG 750

                185                 190                 195

GGC TGG GAC ATG CAG AAT GTG ATC ACA CTG GAC ACT TCC TTT GAG GAT 798

            200                 205                 210

GCA AAG ACA CAA GTC CAG CTG AAG AAG ATC CAC TCT TCT GTC ATC TTG 846

        215                 220                 225

CTC TAC TGT TCC AAA GAC GAG GCT GTT CTC ATT CTG AGT GAG GCC CGC 894

    230                 235                 240

TCC CTT GGC CTC ACC GGG TAT GAT TTC TTC TGG ATT GTC CCC AGC TTG 942

245                 250                 255                 260

GTC TCT GGG AAC ACG GAG CTC ATC CCA AAA GAG TTT CCA TCG GGA CTC 990

                265                 270                 275

ATT TCT GTC TCC TAC GAT GAC TGG GAC TAC AGC CTG GAG GCG AGA GTG 1038

            280                 285                 290

AGG GAC GGC ATT GGC ATC CTA ACC ACC GCT GCA TCT TCT ATG CTG GAG 1086

        295                 300                 305

AAG TTC TCC TAC ATC CCC GAG GCC AAG GCC AGC TGC TAC GGG CAG ATG 1134

    310                 315                 320

GAG AGG CCA GAG GTC CCG ATG CAC ACC TTG CAC CCA TTT ATG GTC AAT 1182

325                 330                 335                 340

GTT ACA TGG GAT GGC AAA GAC TTA TCC TTC ACT GAG GAA GGC TAC CAG 1230

                345                 350                 355

GTG CAC CCC AGG CTG GTG GTG ATT GTG CTG AAC AAA GAC CGG GAA TGG 1278

            360                 365                 370

GAA AAG GTG GGC AAG TGG GAG AAC CAT ACG CTG AGC CTG AGG CAC GCC 1326

        375                 380                 385

GTG TGG CCC AGG TAC AAG TCC TTC TCC GAC TGT GAG CCG GAT GAC AAC 1374

    390                 395                 400

CAT CTC AGC ATC GTC ACC CTG GAG GAG GCC CCA TTC GTC ATC GTG GAA 1422

405                 410                 415                 420

GAC ATA GAC CCC CTA ACC GAG ACG TGT GTG AGG AAC ACC GTG CCA TGT 1470

                425                 430                 435

CGG AAG TTC GTC AAA ATC AAC AAT TCA ACC AAT GAG GGG ATG AAT GTG 1518

            440                 445                 450

AAG AAA TGC TGC AAG GGG TTC TGC ATT GAT ATT CTG AAG AAG CTT TCC 1566

        455                 460                 465

AGA ACT GTG AAG TTT ACT TAC GAC CTC TAT CTG GTG ACC AAT GGG AAG 1614

    470                 475                 480

CAT GGC AAG AAA GTT AAC AAT GTG TGG AAT GGA ATG ATC GGT GAA GTG 1662

485                 490                 495                 500

GTC TAT CAA CGG GCA GTC ATG GCA GTT GGC TCG CTC ACC ATC AAT GAG 1710

                505                 510                 515

GAA CGT TCT GAA GTG GTG GAC TTC TCT GTG CCC TTT GTG GAA ACG GGA 1758

            520                 525                 530

ATC AGT GTC ATG GTT TCA AGA AGT AAT GGC ACC GTC TCA CCT TCT GCT 1806

        535                 540                 545

TTT CTA GAA CCA TTC AGC GCC TCT GTC TGG GTG ATG ATG TTT GTG ATG 1854

    550                 555                 560

CTG CTC ATT GTT TCT GCC ATA GCT GTT TTT GTC TTT GAA TAC TTC AGC 1902

565                 570                 575                 580

CCT GTT GGA TAC AAC AGA AAC TTA GCC AAA GGG AAA GCA CCC CAT GGG 1950

                585                 590                 595

CCT TCT TTT ACA ATT GGA AAA GCT ATA TGG CTT CTT TGG GGC CTG GTG 1998

            600                 605                 610

TTC AAT AAC TCC GTG CCT GTC CAG AAT CCT AAA GGG ACC ACC AGC AAG 2046

        615                 620                 625

ATC ATG GTA TCT GTA TGG GCC TTC TTC GCT GTC ATA TTC CTG GCT AGC 2094

    630                 635                 640

TAC ACA GCC AAT CTG GCT GCC TTC ATG ATC CAA GAG GAA TTT GTG GAC 2142

645                 650                 655                 660

CAA GTG ACC GGC CTC AGT GAC AAA AAG TTT CAG AGA CCT CAT GAC TAT 2190

                665                 670                 675

TGC CCA CCT TTT CGA TTT GGG ACA GTG CCT AAT GGA AGC ACG GAG AGA 2238

            680                 685                 690

AAC ATT CGG AAT AAC TAT CCC TAC ATG CAT CAG TAC ATG ACC AAA TTT 2286

        695                 700                 705

AAT CAG AAA GGA GTA GAG GAC GCC TTG GTC AGC CTG AAA ACG GGG AAG 2334

    710                 715                 720

CTG GAC GCT TTC ATC TAC GAT GCC GCA GTC TTG AAT TAC AAG GCT GGG 2382

725                 730                 735                 740

AGG GAT GAA GGC TGC AAG CTG GTG ACC ATC GGG AGT GGG TAC ATC TTT 2430

                745                 750                 755

GCC ACC ACC GGT TAT GGA ATT GCC CTT CAG AAA GGC TCT CCT TGG AAG 2478

            760                 765                 770

AGG CAG ATC GAC CTG GCC TTG CTT CAG TTT GTG GGT GAT GGT GAG ATG 2526

        775                 780                 785

GAG GAG CTG GAG ACC CTG TGG CTC ACT GGG ATC TGC CAC AAC GAG AAG 2574

    790                 795                 800

AAC GAG GTG ATG AGC AGC CAG CTG GAC ATT GAC AAC ATG GCG GGC GTA 2622

805                 810                 815                 820

TTC TAC ATG CTG GCT GCC GCC ATG GCC CTT AGC CTC ATC ACC TTC ATC 2670

                825                 830                 835

TGG GAG CAC CTC TTC TAC TGG AAG CTG CGC TTC TGT TTC ACG GGC GTG 2718

            840                 845                 850

TGC TCC GAC CGG CCT GGG TTG CTC TTC TCC ATC AGC AGG GGC ATC TAC 2766

        855                 860                 865

AGC TGC ATT CAT GGA GTG CAC ATT GAA GAA AAG AAG AAG TCT CCA GAC 2814

    870                 875                 880

TTC AAT CTG ACG GGA TCC CAG AGC AAC ATG TTA AAA CTC CTC CGG TCA 2862

885                 890                 895                 900

GCC AAA AAC ATT TCC AGC ATG TCC AAC ATG AAC TCC TCA AGA ATG GAC 2910

                905                 910                 915

TCA CCC AAA AGA GCT GCT GAC TTC ATC CAA AGA GGT TCC CTC ATC ATG 2958

            920                 925                 930

GAC ATG GTT TCA GAT AAG GGG AAT TTG ATG TAC TCA GAC AAC AGG TCC 3006

        935                 940                 945

TTT CAG GGG AAA GAG AGC ATT TTT GGA GAC AAC ATG AAC GAA CTC CAA 3054

    950                 955                 960

ACA TTT GTG GCC AAC CGG CAG AAG GAT AAC CTC AAT AAC TAT GTA TTC 3102

965                 970                 975                 980

CAG GGA CAA CAT CCT CTT ACT CTC AAT GAG TCC AAC CCT AAC ACG GTG 3150

                985                 990                 995

GAG GTG GCC GTG AGC ACA GAA TCC AAA GCG AAC TCT AGA CCC CGG CAG 3198

            1000                1005                1010

CTG TGG AAG AAA TCC GTA GAT TCC ATA CGC CAG GAT TCA CTA TCC CAG 3246

        1015                1020                1025

AAT CCA GTC TCC CAG AGG GAT GAG GCA ACA GCA GAG AAT AGG ACC CAC 3294

    1030                1035                1040

TCC CTA AAG AGC CCT AGG TAT CTT CCA GAA GAG ATG GCC CAC TCT GAC 3342

1045                1050                1055                1060

ATT TCA GAA ACG TCA AAT CGG GCC ACG TGC CAC AGG GAA CCT GAC AAC 3390

                1065                1070                1075

AGT AAG AAC CAC AAA ACC AAG GAC AAC TTT AAA AGG TCA GTG GCC TCC 3438

            1080                1085                1090

AAA TAC CCC AAG GAC TGT AGT GAG GTC GAG CGC ACC TAC CTG AAA ACC 3486

        1095                1100                1105

AAA TCA AGC TCC CCT AGA GAC AAG ATC TAC ACT ATA GAT GGT GAG AAG 3534

    1110                1115                1120

GAG CCT GGT TTC CAC TTA GAT CCA CCC CAG TTT GTT GAA AAT GTG ACC 3582

1125                1130                1135                1140

CTG CCC GAG AAC GTG GAC TTC CCG GAC CCC TAC CAG GAT CCC AGT GAA 3630

                1145                1150                1155

AAC TTC CGC AAG GGG GAC TCC ACG CTG CCA ATG AAC CGG AAC CCC TTG 3678

            1160                1165                1170

CAT AAT GAA GAG GGG CTT TCC AAC AAC GAC CAG TAT AAA CTC TAC TCC 3726

        1175                1180                1185

AAG CAC TTC ACC TTG AAA GAC AAG GGT TCC CCG CAC AGT GAG ACC AGC 3774

    1190                1195                1200

GAG CGA TAC CGG CAG AAC TCC ACG CAC TGC AGA AGC TGC CTT TCC AAC 3822

1205                1210                1215                1220

ATG CCC ACC TAT TCA GGC CAC TTC ACC ATG AGG TCC CCC TTC AAG TGC 3870

                1225                1230                1235

GAT GCC TGC CTG CGG ATG GGG AAT CTC TAT GAC ATC GAT GAA GAC CAG 3918

            1240                1245                1250

ATG CTT CAG GAG ACA GGT AAC CCA GCC ACC GGG GAG CAG GTC TAC CAG 3966

        1255                1260                1265

CAG GAC TGG GCA CAG AAC AAT GCC CTT CAA TTA CAA AAG AAC AAG CTA 4014

    1270                1275                1280

AGG ATT AGC CGT CAG CAT TCC TAC GAT AAC ATT GTC GAC AAA CCT AGG 4062

1285                1290                1295                1300

GAG CTA GAC CTT AGC AGG CCC TCC CGG AGC ATA AGC CTC AAG GAC AGG 4110

                1305                1310                1315

GAA CGG CTT CTG GAG GGA AAT TTT TAC GGC AGC CTG TTT AGT GTC CCC 4158

            1320                1325                1330

TCA AGC AAA CTC TCG GGG AAA AAA AGC TCC CTT TTC CCC CAA GGT CTG 4206

        1335                1340                1345

GAG GAC AGC AAG AGG AGC AAG TCT CTC TTG CCA GAC CAC ACC TCC GAT 4254

    1350                1355                1360

AAC CCT TTC CTC CAC TCC CAC AGG GAT GAC CAA CGC TTG GTT ATT GGG 4302

1365                1370                1375                1380

AGA TGC CCC TCG GAC CCT TAC AAA CAC TCG TTG CCA TCC CAG GCG GTG 4350

                1385                1390                1395

AAT GAC AGC TAT CTT CGG TCG TCC TTG AGG TCA ACG GCA TCG TAC TGT 4398

            1400                1405                1410

TCC AGG GAC AGT CGG GGC CAC AAT GAT GTG TAT ATT TCG GAG CAT GTT 4446

        1415                1420                1425

ATG CCT TAT GCT GCA AAT AAG AAT AAT ATG TAC TCT ACC CCC AGG GTT 4494

    1430                1435                1440

TTA AAT TCC TGC AGC AAT AGA CGC GTG TAC AAG AAA ATG CCT AGT ATC 4542

1445                1450                1455                1460

GAA TCT GAT GTT TAAAAATCTT CCATTAATGT TTTATCTATA GGGAAATATA 4594


CGTAATGGCC AATGTTCTGG AGGGTAAATG TTGGATGTCC AATAGTGCCCT GCTAAGAGG 4654

AAGAAGATGT AGGGAGGTAT TTTGTTGTTG TTGTTGTTGG CTCTTTTGCA CACGGCTTCA 4714

TGCCATAATC TTCCACTCAA GGAATCTTGT GAGGTGTGTG CTGAGCATGG CAGACACCAG 4774

ATAGGTGAGT CCTTAACCAA AAATAACTAA CTACATAAGG GCAAGTCTCC GGGACATGCC 4834

TACTGGGTAT GTTGGCAATA ATGATGCATT GGATGCCAAT GGTGATGTTA TGATTTCCTA 4894

TATTCCAAAT TCCATTAAGG TCAGCCCACC ATGTAATTTT CTCATCAGAA ATGCCTAATG 4954

GTTTCTCTAA TACAGAATAA GCAATATGGT GTGCATGTAA ACCTGACACA GACAAAATAA 5014

AAACAGTTAA GAATGCATCT GCACTGTAGT CGGATTTGAA CATGTGCAAG AGATTAGGAA 5074

GTTTGGCTCG TAACAGTTTC AGCTTTCTTG TTATGCCTTC CATCACAGCC CAGGCTCACC 5134

CCAAGAACTC CAGGCTCCCC TAAAGAATAG CAAATCAGTG TGTTCGTGAT GACTGTGCTA 5194

CCTTCATTAT AGTTCATTTC CAAGACACAT CTGGAGCCAA AGGCCCGAGG GACCCTCAGG 5254

TGGGGAGAGC TACAGGAATC TCTTTGGATG TTGATGTGTG TTTCTCTCTA CCCTCGGCTT 5314

CGATGGTCTT GTTCAGAGCT GCATAAACTA ACACATTTAT GTCTCCGAGA TCTAAGTGTG 5374

GATCTTCTGT CTGTGACACA GTGGCCATTG TAGTTTATCC CGAAGACGCC TATGTACGTA 5434

AGTTTGCATT TCCTCCCTTC TGGTGATGAC TCAGGGTTGT ATAGTATCTG TTACCCCTTC 5494

CCTCCCAGAG TAACCATAAC TCGTTCCGTT TCCAAACAGC CATGGTGGTG TCCAATTAGC 5554

TGTGTATCGC TCTTCCCAGA GTTGTTAATG TGGTGACATG CACCAACAGC CGTATGTGTA 5614

CTGTGATCTG TAAGAAGTAC AATGCCATCT GTCTGCCGAA GGCTAGCATG GTTTTAGGTT 5674

TATCTTCCTT CACATCCAGA AATTCTGTTG GACACTCACT TCCACCCCAA ACTCCTCAAA 5734

TCAAAAGCCT TCAAAACACG AGGCACTCTT GGATCTACCC TGAGTATCCT CCAAACTGTG 5794

GATACAGTTT AGTGAGACAA GCAATTTCTC CCTTCTGAGT TATTCTCTCT GTTGGTGGCA 5854

AACCACTTCA TAGCACCAAC AGAGATGTAG GAAAAATTCC TCAAAGTATT TGTCATTTCT 5914

GAGTCGCCTG CATTATCCCA TTCTTATTCT CCTCAAACCT GTGCATATAT GACATGAAAT 5974

GATATCCATT TTTTTTTTAA GTTAGAAACA GAGAGGGGAA TACTTATGCA TGGGGAGCCT 6034

GTTAGCACAG TGCCTGCCAC AAAAACAAGT GCCCCCGACA AGATAGTTGC TATGTTATGA 6094

CACTTTCTCA GATCAGGATT TTCTAGTTTA AAAATTAAAT ATCATAAAAC GGAATTC 6151

(2) INFORMATION FOR SEQ ID NO:2:

(i) SEQUENCE CHARACTERISTICS:
(A) LENGTH: 1464 amino acids
(B) TYPE: amino acid
(D) TOPOLOGY: linear

(ii) MOLECULE TYPE: protein

(xi) SEQUENCE DESCRIPTION: SEQ ID NO:2:

(2) INFORMATION FOR SEQ ID NO:3:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:3:

Lys Glu Phe Pro Ser Gly Leu Ile Ser Val Ser Tyr Asp Asp Trp Asp
1               5                   10                  15

Tyr Ser Leu Glu
            20

(2) INFORMATION FOR SEQ ID NO:4:

(i) SEQUENCE CHARACTERISTICS:
(A) LENGTH: 61 base pairs
(B) TYPE: nucleic acid
(C) STRANDEDNESS: single
(D) TOPOLOGY: linear

(xi) SEQUENCE DESCRIPTION: SEQ ID NO:4:

AAAAGAGTTT CCATCGGGAC TCATTTCTGT CTCCTACGAT GACTGGGACT ACAGCCTGGA 60

G 61

(2) INFORMATION FOR SEQ ID NO:5:

(i) SEQUENCE CHARACTERISTICS:
(A) LENGTH: 61 base pairs
(B) TYPE: nucleic acid
(C) STRANDEDNESS: single
(D) TOPOLOGY: linear

(xi) SEQUENCE DESCRIPTION: SEQ ID NO:5:

AGAAGAGTTT CCATCGGGAC TCATTTCTGT CTCCTACGAT GACTGGGACT ACAGCCTGGA 60

G 61

(2) INFORMATION FOR SEQ ID NO:6:

(i) SEQUENCE CHARACTERISTICS:
(A) LENGTH: 20 amino acids
(B) TYPE: amino acid
(D) TOPOLOGY: linear

(ii) MOLECULE TYPE: peptide

(xi) SEQUENCE DESCRIPTION: SEQ ID NO:6:

Glu Glu Phe Pro Ser Gly Leu Ile Ser Val Ser Tyr Asp Asp Trp Asp
1               5                   10                  15

Tyr Ser Leu Glu
            20

(2) INFORMATION FOR SEQ ID NO:7:

     (i) SEQUENCE CHARACTERISTICS:
          (A) LENGTH: 5983 base pairs
          (B) TYPE: nucleic acid
          (C) STRANDEDNESS: double
          (D) TOPOLOGY: linear

    (ii) MOLECULE TYPE: cDNA

    (ix) FEATURE:
          (A) NAME/KEY: CDS
          (B) LOCATION: 218..4669

    (xi) SEQUENCE DESCRIPTION: SEQ ID NO:7:
GAATTCCTTT GAATTTGCAT CTCTTCAAGA CACAAGATTA AAACAAAATT TACGCTAAAT      60
TGGATTTTAA ATTATCTTCC GTTCATTTAT CCTTCGTCTT TCTTATGTGG ATATGCAAGC     120
GAGAAGAAGG GACTGGACAT TCCCAACATG CTCACTCCCT TAATCTGTCC GTCTAGAGGT     180
TTGGCTTCTA CAAACCAAGG GAGTCGACGA GTTGAAG ATG AAG CCC AGA GCG GAG     235
TGC TGT TCT CCC AAG TTC TGG TTG GTG TTG GCC GTC CTG GCC GTG TCA     283
GGC AGC AGA GCT CGT TCT CAG AAG AGC CCC CCC AGC ATT GGC ATT GCT     331
GTC ATC CTC GTG GGC ACT TCC GAC GAG GTG GCC ATC AAG GAT GCC CAC     379
GAG AAA GAT GAT TTC CAC CAT CTC TCC GTG GTA CCC CGG GTG GAA CTG     427
GTA GCC ATG AAT GAG ACC GAC CCA AAG AGC ATC ATC ACC CGC ATC TGT     475
GAT CTC ATG TCT GAC CGG AAG ATC CAG GGG GTG GTG TTT GCT GAT GAC     523
ACA GAC CAG GAA GCC ATC GCC CAG ATC CTC GAT TTC ATT TCA GCA CAG     571
ACT CTC ACC CCG ATC CTG GGC ATC CAC GGG GGC TCC TCT ATG ATA ATG     619
GCA GAT AAG GAT GAA TCC TCC ATG TTC TTC CAG TTT GGC CCA TCA ATT 667
GAA CAG CAA GCT TCC GTA ATG CTC AAC ATC ATG GAA GAA TAT GAC TGG 715
TAC ATC TTT TCT ATC GTC ACC ACC TAT TTC CCT GGC TAC CAG GAC TTT 763
GTA AAC AAG ATC CGC AGC ACC ATT GAG AAT AGC TTT GTG GGC TGG GAG 811
CTA GAG GAG GTC CTC CTA CTG GAC ATG TCC CTG GAC GAT GGA GAT TCT 859
AAG ATC CAG AAT CAG CTC AAG AAA CTT CAA AGC CCC ATC ATT CTT CTT 907
TAC TGT ACC AAG GAA GAA GCC ACC TAC ATC TTT GAA GTG GCC AAC TCA 955
GTA GGG CTG ACT GGC TAT GGC TAC ACG TGG ATC GTG CCC AGT CTG GTG 1003
GCA GGG GAT ACA GAC ACA GTG CCT GCG GAG TTC CCC ACT GGG CTC ATC 1051
TCT GTA TCA TAT GAT GAA TGG GAC TAT GGC CTC CCC GCC AGA GTG AGA 1099
GAT GGA ATT GCC ATA ATC ACC ACT GCT GCT TCT GAC ATG CTG TCT GAG 1147
CAC AGC TTC ATC CCT GAG CCC AAA AGC AGT TGT TAC AAC ACC CAC GAG 1195
AAG AGA ATC TAC CAG TCC AAT ATG CTA AAT AGG TAT CTG ATC AAT GTC 1243
ACT TTT GAG GGG AGG AAT TTG TCC TTC AGT GAA GAT GGC TAC CAG ATG 1291
CAC CCG AAA CTG GTG ATA ATT CTT CTG AAC AAG GAG AGG AAG TGG GAA 1339
AGG GTG GGG AAG TGG AAA GAC AAG TCC CTG CAG ATG AAG TAC TAT GTG 1387
TGG CCC CGA ATG TGT CCA GAG ACT GAA GAG CAG GAG GAT GAC CAT CTG 1435
AGC ATT GTG ACC CTG GAG GAG GCA CCA TTT GTC ATT GTG GAA AGT GTG 1483
GAC CCT CTG AGT GGA ACC TGC ATG AGG AAC ACA GTC CCC TGC CAA AAA 1531
CGC ATA GTC ACT GAG AAT AAA ACA GAC GAG GAG CCG GGT TAC ATC AAA 1579
AAA TGC TGC AAG GGG TTC TGT ATT GAC ATC CTT AAG AAA ATT TCT AAA 1627
TCT GTG AAG TTC ACC TAT GAC CTT TAC CTG GTT ACC AAT GGC AAG CAT 1675
GGG AAG AAA ATC AAT GGA ACC TGG AAT GGT ATG ATT GGA GAG GTG GTC 1723
ATG AAG AGG GCC TAC ATG GCA GTG GGC TCA CTC ACC ATC AAT GAG GAA 1771
CGA TCG GAG GTG GTC GAC TTC TCT GTG CCC TTC ATA GAG ACA GGC ATC 1819
AGT GTC ATG GTG TCA CGC AGC AAT GGG ACT GTC TCA CCT TCT GCC TTC 1867
TTA GAG CCA TTC AGC GCT GAC GTA TGG GTG ATG ATG TTT GTG ATG CTG 1915
CTC ATC GTC TCA GCC GTG GCT GTC TTT GTC TTT GAG TAC TTC AGC CCT 1963
GTG GGT TAT AAC AGG TGC CTC GCT GAT GGC AGA GAG CCT GGT GGA CCC 2011
TCT TTC ACC ATC GGC AAA GCT ATT TGG TTG CTC TGG GGT CTG GTG TTT 2059
AAC AAC TCC GTA CCT GTG CAG AAC CCA AAG GGG ACC ACC TCC AAG ATC 2107
ATG GTG TCA GTG TGG GCC TTC TTT GCT GTC ATC TTC CTG GCC AGC TAC 2155
ACT GCC AAC TTA GCT GCC TTC ATG ATC CAA GAG GAA TAT GTG GAC CAG 2203
GTT TCT GGC CTG AGC GAC AAA AAG TTC CAG AGA CCT AAT GAC TTC TCA 2251
CCC CCT TTC CGC TTT GGG ACC GTG CCC AAC GGC AGC ACA GAG AGA AAT 2299
ATT CGC AAT AAC TAT GCA GAA ATG CAT GCC TAC ATG GGA AAG TTC AAC 2347
CAG AGG GGT GTA GAT GAT GCA TTG CTC TCC CTG AAA ACA GGG AAA CTG 2395
GAT GCC TTC ATC TAT GAT GCA GCA GTG CTG AAC TAT ATG GCA GGC AGA 2443
GAT GAA GGC TGC AAG CTG GTG ACC ATT GGC AGT GGG AAG GTC TTT GCT 2491
TCC ACT GGC TAT GGC ATT GCC ATC CAA AAA GAT TCT GGG TGG AAG CGC 2539
CAG GTG GAC CTT GCT ATC CTG CAG CTC TTT GGA GAT GGG GAG ATG GAA 2587
GAA CTG GAA GCT CTC TGG CTC ACT GGC ATT TGT CAC AAT GAG AAG AAT 2635
GAG GTC ATG AGC AGC CAG CTG GAC ATT GAC AAC ATG GCA GGG GTC TTC 2683
TAC ATG TTG GGG GCG GCC ATG GCT CTC AGC CTC ATC ACC TTC ATC TGC 2731
GAA CAC CTT TTC TAT TGG CAG TTC CGA CAT TGC TTT ATG GGT GTC TGT 2779
TCT GGC AAG CCT GGC ATG GTC TTC TCC ATC AGC AGA GGT ATC TAC AGC 2827
TGC ATC CAT GGG GTG GCG ATC GAG GAG CGC CAG TCT GTA ATG AAC TCC 2875
CCC ACT GCA ACC ATG AAC AAC ACA CAC TCC AAC ATC CTG CGC CTG CTG 2923
CGC ACG GCC AAG AAC ATG GCT AAC CTG TCT GGT GTG AAT GGC TCA CCG 2971
CAG AGG CCC CTG GAC TTC ATC CGA CGG GAG TCA TCC GTC TAT GAC ATC 3019
TCA GAG CAC CGC CGC AGC TTC ACG CAT TCT GAC TGC AAA TCC TAC AAC 3067
AAC CCG CCC TGT GAG GAG AAC CTC TTC AGT GAC TAC ATC AGT GAG GTA 3115
GAG AGA ACG TTC GGG AAC CTG CAG CTG AAG GAC AGC AAC GTG TAC CAA 3163
GAT CAC TAC CAC CAT CAC CAC CGG CCC CAT AGT ATT GGC AGT GCC AGC 3211
TCC ATC GAT GGG CTC TAC GAC TGT GAC AAC CCA CCC TTC ACC ACC CAG 3259
TCC AGG TCC ATC AGC AAG AAG CCC CTG GAC ATC GGC CTC CCC TCC TCC 3307
AAG CAC AGC CAG CTC AGT GAC CTG TAC GGC AAA TTC TCC TTC AAG AGC 3355
GAC CGC TAC AGT GGC CAC GAC GAC TTG ATC CGC TCC GAT GTC TCT GAC 3403
ATC TCA ACC CAC ACC GTC ACC TAT GGG AAC ATC GAG GGC AAT GCC GCC 3451
AAG AGG CGT AAG CAG CAA TAT AAG GAC AGC CTG AAG AAG CGG CCT GCC 3499
TCG GCC AAG TCC CGC AGG GAG TTT GAC GAG ATC GAG CTG GCC TAC CGT 3547
CGC CGA CCG CCC CGC TCC CCT GAC CAC AAG CGC TAC TTC AGG GAC AAG 3595
GAA GGG CTA CGG GAC TTC TAC CTG GAC CAG TTC CGA ACA AAG GAG AAC 3643
TCA CCC CAC TGG GAG CAC GTA GAC CTG ACC GAC ATC TAC AAG GAG CGG 3691
AGT GAT GAC TTT AAG CGC GAC TCC GTC AGC GGA GGA GGG CCC TGT ACC 3739
AAC AGG TCT CAC ATC AAG CAC GGG ACG GGC GAC AAA CAC GGC GTG GTC 3787
AGC GGG GTA CCT GCA CCT TGG GAG AAG AAC CTG ACC AAC GTG GAG TGG 3835
GAG GAC CGG TCC GGG GGC AAC TTC TGC CGC AGC TGT CCC TCC AAG CTG 3883
CAC AAC TAC TCC ACG ACG GTG ACG GGT CAG AAC TCG GGC AGG CAG GCG 3931
TGC ATC CGG TGT GAG GCT TGC AAG AAA GCA GGC AAC CTG TAT GAC ATC 3979
AGT GAG GAC AAC TCC CTG CAG GAA CTG GAC CAG CCG GCT GCC CCA GTG 4027
GCG GTG ACG TCA AAC GCC TCC ACC ACT AAG TAC CCT CAG AGC CCG ACT 4075
AAT TCC AAG GCC CAG AAG AAG AAC CGG AAC AAA CTG CGC CGG CAG CAC 4123
TCC TAC GAC ACC TTC GTG GAC CTG CAG AAG GAA GAA GCC GCC CTG GCC 4171
CCG CGC AGC GTA AGC CTG AAA GAC AAG GGC CGA TTC ATG GAT GGG AGC 4219
CCC TAC GCC CAC ATG TTT GAG ATG TCA GCT GGC GAG AGC ACC TTT GCC     4267
AAC AAC AAG TCC TCA GTG CCC ACT GCC GGA CAT CAC CAC CAC AAC AAC     4315
CCC GGC GGC GGG TAC ATG CTC AGC AAG TCG CTC TAC CCT GAC CGG GTC     4363
ACG CAA AAC CCT TTC ATC CCC ACT TTT GGG GAC GAC CAG TGC TTG CTC     4411
CAT GGC AGC AAA TCC TAC TTC TTC AGG CAG CCC ACG GTG GCG GGG GCG     4459
TCG AAA GCC AGG CCG GAC TTC CGG GCC CTT GTC ACC AAC AAG CCG GTG     4507
GTC TCG GCC CTT CAT GGG GCC GTG CCA GCC CGT TTC CAG AAG GAC ATC     4555
TGT ATA GGG AAC CAG TCC AAC CCC TGT GTG CCT AAC AAC AAA AAC CCC     4603
AGG GCT TTC AAT GGC TCC AGC AAT GGG CAT GTT TAT GAG AAA CTT TCT     4651
AGT ATT GAG TCT GAT GTC TGAGTGAGGG AACAGAGAGG TTAAGGTGGG            4699
TACGGGAGGG TAAGGCTGTG GGTCGCGTGA TGCGCATGTC ACGGAGGGTG ACGGGGGTGA    4759
ACTTGGTTCC CATTTGCTCC TTTCTTGTTT TAATTTATTT ATGGGGATCC TGGAGTTCTG    4819
GTTCCTACTG GGGGCAACCC TGGTGACCAG CACCATCTCT CCTCCTTTTC ACAGTTCTCT    4879
CCTTCTTCCC CCCGCTGTCA GCCATTCCTG TTCCCATGAG ATGATGCCAT GGGTCTCAGC    4939
AGGGGAGGGT AGAGCGGAGA AAGGAAGGGC AGCATGCGGG CTTCCTCCTG GTGTGGAAGA    4999
GCTCCTTGAT ATCCTCTTTG AGTGAAGCTG GGAGAACCAA AAAGAGGCTA TGTGAGCACA    5059
AAGGTAGCTT TTCCCAAACT GATCTTTTCA TTTAGGTGAG GAAGCAAAAG CATCTATGTG    5119
AGACCATTTA GCACACTGCT TGTGAAAGGA AAGAGGCTCT GGCTAAATTC ATGCTGCTTA    5179
GATGACATCT GTCTAGGAAT CATGTGCCAA GCAGAGGTTG GGAGGCCATT TGTGTTTATA    5239
TATAAGCCAA AAAATGCTTG CTTCAACCCC ATGAGACTCG ATAGTGGTGG TGAACAGAAC    5299
AAAAGGTCAT TGGTGGCAGA GTGGATTCTT GAACAAACTG GAAAGTACGT TATGATAGTG 5359
TCCCACGGTG CCTTGGGGAC AAGAGCAGGT GGATTGTGCG TGCATGTGTG TTCATGCACA 5419
CTTGCACCCA TGTGTAGTCA GGTGCCTCAA GAGAAGGCAA CCTTGACTCT TTCTATTGTT 5479
TCTTTCAATA TCCCCAAGCA GTGTGATTGT TTGGCTTATA TACAGACAGA GATGGCCATG 5539
TATTACCTGA ATTTTGGCTG TGTCTCCCTT CATCCTTCTG GAATAAGGAG AATGAAAATT 5599
CTTGATAAAG AAGATTCTGT GGTCTAAACA AAAAAAGGCG GTGAGCAATC CTGCAAGAGC 5659
AAGGTACATA AACAAGTCCT CAGTGGTTGG CAACTGTTTC AACCTGTTTG AACCAAGAAC 5719
CTTCCAGGAA GGCTAAAGGG AAACCGAATT TCACAGCCAT GATTCTTTTG CCCACACTTG 5779
GGAGCAAAAG ATTCTACAAA GCTCTTTTGA GCATTTAGAC TCTCGACTGG CCAAGGTTTG 5839
GGGAAGAACG AAGCCACCTT TGAAGAAGTA AGGAGTCGTG TATGGTAGGG TAAGTGAGAG 5899
AGGGGGATGT TTCCAATGCT TTGATCCCTT CTTACTTAAC CTGAAGCTAG ACGAGCAGGC 5959
TTCTTCCCCC CAAAACTGAA TTCC 5983

(2) INFORMATION FOR SEQ ID NO:8:

(i) SEQUENCE CHARACTERISTICS:
(A) LENGTH: 1484 amino acids
(B) TYPE: amino acid
(D) TOPOLOGY: linear

(ii) MOLECULE TYPE: protein

(xi) SEQUENCE DESCRIPTION: SEQ ID NO:8:

(2) INFORMATION FOR SEQ ID NO:9:

(i) SEQUENCE CHARACTERISTICS:
(A) LENGTH: 20 amino acids
(B) TYPE: amino acid
(C) STRANDEDNESS: single
(D) TOPOLOGY: linear

(ii) MOLECULE TYPE: peptide

(v) FRAGMENT TYPE: internal

(xi) SEQUENCE DESCRIPTION: SEQ ID NO:9:

Tyr Val Trp Pro Arg Met Cys Pro Glu Thr Glu Glu Gln Glu Asp Asp
1               5                   10                  15

His Leu Ser Ile
            20

(2) INFORMATION FOR SEQ ID NO:10:

(i) SEQUENCE CHARACTERISTICS:
(A) LENGTH: 60 base pairs
(B) TYPE: nucleic acid
(C) STRANDEDNESS: single
(D) TOPOLOGY: linear

(xi) SEQUENCE DESCRIPTION: SEQ ID NO:10:

CTATGTGTGG CCCCGAATGT GTCCAGAGAC TGAAGAGCAG GAGGATGACC ATCTGAGCAT 60

(2) INFORMATION FOR SEQ ID NO:11:

(i) SEQUENCE CHARACTERISTICS:
(A) LENGTH: 60 base pairs
(B) TYPE: nucleic acid
(C) STRANDEDNESS: single
(D) TOPOLOGY: linear

(xi) SEQUENCE DESCRIPTION: SEQ ID NO:11:

CTATGTGTGG CCCCGAATGT GTCCAGAGAC TGAAGAGCAG GAGGATGACC ATCTGAACAT 60

(2) INFORMATION FOR SEQ ID NO:12:

(i) SEQUENCE CHARACTERISTICS:
(A) LENGTH: 20 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:12:

Tyr Val Trp Pro Arg Met Cys Pro Glu Thr Glu Glu Gln Glu Asp Asp
1               5                   10                  15

His Leu Asn Ile
            20

(2) INFORMATION FOR SEQ ID NO:13:

     (i) SEQUENCE CHARACTERISTICS:
          (A) LENGTH: 1785 base pairs
          (B) TYPE: nucleic acid
          (C) STRANDEDNESS: double
          (D) TOPOLOGY: linear

    (ix) FEATURE:
          (A) NAME/KEY: CDS
          (B) LOCATION: 1..1785

    (xi) SEQUENCE DESCRIPTION: SEQ ID NO:13:

GCC TTC TAC AGG CAC CTA CTG AAT GTC ACC TGG GAG GGC CGA GAC TTC      48
Ala Phe Tyr Arg His Leu Leu Asn Val Thr Trp Glu Gly Arg Asp Phe
  1               5                  10                  15

TCC TTC AGC CCT GGT GGG TAC CTG GTC CAG CCC ACC ATG GTG GTG ATC      96
Ser Phe Ser Pro Gly Gly Tyr Leu Val Gln Pro Thr Met Val Val Ile
             20                  25                  30

GCC CTC AAC CGG CAC CGC CTC TGG GAG ATG GTG GGG CGC TGG GAG CAT     144
Ala Leu Asn Arg His Arg Leu Trp Glu Met Val Gly Arg Trp Glu His
         35                  40                  45

GGC GTC CTA TAC ATG AAG TAC CCC GTG TGG CCT CGC TAC AGT GCC TCT     192
Gly Val Leu Tyr Met Lys Tyr Pro Val Trp Pro Arg Tyr Ser Ala Ser
     50                  55                  60

CTG CAG CCT GTG GTG GAC AGT CGG CAC CTG ACG GTG GCC ACG CTG GAA     240
Leu Gln Pro Val Val Asp Ser Arg His Leu Thr Val Ala Thr Leu Glu
 65                  70                  75                  80

GAG CGG CCC TTT GTC ATC GTG GAG AGC CCT GAC CCT GGC ACA GGA GGC     288
Glu Arg Pro Phe Val Ile Val Glu Ser Pro Asp Pro Gly Thr Gly Gly
                 85                  90                  95

TGT GTC CCC AAC ACC GTG CCC TGC CGC AGG CAG AGC AAC CAC ACC TTC     336
Cys Val Pro Asn Thr Val Pro Cys Arg Arg Gln Ser Asn His Thr Phe
            100                 105                 110

AGC AGC GGG GAC GTG GCC CCC TAC ACC AAG CTC TGC TGT AAG GGA TTC     384
Ser Ser Gly Asp Val Ala Pro Tyr Thr Lys Leu Cys Cys Lys Gly Phe
        115                 120                 125

TGC ATC GAC ATC CTC AAG AAG CTG GCC AGA GTG GTC AAA TTC TCC TAC     432
Cys Ile Asp Ile Leu Lys Lys Leu Ala Arg Val Val Lys Phe Ser Tyr
    130                 135                 140

GAC CTG TAC CTG GTG ACC AAC GGC AAG CAT GGC AAG CGG GTG CGC GGC 480
Asp Leu Tyr Leu Val Thr Asn Gly Lys His Gly Lys Arg Val Arg Gly
145                 150                 155                 160

GTA TGG AAC GGC ATG ATT GGG GAG GTG TAC TAC AAG CGG GCA GAC ATG 528
Val Trp Asn Gly Met Ile Gly Glu Val Tyr Tyr Lys Arg Ala Asp Met
                165                 170                 175

GCC ATC GGC TCC CTC ACC ATC AAT GAG GAA CGC TCC GAG ATC GTA GAC 576
Ala Ile Gly Ser Leu Thr Ile Asn Glu Glu Arg Ser Glu Ile Val Asp
            180                 185                 190

TTC TCT GTA CCC TTT GTG GAG ACG GGC ATC AGT GTG ATG GTG GCT CGC 624
Phe Ser Val Pro Phe Val Glu Thr Gly Ile Ser Val Met Val Ala Arg
        195                 200                 205

AGC AAT GGC ACC GTC TCC CCC TCG GCC TTC TTG GAG CCA TAT AGC CCT 672
Ser Asn Gly Thr Val Ser Pro Ser Ala Phe Leu Glu Pro Tyr Ser Pro
    210                 215                 220

GCA GTG TGG GTG ATG ATG TTT GTC ATG TGC CTC ACT GTG GTG GCC ATC 720
Ala Val Trp Val Met Met Phe Val Met Cys Leu Thr Val Val Ala Ile
225                 230                 235                 240

ACC GTC TTC ATG TTC GAG TAC TTC AGC CCT GTC AGC TAC AAC CAG AAC 768
Thr Val Phe Met Phe Glu Tyr Phe Ser Pro Val Ser Tyr Asn Gln Asn
                245                 250                 255

CTC ACC AGA GGC AAG AAG TCC GGG GGC CCA GCT TTC ACT ATC GGC AAG 816
Leu Thr Arg Gly Lys Lys Ser Gly Gly Pro Ala Phe Thr Ile Gly Lys
            260                 265                 270

TCC GTG TGG CTG CTG TGG GCG CTG GTC TTC AAC AAC TCA GTG CCC ATC 864
Ser Val Trp Leu Leu Trp Ala Leu Val Phe Asn Asn Ser Val Pro Ile
        275                 280                 285

GAG AAC CCG CGG GGC ACC ACC AGC AAG ATC ATG GTT CTG GTC TGG GCC 912
Glu Asn Pro Arg Gly Thr Thr Ser Lys Ile Met Val Leu Val Trp Ala
    290                 295                 300

TTC TTT GCT GTC ATC TTC CTC GCC AGC TAC ACG GCC AAC CTG GCC GCC 960
Phe Phe Ala Val Ile Phe Leu Ala Ser Tyr Thr Ala Asn Leu Ala Ala
305                 310                 315                 320

TTC ATG ATC CAA GAG CAA TAC ATC GAC ACT GTG TCG GGC CTC AGT GAC 1008
Phe Met Ile Gln Glu Gln Tyr Ile Asp Thr Val Ser Gly Leu Ser Asp
                325                 330                 335

AAG AAG TTT CAG CGG CCT CAA GAT CAG TAC CCA CCT TTC CGC TTC GGC 1056
Lys Lys Phe Gln Arg Pro Gln Asp Gln Tyr Pro Pro Phe Arg Phe Gly
            340                 345                 350

ACG GTG CCC AAC GGC AGC ACG GAG CGG AAC ATC CGC AGT AAC TAC CGT 1104
Thr Val Pro Asn Gly Ser Thr Glu Arg Asn Ile Arg Ser Asn Tyr Arg
        355                 360                 365

GAC ATG CAC ACC CAC ATG GTC AAG TTC AAC CAG CGC TCG GTG GAG GAC 1152
Asp Met His Thr His Met Val Lys Phe Asn Gln Arg Ser Val Glu Asp
    370                 375                 380

GCG CTC ACC AGC CTC AAG ATG GGG AAG CTG GAT GCC TTC ATC TAT GAT 1200
Ala Leu Thr Ser Leu Lys Met Gly Lys Leu Asp Ala Phe Ile Tyr Asp
385                 390                 395                 400

GCT GCT GTC CTC AAC TAC ATG GCA GGC AAG GAC GAG GGC TGC AAG CTG 1248
Ala Ala Val Leu Asn Tyr Met Ala Gly Lys Asp Glu Gly Cys Lys Leu
                405                 410                 415

GTC ACC ATT GGG TCT GGC AAG GTC TTT GCT ACC ACT GGC TAC GGC ATC 1296
Val Thr Ile Gly Ser Gly Lys Val Phe Ala Thr Thr Gly Tyr Gly Ile
            420                 425                 430

GCC ATG CAG AAG GAC TCC CAC TGG AAG CGG GCC ATA GAC CTG GCG CTC 1344
Ala Met Gln Lys Asp Ser His Trp Lys Arg Ala Ile Asp Leu Ala Leu
        435                 440                 445

TTG CAG TTC CTG GGG GAC GGA GAG ACA CAG AAA CTG GAG ACA GTG TGG 1392
Leu Gln Phe Leu Gly Asp Gly Glu Thr Gln Lys Leu Glu Thr Val Trp
    450                 455                 460

CTC TCA GGG ATC TGC CAG AAT GAG AAG AAC GAG GTG ATG AGC AGC AAG 1440
Leu Ser Gly Ile Cys Gln Asn Glu Lys Asn Glu Val Met Ser Ser Lys
465                 470                 475                 480

CTG GAC ATC GAC AAC ATG GCA GGC GTC TTC TAC ATG CTG CTG GTG GCC 1488
Leu Asp Ile Asp Asn Met Ala Gly Val Phe Tyr Met Leu Leu Val Ala
                485                 490                 495

ATG GGG CTG GCC CTG CTG GTC TTC GCC TGG GAG CAC CTG GTC TAC TGG 1536
Met Gly Leu Ala Leu Leu Val Phe Ala Trp Glu His Leu Val Tyr Trp
            500                 505                 510

AAG CTG CGC CAC TCG GTG CCC AAC TCA TCC CAG CTG GAC TTC CTG CTG 1584
Lys Leu Arg His Ser Val Pro Asn Ser Ser Gln Leu Asp Phe Leu Leu
        515                 520                 525

GCT TTC AGC AGG GGC ATC TAC AGC TGC TTC AGC GGG GTG CAG AGC CTC 1632
Ala Phe Ser Arg Gly Ile Tyr Ser Cys Phe Ser Gly Val Gln Ser Leu
    530                 535                 540

GCC AGC CCA CCG CGG CAG GCC AGC CCG GAC CTC ACG GCC AGC TCG GCC 1680
Ala Ser Pro Pro Arg Gln Ala Ser Pro Asp Leu Thr Ala Ser Ser Ala
545                 550                 555                 560

CAG GCC AGC GTG CTC AAG ATC GTG CAG GCA GCC CGC GAC ATG GTG ACC 1728
Gln Ala Ser Val Leu Lys Ile Val Gln Ala Ala Arg Asp Met Val Thr
                565                 570                 575

ACG GCG GGC GTA AGC AGC TCC CTG GAC CGC GCC ACT CGC ACC ATC GAG 1776
Thr Ala Gly Val Ser Ser Ser Leu Asp Arg Ala Thr Arg Thr Ile Glu
            580                 585                 590

AAT TGG GGT 1785
Asn Trp Gly
        595

(2) INFORMATION FOR SEQ ID NO:14:

(i) SEQUENCE CHARACTERISTICS:
(A) LENGTH: 595 amino acids
(B) TYPE: amino acid
(D) TOPOLOGY: linear

(ii) MOLECULE TYPE: protein

(xi) SEQUENCE DESCRIPTION: SEQ ID NO:14:

Ala Phe Tyr Arg His Leu Leu Asn Val Thr Trp Glu Gly Arg Asp Phe
1               5                   10                  15

Ser Phe Ser Pro Gly Gly Tyr Leu Val Gln Pro Thr Met Val Val Ile
            20                  25                  30

Ala Leu Asn Arg His Arg Leu Trp Glu Met Val Gly Arg Trp Glu His
        35                  40                  45

Gly Val Leu Tyr Met Lys Tyr Pro Val Trp Pro Arg Tyr Ser Ala Ser
    50                  55                  60

Leu Gln Pro Val Val Asp Ser Arg His Leu Thr Val Ala Thr Leu Glu
65                  70                  75                  80

Glu Arg Pro Phe Val Ile Val Glu Ser Pro Asp Pro Gly Thr Gly Gly
                85                  90                  95

Cys Val Pro Asn Thr Val Pro Cys Arg Arg Gln Ser Asn His Thr Phe
            100                 105                 110

Ser Ser Gly Asp Val Ala Pro Tyr Thr Lys Leu Cys Cys Lys Gly Phe
        115                 120                 125

Cys Ile Asp Ile Leu Lys Lys Leu Ala Arg Val Val Lys Phe Ser Tyr
    130                 135                 140

Asp Leu Tyr Leu Val Thr Asn Gly Lys His Gly Lys Arg Val Arg Gly
145                 150                 155                 160

Val Trp Asn Gly Met Ile Gly Glu Val Tyr Tyr Lys Arg Ala Asp Met
                165                 170                 175

Ala Ile Gly Ser Leu Thr Ile Asn Glu Glu Arg Ser Glu Ile Val Asp
            180                 185                 190

Phe Ser Val Pro Phe Val Glu Thr Gly Ile Ser Val Met Val Ala Arg
        195                 200                 205

Ser Asn Gly Thr Val Ser Pro Ser Ala Phe Leu Glu Pro Tyr Ser Pro
    210                 215                 220

Ala Val Trp Val Met Met Phe Val Met Cys Leu Thr Val Val Ala Ile
225                 230                 235                 240

Thr Val Phe Met Phe Glu Tyr Phe Ser Pro Val Ser Tyr Asn Gln Asn
                245                 250                 255

Leu Thr Arg Gly Lys Lys Ser Gly Gly Pro Ala Phe Thr Ile Gly Lys
            260                 265                 270

Ser Val Trp Leu Leu Trp Ala Leu Val Phe Asn Asn Ser Val Pro Ile
        275                 280                 285

Glu Asn Pro Arg Gly Thr Thr Ser Lys Ile Met Val Leu Val Trp Ala
    290                 295                 300

Phe Phe Ala Val Ile Phe Leu Ala Ser Tyr Thr Ala Asn Leu Ala Ala
305                 310                 315                 320

Phe Met Ile Gln Glu Gln Tyr Ile Asp Thr Val Ser Gly Leu Ser Asp
                325                 330                 335

Lys Lys Phe Gln Arg Pro Gln Asp Gln Tyr Pro Pro Phe Arg Phe Gly
            340                 345                 350

Thr Val Pro Asn Gly Ser Thr Glu Arg Asn Ile Arg Ser Asn Tyr Arg
        355                 360                 365

Asp Met His Thr His Met Val Lys Phe Asn Gln Arg Ser Val Glu Asp
    370                 375                 380

Ala Leu Thr Ser Leu Lys Met Gly Lys Leu Asp Ala Phe Ile Tyr Asp
385                 390                 395                 400

Ala Ala Val Leu Asn Tyr Met Ala Gly Lys Asp Glu Gly Cys Lys Leu
                405                 410                 415

Val Thr Ile Gly Ser Gly Lys Val Phe Ala Thr Thr Gly Tyr Gly Ile
            420                 425                 430

Ala Met Gln Lys Asp Ser His Trp Lys Arg Ala Ile Asp Leu Ala Leu
        435                 440                 445

Leu Gln Phe Leu Gly Asp Gly Glu Thr Gln Lys Leu Glu Thr Val Trp
    450                 455                 460

Leu Ser Gly Ile Cys Gln Asn Glu Lys Asn Glu Val Met Ser Ser Lys
465                 470                 475                 480

Leu Asp Ile Asp Asn Met Ala Gly Val Phe Tyr Met Leu Leu Val Ala
                485                 490                 495

Met Gly Leu Ala Leu Leu Val Phe Ala Trp Glu His Leu Val Tyr Trp
            500                 505                 510

Lys Leu Arg His Ser Val Pro Asn Ser Ser Gln Leu Asp Phe Leu Leu
        515                 520                 525

Ala Phe Ser Arg Gly Ile Tyr Ser Cys Phe Ser Gly Val Gln Ser Leu
    530                 535                 540

Ala Ser Pro Pro Arg Gln Ala Ser Pro Asp Leu Thr Ala Ser Ser Ala
545                 550                 555                 560

Gln Ala Ser Val Leu Lys Ile Val Gln Ala Ala Arg Asp Met Val Thr
                565                 570                 575

Thr Ala Gly Val Ser Ser Ser Leu Asp Arg Ala Thr Arg Thr Ile Glu
            580                     585                     590

Asn Trp Gly
        595

(2) INFORMATION FOR SEQ ID NO:15:

     (i) SEQUENCE CHARACTERISTICS:
          (A) LENGTH: 4659 base pairs
          (B) TYPE: nucleic acid
          (C) STRANDEDNESS: double
          (D) TOPOLOGY: linear

    (ii) MOLECULE TYPE: cDNA

    (ix) FEATURE:
          (A) NAME/KEY: misc_feature
          (B) LOCATION: 2781..2838
          (D) OTHER INFORMATION: /function= "transmembrane domain"

    (ix) FEATURE:
          (A) NAME/KEY: misc_feature
          (B) LOCATION: 2895..2958
          (D) OTHER INFORMATION: /function= "transmembrane domain"

    (ix) FEATURE:
          (A) NAME/KEY: misc_feature
          (B) LOCATION: 2988..3045
          (D) OTHER INFORMATION: /function= "transmembrane domain"

    (ix) FEATURE:
          (A) NAME/KEY: misc_feature
          (B) LOCATION: 3534..3597
          (D) OTHER INFORMATION: /function= "transmembrane domain"

    (ix) FEATURE:
          (A) NAME/KEY: CDS
          (B) LOCATION: 1099..3753

    (ix) FEATURE:
          (A) NAME/KEY: mat_peptide
          (B) LOCATION: 1153..3753
          (D) OTHER INFORMATION: /product= "NMDAR1-1"

    (ix) FEATURE:
          (A) NAME/KEY: sig_peptide
          (B) LOCATION: 1099..1152

    (xi) SEQUENCE DESCRIPTION: SEQ ID NO:15:

GAATTCCGGT AAGGCTCTGG AAAAGGGGGC GCTGGGAGCG CATTGCGAGG GGGCTGGAGA      60

GGGAGAGAGG AGCGGAAGCT GAGGGTGTGA AACGGCTGGC CCCGAACACA CCTCGCGGCG     120

CTCCAGTGAT TCCTGGTGTC CGACCTCAGC CCCAGTCAGT GCGGGTCCAG TTTCCAGGCT     180

CTCGCGGAAG GCCTGGCTGA GCACATGCGG CAGCCACGGT CGCCCTCCCT ATTCCTCTTA 240

GCCCGAGGAG GGGGGTCCCA AGTTACATGG CCACGCAGAT GGGGCCTCTC CCTCATTTCT 300

GAACCTTGTG GGGAGGGGAA CCTTGAAGGG AGCGCCCCCC AGAGCCATGG CTTAGGGCCT 360

CCCCCACCCC TCTGGAGCTC CAGTCTGCAA GAGTCAGGAG CCGAAATATC GCTGACTGTG 420

GGTGACGACT CTTGCGCGCA CACACACATA CAAGCGGGCA CGACGCGTTC GGTCCTATTA 480

AAAGGCACGC AAGGGTGCGG CTGCACGCGG TGACACGGAC CCCTCTAACG TTTCCAAACT 540

GAGCTCCCTG CAGGTCCCCG ACAGCACAGG CCCCTGTCCC AGGACCCCTC CAGGCACGCG 600

CTCACACGCA CACGCGCGCT CCCCGGCTCA CGCGCGCTCC GACACACACG CTCACGCGAA 660

CGCAGGCGCA CGCTCTGGCG CGGGAGGCGC CCCTTCGCCT CCGTGTTGGG AAGCGGGGGC 720

GGCGGGAGGG GCAGGAGACG TTGGCCCCGC TCGCGTTTCT GCAGCTGCTG CAGTCGCCGC 780

AGCGTCCGGA CCGGAACCAG CGCCGTCCGC GGAGCCGCCG CCGCCGCCGC CGGGCCCTTT 840

CCAAGCCGGG CGCTCGGAGC TGTGCCCGGC CCCGCTTCAG CACCGCGGAC AGCTCCGGCC 900

GCGTGGGGCT GAGCCGAGCC CCCGCGCACG CTTCAGCCCC CTTCCCTCGG CCGACGTCCC 960

GGGACCGCCG CTCCGGGGGA GACGTGGCGT CCGCAGCCCG CGGGGCCGGG CGAGCGCAGG 1020

ACGGCCCGGA AGCCCCGCGG GGGATGCGCC GAGGGCCCGC GTTCGCGCCG CGCAGAGCCA 1080

GGCCCGCGGC CCGAGCCC ATG AGC ACC ATG CGC CTG CTG ACG CTC GCC CTG 1131
Met Ser Thr Met Arg Leu Leu Thr Leu Ala Leu
-18 -15 -10

CTG TTC TCC TGC TCC GTC GCC CGT GCC GCG TGC GAC CCC AAG ATC GTC 1179
Leu Phe Ser Cys Ser Val Ala Arg Ala Ala Cys Asp Pro Lys Ile Val
-5 1 5

AAC ATT GGC GCG GTG CTG AGC ACG CGG AAG CAC GAG CAG ATG TTC CGC 1227
Asn Ile Gly Ala Val Leu Ser Thr Arg Lys His Glu Gln Met Phe Arg
10 15 20 25

GAG GCC GTG AAC CAG GCC AAC AAG CGG CAC GGC TCC TGG AAG ATT CAG 1275
Glu Ala Val Asn Gln Ala Asn Lys Arg His Gly Ser Trp Lys Ile Gln
30 35 40

CTC AAT GCC ACC TCC GTC ACG CAC AAG CCC AAC GCC ATC CAG ATG GCT 1323
Leu Asn Ala Thr Ser Val Thr His Lys Pro Asn Ala Ile Gln Met Ala
45 50 55

CTG TCG GTG TGC GAG GAC CTC ATC TCC AGC CAG GTC TAC GCC ATC CTA 1371
Leu Ser Val Cys Glu Asp Leu Ile Ser Ser Gln Val Tyr Ala Ile Leu
60 65 70

GTT AGC CAT CCA CCT ACC CCC AAC GAC CAC TTC ACT CCC ACC CCT GTC 1419
Val Ser His Pro Pro Thr Pro Asn Asp His Phe Thr Pro Thr Pro Val
75 80 85

TCC TAC ACA GCC GGC TTC TAC CGC ATA CCC GTG CTG GGG CTG ACC ACC 1467
Ser Tyr Thr Ala Gly Phe Tyr Arg Ile Pro Val Leu Gly Leu Thr Thr
90 95 100 105

CGC ATG TCC ATC TAC TCG GAC AAG AGC ATC CAC CTG AGC TTC CTG CGC 1515
Arg Met Ser Ile Tyr Ser Asp Lys Ser Ile His Leu Ser Phe Leu Arg
110 115 120

ACC GTG CCG CCC TAC TCC CAC CAG TCC AGC GTG TGG TTT GAG ATG ATG 1563
Thr Val Pro Pro Tyr Ser His Gln Ser Ser Val Trp Phe Glu Met Met
125 130 135

CGT GTC TAC AGC TGG AAC CAC ATC ATC CTG CTG GTC AGC GAC GAC CAC 1611
Arg Val Tyr Ser Trp Asn His Ile Ile Leu Leu Val Ser Asp Asp His
140 145 150

GAG GGC CGG GCG GCT CAG AAA CGC CTG GAG ACG CTG CTG GAG GAG CGT 1659
Glu Gly Arg Ala Ala Gln Lys Arg Leu Glu Thr Leu Leu Glu Glu Arg
155 160 165

GAG TCC AAG GCA GAG AAG GTG CTG CAG TTT GAC CCA GGG ACC AAG AAC 1707
Glu Ser Lys Ala Glu Lys Val Leu Gln Phe Asp Pro Gly Thr Lys Asn
170 175 180 185

GTG ACG GCC CTG CTG ATG GAG GCG AAA GAG CTG GAG GCC CGG GTC ATC 1755
Val Thr Ala Leu Leu Met Glu Ala Lys Glu Leu Glu Ala Arg Val Ile
190 195 200

ATC CTT TCT GCC AGC GAG GAC GAT GCT GCC ACT GTA TAC CGC GCA GCC 1803
Ile Leu Ser Ala Ser Glu Asp Asp Ala Ala Thr Val Tyr Arg Ala Ala
205 210 215

GCG ATG CTG AAC ATG ACG GGC TCC GGG TAC GTG TGG CTG GTC GGC GAG 1851
Ala Met Leu Asn Met Thr Gly Ser Gly Tyr Val Trp Leu Val Gly Glu
220 225 230

CGC GAG ATC TCG GGG AAC GCC CTG CGC TAC GCC CCA GAC GGC ATC CTC 1899
Arg Glu Ile Ser Gly Asn Ala Leu Arg Tyr Ala Pro Asp Gly Ile Leu
235 240 245

GGG CTG CAG CTC ATC AAC GGC AAG AAC GAG TCG GCC CAC ATC AGC GAC 1947
Gly Leu Gln Leu Ile Asn Gly Lys Asn Glu Ser Ala His Ile Ser Asp
250 255 260 265

GCC GTG GGC GTG GTG GCC CAG GCC GTG CAC GAG CTC CTC GAG AAG GAG 1995
Ala Val Gly Val Val Ala Gln Ala Val His Glu Leu Leu Glu Lys Glu
270 275 280

AAC ATC ACC GAC CCG CCG CGG GGC TGC GTG GGC AAC ACC AAC ATC TGG 2043
Asn Ile Thr Asp Pro Pro Arg Gly Cys Val Gly Asn Thr Asn Ile Trp
285 290 295

AAG ACC GGG CCG CTC TTC AAG AGA GTG CTG ATG TCT TCC AAG TAT GCG 2091
Lys Thr Gly Pro Leu Phe Lys Arg Val Leu Met Ser Ser Lys Tyr Ala
300 305 310

GAT GGG GTG ACT GGT CGC GTG GAG TTC AAT GAG GAT GGG GAC CGG AAG 2139
Asp Gly Val Thr Gly Arg Val Glu Phe Asn Glu Asp Gly Asp Arg Lys
315 320 325

TC GCC AAC TAC AGC ATC ATG AAC CTG CAG AAC CGC AAG CTG GTG CAA 2187
he Ala Asn Tyr Ser Ile Met Asn Leu Gln Asn Arg Lys Leu Val Gln
30 335 340 345

TG GGC ATC TAC AAT GGC ACC CAC GTC ATC CCT AAT GAC AGG AAG ATC 2235
al Gly Ile Tyr Asn Gly Thr His Val Ile Pro Asn Asp Arg Lys Ile
350 355 360

TC TGG CCA GGC GGA GAG ACA GAG AAG CCT CGA GGG TAC CAG ATG TCC 2283
le Trp Pro Gly Gly Glu Thr Glu Lys Pro Arg Gly Tyr Gln Met Ser
365 370 375

CC AGA CTG AAG ATT GTG ACG ATC CAC CAG GAG CCC TTC GTG TAC GTC 2331
hr Arg Leu Lys Ile Val Thr Ile His Gln Glu Pro Phe Val Tyr Val
380 385 390

AG CCC ACG CTG AGT GAT GGG ACA TGC AAG GAG GAG TTC ACA GTC AAC 2379
ys Pro Thr Leu Ser Asp Gly Thr Cys Lys Glu Glu Phe Thr Val Asn
395 400 405

GC GAC CCA GTC AAG AAG GTG ATC TGC ACC GGG CCC AAC GAC ACG TCG 2427
ly Asp Pro Val Lys Lys Val Ile Cys Thr Gly Pro Asn Asp Thr Ser
10 415 420 425

CG GGC AGC CCC CGC CAC ACG GTG CCT CAG TGT TGC TAC GGC TTT TGC 2475
ro Gly Ser Pro Arg His Thr Val Pro Gln Cys Cys Tyr Gly Phe Cys
430 435 440

TC GAC CTG CTC ATC AAG CTG GCA CGG ACC ATG AAC TTC ACC TAC GAG 2523
le Asp Leu Leu Ile Lys Leu Ala Arg Thr Met Asn Phe Thr Tyr Glu
445 450 455

TG CAC CTG GTG GCA GAT GGC AAG TTC GGC ACA CAG GAG CGG GTG AAC 2571
al His Leu Val Ala Asp Gly Lys Phe Gly Thr Gln Glu Arg Val Asn
460 465 470

AC AGC AAC AAG AAG GAG TGG AAT GGG ATG ATG GGC GAG CTG CTC AGC 2619
n Ser Asn Lys Lys Glu Trp Asn Gly Met Met Gly Glu Leu Leu Ser
475 480 485

G CAG GCA GAC ATG ATC GTG GCG CCG CTA ACC ATA AAC AAC GAG CGC 2667
y Gln Ala Asp Met Ile Val Ala Pro Leu Thr Ile Asn Asn Glu Arg
0 495 500 505

G CAG TAC ATC GAG TTT TCC AAG CCC TTC AAG TAC CAG GGC CTG ACT 2715
a Gln Tyr Ile Glu Phe Ser Lys Pro Phe Lys Tyr Gln Gly Leu Thr
510 515 520

T CTG GTC AAG AAG GAG ATT CCC CGG AGC ACG CTG GAC TCG TTC ATG 2763
e Leu Val Lys Lys Glu Ile Pro Arg Ser Thr Leu Asp Ser Phe Met
525 530 535

G CCG TTC CAG AGC ACA CTG TGG CTG CTG GTG GGG CTG TCG GTG CAC 2811
n Pro Phe Gln Ser Thr Leu Trp Leu Leu Val Gly Leu Ser Val His
540 545 550

G GTG GCC GTG ATG CTG TAC CTG CTG GAC CGC TTC AGC CCC TTC GGC 2859
l Val Ala Val Met Leu Tyr Leu Leu Asp Arg Phe Ser Pro Phe Gly
555 560 565

CGG TTC AAG GTG AAC AGC GAG GAG GAG GAG GAG GAC GCA CTG ACC CTG 2907
Arg Phe Lys Val Asn Ser Glu Glu Glu Glu Glu Asp Ala Leu Thr Leu
570 575 580 585

TCC TCG GCC ATG TGG TTC TCC TGG GGC GTC CTG CTC AAC TCC GGC ATC 2955
Ser Ser Ala Met Trp Phe Ser Trp Gly Val Leu Leu Asn Ser Gly Ile
590 595 600

GGG GAA GGC GCC CCC AGA AGC TTC TCA GCG CGC ATC CTG GGC ATG GTG 3003
Gly Glu Gly Ala Pro Arg Ser Phe Ser Ala Arg Ile Leu Gly Met Val
605 610 615

TGG GCC GGC TTT GCC ATG ATC ATC GTG GCC TCC TAC ACC GCC AAC CTG 3051
Trp Ala Gly Phe Ala Met Ile Ile Val Ala Ser Tyr Thr Ala Asn Leu
620 625 630

GCG GCC TTC CTG GTG CTG GAC CGG CCG GAG GAG CGC ATC ACG GGC ATC 3099
Ala Ala Phe Leu Val Leu Asp Arg Pro Glu Glu Arg Ile Thr Gly Ile
635 640 645

AAC GAC CCT CGG CTG AGG AAC CCC TCG GAC AAG TTT ATC TAC GCC ACG 3147
Asn Asp Pro Arg Leu Arg Asn Pro Ser Asp Lys Phe Ile Tyr Ala Thr
650 655 660 665

GTG AAG CAG AGC TCC GTG GAT ATC TAC TTC CGG CGC CAG GTG GAG CTG 3195
Val Lys Gln Ser Ser Val Asp Ile Tyr Phe Arg Arg Gln Val Glu Leu
670 675 680

AGC ACC ATG TAC CGG CAT ATG GAG AAG CAC AAC TAC GAG AGT GCG GCG 3243
Ser Thr Met Tyr Arg His Met Glu Lys His Asn Tyr Glu Ser Ala Ala
685 690 695

GAG GCC ATC CAG GCC GTG AGA GAC AAC AAG CTG CAT GCC TTC ATC TGG 3291
Glu Ala Ile Gln Ala Val Arg Asp Asn Lys Leu His Ala Phe Ile Trp
700 705 710

GAC TCG GCG GTG CTG GAG TTC GAG GCC TCG CAG AAG TGC GAC CTG GTG 3339
Asp Ser Ala Val Leu Glu Phe Glu Ala Ser Gln Lys Cys Asp Leu Val
715 720 725

ACG ACT GGA GAG CTG TTT TTC CGC TCG GGC TTC GGC ATA GGC ATG CGC 3387
Thr Thr Gly Glu Leu Phe Phe Arg Ser Gly Phe Gly Ile Gly Met Arg
730 735 740 745

AAA GAC AGC CCC TGG AAG CAG AAC GTC TCC CTG TCC ATC CTC AAG TCC 3435
Lys Asp Ser Pro Trp Lys Gln Asn Val Ser Leu Ser Ile Leu Lys Ser
750 755 760

CAC GAG AAT GGC TTC ATG GAA GAC CTG GAC AAG ACG TGG GTT CGG TAT 3483
His Glu Asn Gly Phe Met Glu Asp Leu Asp Lys Thr Trp Val Arg Tyr
765 770 775

CAG GAA TGT GAC TCG CGC AGC AAC GCC CCT GCG ACC CTT ACT TTT GAG 3531
Gln Glu Cys Asp Ser Arg Ser Asn Ala Pro Ala Thr Leu Thr Phe Glu
780 785 790

AAC ATG GCC GGG GTC TTC ATG CTG GTA GCT GGG GGC ATC GTG GCC GGG 3579
Asn Met Ala Gly Val Phe Met Leu Val Ala Gly Gly Ile Val Ala Gly
795 800 805

ATC TTC CTG ATT TTC ATC GAG ATT GCC TAC AAG CGG CAC AAG GAT GCT 3627
Ile Phe Leu Ile Phe Ile Glu Ile Ala Tyr Lys Arg His Lys Asp Ala
810 815 820 825

CGC CGG AAG CAG ATG CAG CTG GCC TTT GCC GCC GTT AAC GTG TGG CGG 3675
Arg Arg Lys Gln Met Gln Leu Ala Phe Ala Ala Val Asn Val Trp Arg
830 835 840

AAG AAC CTG CAG CAG TAC CAT CCC ACT GAT ATC ACG GGC CCG CTC AAC 3723
Lys Asn Leu Gln Gln Tyr His Pro Thr Asp Ile Thr Gly Pro Leu Asn
845 850 855

CTC TCA GAT CCC TCG GTC AGC ACC GTG GTG TGAGGCCCCC GGAGGCGCCC 3773
Leu Ser Asp Pro Ser Val Ser Thr Val Val
860 865

ACCTGCCCAG TTAGCCCGGC CAAGGACACT GATGGGTCCT GCTGCTCGGG AAGGCCTGAG 3833

GGAAGCCCAC CCGCCCCAGA GACTGCCCAC CCTGGGCCTC CCGTCCGTCC GCCCGCCCAC 3893

CCCGCTGCCT GGCGGGCAGC CCCTGCTGGA CCAAGGTGCG GACCGGAGCG GCTGAGGACG 3953

GGGCAGAGCT GAGTCGGCTG GGCAGGGCGC AGGGCGCTCC GGCAGAGGCA GGGCCCTGGG 4013

GTCTCTGAGC AGTGGGGAGC GGGGGCTAAC TGGCCCCAGG CGAAGGGGCT TGGAGCAGAG 4073

ACGGCAGCCC CATCCTTCCC GCAGCACCAG CCTGAGCCAC AGTGGGGCCC ATGGCCCCAG 4133

CTGGCTGGGT CGCCCCTCCT CGGGCGCCTG CGCTCCTCTG CAGCCTGAGC TCCACCCTCC 4193

CCTCTTCTTG CGGCACCGCC CACCCACACC CCGTCTGCCC CTTGACCCCA CACGCCGGGG 4253

CTGGCCCTGC CCTCCCCCAC GGCCGTCCCT GACTTCCCAG CTGGCAGCGC CTCCCGCCGC 4313

CTCGGGCCGC CTCCTCCAGA CTCGAGAGGG CTGAGCCCCT CCTCTCCTCG TCCGGCCTGC 4373

AGCCCAGAAC GGGCCTCCCC GGGGGTCCCC GGACGCTGGC TCGGGACTGT CTTCAACCCT 4433

GCCCTGCACC TTGGGCACGG GAGAGCGCCA CCCGCCCGCC CCCGCCCTCG CTCCGGGTGC 4493

GTGACCGGCC CGCCACCTTG TACAGAACCA GCACTCCCAG GGCCCGAGCG CGTGCCTTCC 4553

CCGTGCGGCC CGTGCGCAGC CGCGCTCTGC CCCTCCGTCC CCAGGGTGCA GGCGCGCACC 4613

GCCCAACCCC CACCTCCCGG TGTATGCAGT GGTGATGCCG GAATTC
4659

(2) INFORMATION FOR SEQ ID NO:16:

(i) SEQUENCE CHARACTERISTICS:
(A) LENGTH: 885 amino acids
(B) TYPE: amino acid
(D) TOPOLOGY: linear

(ii) MOLECULE TYPE: protein

(xi) SEQUENCE DESCRIPTION: SEQ ID NO:16:

Met Ser Thr Met Arg Leu Leu Thr Leu Ala Leu Leu Phe Ser Cys Ser
-18 -15 -10 -5

Val Ala Arg Ala Ala Cys Asp Pro Lys Ile Val Asn Ile Gly Ala Val
1 5 10

Leu Ser Thr Arg Lys His Glu Gln Met Phe Arg Glu Ala Val Asn Gln
15 20 25 30

Ala Asn Lys Arg His Gly Ser Trp Lys Ile Gln Leu Asn Ala Thr Ser
        35                  40                  45

Val Thr His Lys Pro Asn Ala Ile Gln Met Ala Leu Ser Val Cys Glu
    50                  55                  60

Asp Leu Ile Ser Ser Gln Val Tyr Ala Ile Leu Val Ser His Pro Pro
65 70 75

Thr Pro Asn Asp His Phe Thr Pro Thr Pro Val Ser Tyr Thr Ala Gly
80 85 90

Phe Tyr Arg Ile Pro Val Leu Gly Leu Thr Thr Arg Met Ser Ile Tyr
95 100 105 110

Ser Asp Lys Ser Ile His Leu Ser Phe Leu Arg Thr Val Pro Pro Tyr
        115                 120                 125

Ser His Gln Ser Ser Val Trp Phe Glu Met Met Arg Val Tyr Ser Trp
    130                 135                 140

Asn His Ile Ile Leu Leu Val Ser Asp Asp His Glu Gly Arg Ala Ala
145 150 155

Gln Lys Arg Leu Glu Thr Leu Leu Glu Glu Arg Glu Ser Lys Ala Glu
160 165 170

Lys Val Leu Gln Phe Asp Pro Gly Thr Lys Asn Val Thr Ala Leu Leu
175 180 185 190

Met Glu Ala Lys Glu Leu Glu Ala Arg Val Ile Ile Leu Ser Ala Ser
        195                 200                 205

Glu Asp Asp Ala Ala Thr Val Tyr Arg Ala Ala Ala Met Leu Asn Met
    210                 215                 220

Thr Gly Ser Gly Tyr Val Trp Leu Val Gly Glu Arg Glu Ile Ser Gly
225 230 235

Asn Ala Leu Arg Tyr Ala Pro Asp Gly Ile Leu Gly Leu Gln Leu Ile
240 245 250

Asn Gly Lys Asn Glu Ser Ala His Ile Ser Asp Ala Val Gly Val Val
255 260 265 270

Ala Gln Ala Val His Glu Leu Leu Glu Lys Glu Asn Ile Thr Asp Pro
        275                 280                 285

Pro Arg Gly Cys Val Gly Asn Thr Asn Ile Trp Lys Thr Gly Pro Leu
    290                 295                 300

Phe Lys Arg Val Leu Met Ser Ser Lys Tyr Ala Asp Gly Val Thr Gly
305 310 315

Arg Val Glu Phe Asn Glu Asp Gly Asp Arg Lys Phe Ala Asn Tyr Ser
320 325 330

Ile Met Asn Leu Gln Asn Arg Lys Leu Val Gln Val Gly Ile Tyr Asn
335 340 345 350

Gly Thr His Val Ile Pro Asn Asp Arg Lys Ile Ile Trp Pro Gly Gly
        355                 360                 365

Glu Thr Glu Lys Pro Arg Gly Tyr Gln Met Ser Thr Arg Leu Lys Ile
    370                 375                 380

Val Thr Ile His Gln Glu Pro Phe Val Tyr Val Lys Pro Thr Leu Ser
385 390 395

Asp Gly Thr Cys Lys Glu Glu Phe Thr Val Asn Gly Asp Pro Val Lys
400 405 410

Lys Val Ile Cys Thr Gly Pro Asn Asp Thr Ser Pro Gly Ser Pro Arg
415 420 425 430

His Thr Val Pro Gln Cys Cys Tyr Gly Phe Cys Ile Asp Leu Leu Ile
        435                 440                 445

Lys Leu Ala Arg Thr Met Asn Phe Thr Tyr Glu Val His Leu Val Ala
    450                 455                 460

Asp Gly Lys Phe Gly Thr Gln Glu Arg Val Asn Asn Ser Asn Lys Lys
465 470 475

Glu Trp Asn Gly Met Met Gly Glu Leu Leu Ser Gly Gln Ala Asp Met
480 485 490

Ile Val Ala Pro Leu Thr Ile Asn Asn Glu Arg Ala Gln Tyr Ile Glu
495 500 505 510

Phe Ser Lys Pro Phe Lys Tyr Gln Gly Leu Thr Ile Leu Val Lys Lys
        515                 520                 525

Glu Ile Pro Arg Ser Thr Leu Asp Ser Phe Met Gln Pro Phe Gln Ser
    530                 535                 540

Thr Leu Trp Leu Leu Val Gly Leu Ser Val His Val Val Ala Val Met
545 550 555

Leu Tyr Leu Leu Asp Arg Phe Ser Pro Phe Gly Arg Phe Lys Val Asn
560 565 570

Ser Glu Glu Glu Glu Glu Asp Ala Leu Thr Leu Ser Ser Ala Met Trp
575 580 585 590

Phe Ser Trp Gly Val Leu Leu Asn Ser Gly Ile Gly Glu Gly Ala Pro
        595                 600                 605

Arg Ser Phe Ser Ala Arg Ile Leu Gly Met Val Trp Ala Gly Phe Ala
    610                 615                 620

Met Ile Ile Val Ala Ser Tyr Thr Ala Asn Leu Ala Ala Phe Leu Val
625 630 635

Leu Asp Arg Pro Glu Glu Arg Ile Thr Gly Ile Asn Asp Pro Arg Leu
640 645 650

Arg Asn Pro Ser Asp Lys Phe Ile Tyr Ala Thr Val Lys Gln Ser Ser
655 660 665 670

Val Asp Ile Tyr Phe Arg Arg Gln Val Glu Leu Ser Thr Met Tyr Arg
        675                 680                 685

His Met Glu Lys His Asn Tyr Glu Ser Ala Ala Glu Ala Ile Gln Ala
    690                 695                 700

Val Arg Asp Asn Lys Leu His Ala Phe Ile Trp Asp Ser Ala Val Leu
705 710 715

Glu Phe Glu Ala Ser Gln Lys Cys Asp Leu Val Thr Thr Gly Glu Leu
720 725 730

Phe Phe Arg Ser Gly Phe Gly Ile Gly Met Arg Lys Asp Ser Pro Trp
735 740 745 750

Lys Gln Asn Val Ser Leu Ser Ile Leu Lys Ser His Glu Asn Gly Phe
        755                 760                 765

Met Glu Asp Leu Asp Lys Thr Trp Val Arg Tyr Gln Glu Cys Asp Ser
    770                 775                 780

Arg Ser Asn Ala Pro Ala Thr Leu Thr Phe Glu Asn Met Ala Gly Val
785 790 795

Phe Met Leu Val Ala Gly Gly Ile Val Ala Gly Ile Phe Leu Ile Phe
800 805 810

Ile Glu Ile Ala Tyr Lys Arg His Lys Asp Ala Arg Arg Lys Gln Met
815 820 825 830

Gln Leu Ala Phe Ala Ala Val Asn Val Trp Arg Lys Asn Leu Gln Gln
        835                 840                 845

Tyr His Pro Thr Asp Ile Thr Gly Pro Leu Asn Leu Ser Asp Pro Ser
    850                 855                 860

Val Ser Thr Val Val
865

(2) INFORMATION FOR SEQ ID NO:17:

(i) SEQUENCE CHARACTERISTICS:
(A) LENGTH: 28 base pairs
(B) TYPE: nucleic acid
(C) STRANDEDNESS: double
(D) TOPOLOGY: linear

(ii) MOLECULE TYPE: cDNA

(xi) SEQUENCE DESCRIPTION: SEQ ID NO:17:

GAAGAACCTG CAGCAGTACC ATCCCACT 28

(2) INFORMATION FOR SEQ ID NO:18:

(i) SEQUENCE CHARACTERISTICS:
(A) LENGTH: 391 base pairs
(B) TYPE: nucleic acid
(C) STRANDEDNESS: double
(D) TOPOLOGY: linear

(ii) MOLECULE TYPE: cDNA

(xi) SEQUENCE DESCRIPTION: SEQ ID NO:18:

GAAGAACCTG CAGAGCACCG GGGGTGGACG CGGCGCTTTG CAAAACCAAA AAGACACAGT 60

GCTGCCGCGA CGCGCTATTG AGAGGGAGGA GGGCCAGCTG CAGCTGTGTT CCCGTCATAG 120

GGAGAGCTGA GACTCCCCGC CCGCCCTCCT CTGCCCCCTC CCCCGCAGAC AGACAGACAG 180

ACGGATGGGA CAGCGGCCCG GCCCACGCAG AGCCCCGGAG CACCACGGGG TCGGGGGAGG 240

AGCACCCCCA GCCTCCCCCA GGCTGCGCCT GCCCGCCCGC CGGTTGGCCG GCTGGCCGGT 300

CCACCCCGTC CCGGCCCCGC GCGTGCCCCC AGCGTGGGGC TAACGGGCGC CTTGTCTGTG 360

TATTTCTATT TTGCAGCAGT ACCATCCCAC T 391

(2) INFORMATION FOR SEQ ID NO:19:

(i) SEQUENCE CHARACTERISTICS:
(A) LENGTH: 502 base pairs
(B) TYPE: nucleic acid
(C) STRANDEDNESS: double
(D) TOPOLOGY: linear

(ii) MOLECULE TYPE: cDNA

(xi) SEQUENCE DESCRIPTION: SEQ ID NO:19:

GAAGAACCTG CAGGATAGAA AGAGTGGTAG AGCAGAGCCT GACCCTAAAA AGAAAGCCAC 60

ATTTAGGGCT ATCACCTCCA CCCTGGCTTC CAGCTTCAAG AGGCGTAGGT CCTCCAAAGA 120

CACGAGCACC GGGGGTGGAC GCGGCGCTTT GCAAAACCAA AAAGACACAG TGCTGCCGCG 180

ACGCGCTATT GAGAGGGAGG AGGGCCAGCT GCAGCTGTGT TCCCGTCATA GGGAGAGCTG 240

AGACTCCCCG CCCGCCCTCC TCTGCCCCCT CCCCCGCAGA CAGACAGACA GACGGATGGG 300

ACAGCGGCCC GGCCCACGCA GAGCCCCGGA GCACCACGGG GTCGGGGGAG GAGCACCCCC 360

AGCCTCCCCC AGGCTGCGCC TGCCCGCCCG CCGGTTGGCC GGCTGGCCGG TCCACCCCGT 420

CCCGGCCCCG CGCGTGCCCC CAGCGTGGGG CTAACGGGCG CCTTGTCTGT GTATTTCTAT 480

TTTGCAGCAG TACCATCCCA CT 502

(2) INFORMATION FOR SEQ ID NO:20:

(i) SEQUENCE CHARACTERISTICS:
(A) LENGTH: 502 base pairs
(B) TYPE: nucleic acid
(C) STRANDEDNESS: double
(D) TOPOLOGY: linear

(ii) MOLECULE TYPE: cDNA

(xi) SEQUENCE DESCRIPTION: SEQ ID NO:20:

GAAGAACCTG CAGGATAGAA AGAGTGGTAG AGCAGAGCCT GACCCTAAAA AGAAAGCCAC 60

ATTTAGGGCT ATCACCTCCA CCCTGGCTTC CAGCTTCAAG AGGCGTAGGT CCTCCAAAGA 120

CACGAGCACC GGGGGTGGAC GCGGCGCTTT GCAAAACCAA AAAGACACAG TGCTGCCGCG 180

ACGCGCTATT GAGAGGGAGG AGGGCCAGCT GCAGCTGTGT TCCCGTCATA GGGAGAGCTG 240

AGACTCCCCG CCCGCCCTCC TCTGCCCCCT CCCCCGCAGA CAGACAGACA GACGGATGGG 300

ACAGCGGCCC GGCCCACGCA GAGCCCCGGA GCACCACGGG GTCGGGGGAG GAGCACCCCC 360

AGCCTCCCCC AGGCTGCGCC TGCCCGCCCG CCGGTTGGCC GGCTGGCCGG TCCACCCCGT 420

CCCGGCCCCG CGCGTGCCCC CAGCGTGGGG CTAACGGGCG CCTTGTCTGT GTATTTCTAT 480

TTTGCAGCAG TACCATCCCA CT 502

(2) INFORMATION FOR SEQ ID NO:21:

(i) SEQUENCE CHARACTERISTICS:
(A) LENGTH: 65 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:21:

Ala Gly Gly Ile Val Ala Gly Ile Phe Leu Ile Phe Ile Glu Ile Ala
1               5                   10                  15

Tyr Lys Arg His Lys Asp Ala Arg Arg Lys Gln Met Gln Leu Ala Phe
            20                  25                  30

Ala Ala Val Asn Val Trp Arg Lys Asn Leu Gln Gln Tyr His Pro Thr
        35                  40                  45

Asp Ile Thr Gly Pro Leu Asn Leu Ser Asp Pro Ser Val Ser Thr Val
    50                  55                  60

Val
65

(2) INFORMATION FOR SEQ ID NO:22:

(i) SEQUENCE CHARACTERISTICS:
(A) LENGTH: 102 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:22:

Ala Gly Gly Ile Val Ala Gly Ile Phe Leu Ile Phe Ile Glu Ile Ala
1               5                   10                  15

Tyr Lys Arg His Lys Asp Ala Arg Arg Lys Gln Met Gln Leu Ala Phe
            20                  25                  30

Ala Ala Val Asn Val Trp Arg Lys Asn Leu Gln Asp Arg Lys Ser Gly
        35                  40                  45

Arg Ala Glu Pro Asp Pro Lys Lys Lys Ala Thr Phe Arg Ala Ile Thr
    50                  55                  60

Ser Thr Leu Ala Ser Ser Phe Lys Arg Arg Arg Ser Ser Lys Asp Thr
65                  70                  75                  80

Gln Tyr His Pro Thr Asp Ile Thr Gly Pro Leu Asn Leu Ser Asp Pro
                85                  90                  95

Ser Val Ser Thr Val Val
            100

(2) INFORMATION FOR SEQ ID NO:23:

(i) SEQUENCE CHARACTERISTICS:
(A) LENGTH: 47 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:23:

Lys Arg Leu Glu Thr Leu Leu Glu Glu Arg Glu Ser Lys Ser Lys Lys
1               5                   10                  15

Arg Asn Tyr Glu Asn Leu Asp Gln Leu Ser Tyr Asp Asn Lys Arg Gly
            20                  25                  30

Pro Lys Ala Glu Lys Val Leu Gln Phe Asp Pro Gly Thr Lys Asn
        35                  40                  45

(2) INFORMATION FOR SEQ ID NO:24:

(i) SEQUENCE CHARACTERISTICS:
(A) LENGTH: 26 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:24:

Lys Arg Leu Glu Thr Leu Leu Glu Glu Arg Glu Ser Lys Ala Glu Lys
1               5                   10                  15

Val Leu Gln Phe Asp Pro Gly Thr Lys Asn
            20                  25

(2) INFORMATION FOR SEQ ID NO:25:

(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:25:

GGGGTTTAGA TCTGGGTNAT GATGTTYGTN ATG                                33

(2) INFORMATION FOR SEQ ID NO:26:

(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:26:

GGGGTTTAGA TCTGCNGCRT CRTADATRAA NGC                                33

(2) INFORMATION FOR SEQ ID NO:27:

(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:27:

GGGGTTTGGA TCCAAAGAAT GGAACGGAAT GATG                               34

(2) INFORMATION FOR SEQ ID NO:28:

(i) SEQUENCE CHARACTERISTICS:
(A) LENGTH: 34 base pairs
(B) TYPE: nucleic acid
(C) STRANDEDNESS: single
(D) TOPOLOGY: linear

(xi) SEQUENCE DESCRIPTION: SEQ ID NO:28:

GGGGTTTAAG CTTCTCGTAG TTGTGCTTCT CCAT 34